# EUROPÄISCHE PATENTSCHRIFT

(11) **EP 1 356 291 B1**
(45) Veröffentlichungstag und Bekanntmachung des Hinweises auf die Patenterteilung: **04.05.2011**
(21) Anmeldenummer: 01984800.1
(22) Anmeldetag: 04.12.2001
(51) Int. Cl.: G01N 33/554

(54) **VERFAHREN ZUR HERSTELLUNG EINES BIOANALYTISCHEN REAGENZES, NACHWEISVERFAHREN BERUHEND AUF DER VERWENDUNG DES BIOANALYTISCHEN REAGENZ, SOWIE DESSEN VERWENDUNG**
METHOD FOR PRODUCTION OF A BIOANALYTICAL REAGENT, DETECTION METHOD BASED ON USE OF SAID BIOANALYTICAL REAGENT, AS WELL AS THE USE OF SAID METHOD
PROCEDE DE PRODUCTION D'UN REACTIF BIOANALYTIQUE, PROCEDE D'IDENTIFICATION FONDE SUR L'UTILISATION DU REACTIF BIOANALYTIQUE ET UTILISATION DUDIT PROCEDE

(30) Priorität: 06.12.2000 CH 237400
(43) Veröffentlichungstag der Anmeldung: 29.10.2003
(73) Patentinhaber: École Polytechnique Fédérale de Lausanne (EPFL), 1015 Lausanne (CH)
(72) Erfinder: VOGEL, Horst, CH-1028 Préverenges (CH); PICK, Horst, M., CH-1028 Préverenges (CH); PREUSS, Axel, K., New York, NY 10019 (US); TAIRI, Ana-Paula, CH-1006 Lausanne (CH); SCHMID-OSBORNE, Evelyne, Singapore 258056 (SG); PAWLAK, Michael, 79275 Laufenburg (DE)
(74) Vertreter: Hepp, Dieter
(86) Internationale Anmeldenummer: PCT/EP2001/014134
(87) Internationale Veröffentlichungsnummer: WO 2002/046766

(56) Entgegenhaltungen:
- WO-A-01/82958
- WO-A-97/45534
- US-A- 4 877 619
- HEYSE STEPHAN ET AL: "Covalent attachment of functionalized lipid bilayers to planar waveguides for measuring protein binding to biomimetic membranes." PROTEIN SCIENCE, Bd. 4, Nr. 12, 1995, Seiten 2532-2544, XP001088397 ISSN: 0961-8368

## Beschreibung

Die Erfindung betrifft verschiedene Ausführungsformen eines bioanalytisches Reagens mit mindestens einem Vesikel, das aus einer lebenden Zelle erzeugt wird, umfassend mindestens einen Rezeptor, dadurch gekennzeichnet, dass ein von diesem Rezeptor in der verwendeten lebenden Zelle ausgelöster Mechanismus einer Signaltransduktion in besagtem Vesikel als Bestandteil des bioanalytischen Reagens erhalten bleibt. Die Erfindung betrifft ausserdem Verfahren zur Herstellung eines erfindungsgemässen bioanalytischen Reagens sowie auf dem Einsatz dieses Reagens basierende bioanalytische Nachweisverfahren sowie die Verwendung dieses Nachweisverfahren und des bioanalytischen Reagens.

Lebende Organismen oder Zellen können unterschiedliche äussere Signale (Licht, Hormone, Gerüche etc.) wahrnehmen und darauf reagieren. Diese Signale werden über Rezeptoren an Zelloberflächen (= Plasmamembran-Rezeptoren) empfangen und verarbeitet, welche die Signale durch die Membran leiten und im Zellinnem eine Vielzahl von Prozessen aktivieren, die zu einer zellulären Antwort führen.

Viele Plasmamembran-Rezeptoren sind wichtige Zielmoleküle von therapeutisch aktiven Substanzen. Eine medizinisch wichtige Rezeptoren-Familie sind G-Protein-gekoppelte Rezeptoren (G protein-coupled receptors, GPCR). Sie bilden die grösste Gruppe von membranständigen Rezeptoren und steuern die zelluläre Antwort mittels der intermediären Rolle von G-Proteinen. Daher sind GPCR ein wichtiges therapeutisches Ziel für den medikamentösen Einsatz im menschlichen Körper (Neer, E. J., Cell, 80 (1995) 249 - 257; Bourne, H. R., Curr. Opin. Cell Biol., 9 (1997) 134 - 142; Wess J., FASEB J., 11 (1997) 346-354).

Eine Schlüsselrolle für die Interaktion von Rezeptoren mit G-Proteinen spielt die G_{α} Untereinheit von G-Proteinen (Milligan G, Mullaney I, McKenzie FR: " Specificity of interactions of receptors and effectors with GTP-binding proteins in native membranes", Biochem. Soc. Symp. 56 (1990) 21-34). Das gängige Model für die Aktivierung von G-Proteinen besagt, dass dieses heterotrimere, membranständige Protein zerfällt in eine freie Gα, GTP (Guanosintriphosphat)-gebundene Untereinheit und ein freies G_{βγ} -Dimeres. Nach GTP-Hydrolyse assoziiert G_{α}-(GDP) wieder mit G_{βγ} (Willard F. S., Crouch M. F., Immunol. Cell. Biol., 78 (2000) 387-394).

Eine andere therapeutisch wichtige Klasse von Rezeptoren sind Kanalproteine, welche extrazelluläre Signale detektieren und sie in zelluläre Antworten überführen (F. M. Ashcroft: "Ion channels and disease", Academic Press, San Diego, 2000). Die Funktion der Kanalproteine besteht im Öffnen und Schliessen von Ionenkanälen.

Für die Funktionsfähigkeit des von G-Protein-gekoppelten Rezeptoren vermittelten Mechanismus einer Signaltransduktion ist das Vorhandensein des endoplasmatischen Reticulums im Zellinneren von entscheidender Bedeutung. Das Endoplasmatische Reticulum ist der wichtigste intrazelluläre Calciumspeicher, über den Calciumionen (sekundärer Botenstoff) nach Aktivierung von G-Protein-gekoppelten Rezeptoren ins Cytoplasma freigesetzt werden (siehe z. B. Muallem S, Wilkie TM: "G protein-dependent, Ca2+ signaling complexes in polarized cells", Cell Calcium 26 (1999) 173-180).

Weitere Calciumspeicher von geringerer Bedeutung sind der Zellkern und die Mitochondrien. Das endoplasmatische Reticulum akkumuliert Calciumionen über die Ca²⁺-ATPase als Ionenpumpe und setzt Calcium über entsprechende Rezeptor-Ionenkanäle wieder frei, die durch die Botenstoffe Inositol-1,4,5-triphosphat (IP3) und cyclisches Adenosindiphosphat (cADP) gesteuert werden (Brini M., Carafoli E.: "Calcium signalling: a historical account, recent developments and future perspectives", Cell. Mol. Life Sci., 57 (2000) 354-370). Die basale Ca²⁺ Konzentration im endoplasmatischen Reticulum beträgt ungefähr 500 µM und fällt auf etwa 100 µM ab, wenn die Calciumionen in das Cytoplasma strömen (Yu R., Hinkle P. M., "Rapid Turnover of Calcium in the Endoplasmic Reticulum During Signaling: Studies with Cameleon Calcium Indicators", J. Biol. Chem., 275 (2000) 23648 - 23653.

Der Nachweis einer Zu- oder Abnahme der Calciumkonzentration innerhalb einer Zelle oder innerhalb eines Vesikels kann beispielsweise unter Verwendung von ionenseletiven Indikatorfarbstoffen geführt werden.

Trotz der grossen Bedeutung dieser Proteine fehlt es an effizienten Screeningassays, um einerseits ein Verständnis der Rezeptorfunktionen und Signaltransduktionsprozesse auf molekularer Ebene zu erlangen, andererseits neue therapeutische Wirkstoffe ("drugs") zu entdecken und zu entwickeln. Traditionelle Untersuchungsmethoden basieren auf Assays (1) mit ganzen Zellen, (2) mit solubilisierten und gereinigten Rezeptoren oder (3) mit in künstlichen Lipidmembranen rekonstituierten Rezeptoren (Fernandes P. B., Curr. Opin. Chem. Biol., 2 (1998) 597-603; Gonzalez J. E., Negulescu P. A., Curr. Opin. Biotechnol., 9 (1998) 624-631; Zysk J. R., Baumbach W. R., Comb. Chem. High Throughput Screen., 1 (1998) 171-183).

Vesikel wurden in früheren Zeiten vor allem in der Pharmakologie zur Verabreichung von Medikamenten in einem Organismus beschrieben. So werden beispielsweise in US-Patent 4877619 künstliche liposomale multilamellare Vesikel mit Phosphatidylchlinen mit Fettsäure-Seitenketten und darin einzukapselnden therapeutischen Substanzen beschrieben. Derartige Arbeiten waren ausschließlich auf die Medikamentenverabreichung und auf keinerlei analytische Untersuchungen ausgerichtet.

In der internationalen Anmeldung WO 01/82958 werden Verfahren zur Herstellung von Exosomen, d.h. innerhalb einer Zelle gebildeten Vesikeln, beschrieben. Exosomen enthalten an ihrer Oberfläche keine Zelloberflächenrezeptoren und da sie sich aus endosomalen Membranen formieren, auch keine Proteine oder andere Moleküle die an intrazellulären Signaltransduktionskaskaden beteiligt sind. Exosomen sind daher nicht geeignet für Versuche zur Untersuchung der Funktionsfähigkeit von Signaltransductionsprozessen, die durch Plasmamembranrezeptoren ausgelöst werden.
(1) In vivo Screening-Methoden verwenden geeignete lebende biologische Zellen, welche natürliche oder heterologe Rezeptorproteine an der Zelloberfläche exprimieren. In einem Assay werden definierte Konzentrationen von Testverbindungen zu der wässrigen Phase in der Umgebung der lebenden Zellen hinzugegeben, um zu untersuchen, ob diese Verbindungen sich in spezifischer Weise an Rezeptorproteine in der Zellmembran (Plasmamembran) anlagern und zum Beispiel eine zelluläre Antwort stimulieren oder inhibieren. Typische zelluläre Antworten sind Änderungen der intrazellulären Ioneizzusammensetzung (z. B. der Na⁺- oder K⁺-Konzentrationen oder des pH), das Auslösen sekundärer Signalkaskaden (z. B. durch Freisetzung von cAMP (cyklisches Adenosinmonophosphat), oder Ca²⁺ ) oder Änderungen der Aktivierung intrazellulärer Enzyme (z. B. von Kinasen, Phosphatasen, etc.). Dabei können auch einige oder alle der genannten Prozesse gleichzeitig und / oder miteinander gekoppelt auftreten. Aus der Art, Stärke und Zeitabhängigkeit der intrazellulären Antworten lassen sich wichtige Informationen über die Wechselwirkung zwischen der Testverbindung und dem Plasmamembran-Rezeptor sowie den nachfolgenden Signaltransduktionsprozess gewinnen. Die Bindung einer Testverbindung an die Zelloberfläche wird im allgemeinen unter Verwendung eines direkt oder indirekt mit dieser Verbindung assoziierten Labels gemessen, oder in einem kompetitiven Assay unter Verwendung eines gelabelten Kompetitors (Smith R. G., Sestili M. A., Clin. Chem., 26 (1980) 543-550; Zuck P., Lao Z., Skwish S., Glickman J. F., Yang K., Burbaum J., Inglese J., Proc. Natl. Acad. Sci. U S A, 96 (1999) 11122 - 11127). Dabei kann beispielsweise untersucht werden, ob die Bindung der Testverbindung zu einer Rezeptoraktivierung führt.
   Eine Inhibition der Aktivierung wird im allgemeinen in Gegenwart eines bekannten Agonisten, anhand des Einflusses der Testverbindung auf die Aktivierung durch diesen Agonisten, untersucht. Derartige Standardmethoden sind z. B. beschrieben in: Fernandes P. B., Curr. Opin. Chem. Biol., 2 (1998) 597 - 603 und in Gonzalez J. E., Negulescu P. A., Curr. Opin. Biotechnol., 9 (1998) 624 - 631.
   Analytische Methoden unter Verwendung lebender Zellen haben eine Reihe von inhärenten Nachteilen: (I). Die erforderliche Labor-Infrastruktur für kontinuierliche Kultivierung von Zellen ist relativ aufwendig. (II). Lebende biologische Zellen verändern ständig ihre Eigenschaften aufgrund von Änderungen der Zellphysiologie. Diese Änderungen umfassen Unterschiede im Stadium des Zellwachstumszyklus, der Differenzierung und der Stärke der Protein-Expression, so dass die Gewährleistung reproduzierbarer, gleichwertiger Versuchsbedingungen in parallelen oder repetitiven Assays schwierig ist. (III). Ein weiterer Nachteil besteht darin, dass Möglichkeiten zur Minaturisierung von Assays basierend auf ganzen Zellen beispielsweise begrenzt sind durch die erforderlichen zuzuführenden Volumina von Nährmitteln.
   Im allgemeinen werden beim pharmakologischen Wirkstoffscreening noch relativ zeitaufwendige Ligandenbindungstests und Rezeptorfunktionstests getrennt voneinander durchgeführt (Hodgson J, Bio/Technology 9 (1992) 973). Andererseits zählen Membranproteine wie die G-Protein-gekoppelten Rezeptoren und die kanalbildenden Rezeptoren zu den wichtigsten Zielproteinen für aktive Wirkstoffe (Knowles J, "Medicines for the new millenium hunting down diseases", Odyssey Vol. 3 (1997)). In diesem Zusammenhang werden immer noch klassische Patch-clamp-Methoden als funktionelle Rezeptortests eingesetzt. Der Vorteil dieser elektrophysiologischen Methode liegt darin, dass die Funktion der entsprechenden an kanalbildende Proteine gekoppelten Rezeptoren über die gemessenen elektrischen Eigenschaften der kanalbildenden Proteine direkt messbar ist. Die Methode ist hochspezifisch und extrem empfindlich - man kann im Prinzip die Kanalaktivität einzelner Rezeptoren messen. Dabei werden Glasmikropipetten mit einem Öffnungsdurchmesser von typischerweise 0.1 - 10 µm auf die Oberfläche einer biologischen Zelle gesetzt. Die Membranfläche, die von der Mikropipette abgedeckt wird, nennt man "Patch". Wenn der Kontakt zwischen der Glaselektrode und der Zellmembranoberfläche elektrisch hinreichend isoliert ist, kann mit Hilfe von Mikroelektroden, die einerseits in der Glaspipette und andererseits im membrangegenüberliegenden Milieu plaziert sind, der Ionenstrom über den Membranpatch elektrisch gemessen werden (Hamill O. P., Marty A. et al., "Improved patch-clamp techniques for high-resolution current recording from cells and cell-free membrane patches", Pflügers Arch., 391 (1981) 85-100).
   Im Zusammenhang mit Wirkstoffscreening weist die traditionelle Patch-clamp-Technik allerdings auch entscheidende Nachteile auf. Patch-clamp- Messungen sind äusserst zeitaufwendig, erfordern speziell geschultes Personal mit langen Erfahrungen auf diesem Gebiet und sind für HTS ("High Throughput Screening") praktisch nicht einsetzbar.
   In letzter Zeit wurden automatische Verfahren entwickelt, die es erlauben, Arrays von Glasmikroelektroden auf geeignet angeordnete Zellen zu positionieren oder umgekehrt, Zellen oder Membranfragmente automatisch (in elektrischen Feldern oder durch geeignete Flussvorrichtungen) an über (sub-)mikrometer-grosse Öffnungen in festen Trägem zu positionieren, wie z.B. Glassplättchen, Silikonwafer, deren Oberflächen eventuell chemisch modifiziert sind (Vogel H., Schmidt C., "Positionierung und elektrophysiologische Charakterisierung einzelner Zellen und rekonstituierter Membransysteme auf mikrostrukturierten Trägem", PCT/IB98/01150, WO 99/31503; Schmidt C., Mayer M., Vogel H., "A chip-based biosensor for functional analysis of single ion channels", Angew. Chemie Int. Ed., 39 (2000) 3137 - 3140; Klemic K. G., Buck E. et al., "Quartz microchip partitions for improved planar lipid bilayer recording of single channel currents", Biophys. J. 266A; Klemic K. G., et al., "Design of a microfabricated quartz electrode for ultra-low noise patch clamp recording", Biophys. J. A399).
   Zusammenfassend ist jedoch feszustellen, dass in keiner dieser Methoden die Verwendung "nativer", d.h. aus einer lebenden Zelle hergestellter, Membranvesikel beschrieben ist.
(2) Assays mit in Detergentien solubilisierten Rezeptorproteinen sind aufwendig und setzen in der Regel die Aufreinigung aus einer natürlichen Quelle (Organismus) oder den Einsatz eines rekombinanten Expressionssystems voraus. Während dieses aufwendigen Verfahrens bleibt die Rezeptoraktivität im allgemeinen nur teilweise erhalten. Zusätzlich zur Rezeptorreinigung müssen Bindungsassays unter Verwendung fluoreszenter oder radioaktiver Markierungsmoleküle ("Labels") entwickelt werden. In derartigen Bindungsassays kann die Art der Wechselwirkung zwischen einem Agonisten oder Antagonisten und einem Rezeptor, jedoch nicht die Stimulierung oder Inhibition der Signaltransduktionskaskade, untersucht werden, was ein zentraler Bestandteil der Plasmamembran-Rezeptorfunktion ist.
(3) Andere traditionelle Methoden basieren auf in Lipidvesikeln rekonstituierten Rezeptoren. Dieses erfordert zunächst die Isolierung und Aufreinigung des jeweiligen Rezeptors in geeigneten Detergentien und anschliessend die Einfügung des in einem Detergens solubilisierten Rezeptors in künstliche oder "native" Lipiddoppelschicht-Vesikel. Die zeitaufwendige Aufreinigung setzt entweder rezeptorreiche natürliche Zellen oder rekombinante Über-Expression in Zellen voraus. Rekonstitutions-protokolle müssen individuell an den jeweiligen Rezeptor angepasst werden. Veränderungen der Zusammensetzung der Lipid- oder der wässrigen Phase können sich stark auf die Effizienz des Rezeptor-Einbaus auswirken. Bei der Rekonstitution in der Vesikelmembran kann die Orientierung des Rezeptors nicht kontrolliert werden.

Mit in Vesikeln rekonstituierten Rezeptoren können sowohl die Bindung von Testverbindungen an den Rezeptor als auch die Aktivierung von Ionenflüssen untersucht werden. Jedoch enthalten diese Vesikel nicht die Komponenten für die vollständige Signaltransduktionskaskade, wie z.B. G-Proteine, Proteinkinasen, Phosphatasen, Phosphoinosite oder Phospholipasen, welche nur schwer gemeinsam mit Rezeptoren in Vesikeln rekonstituiert werden können.

In Protein Science (1995), 4(12), 2532 - 2544 werden Modellstudien zur Untersuchung der Wechselwirkungen zwischen membrangebundenen Rezeptoren und ihren Liganden beschrieben. Hierzu wurden mit einem Lipidanker versehene Rezeptormoleküle in eine auf einem festen Träger aufgebrachte Doppellipidschichtmembran eingefugt. Die Doppellipidschichtmembranen wurden durch Spreiten von Micellen oder Lipid-Vesikeln auf dem festen Träger aufgebracht. Diese Methoden sind jedoch nicht geeignet für Versuche, in denen die Funktionsfähigkeit von Signaltransduktionsprozessen untersucht werden soll. Insbesondere sind die eingefügten Rezeptoren hier nicht in die Oberfläche eines Vesikels eingefügt, was der Einbettung oder Anbindung in eine Zelloberfläche nahekommen würde, sondern vermittelt über die Doppellipidschichtmembran an eine 2-dimensionelle Oberfläche gebunden.

Es besteht daher ein Bedarf nach einem bioanalytischen Reagens, in welchem Rezeptoren in einer solchen Form und einer solchen bio-kompatiblen Umgebung vorliegen, dass der vollständige, an den jeweiligen Rezeptor gekoppelte Signaltransduktionsmechanismus einem bioanalytischen Untersuchungsverfahren zugänglich ist und Änderungen der Rezeptorantwort bzw. der übrigen am Signaltransduktionsmechanismus beteiligten Komponenten, in Gegenwart verschiedener in einer Probe zugeführter biologischer oder biochemischer oder synthetischer Komponenten oder infolge von anderen Änderungen anderer auf den Transduktionsmechanismus einwirkender äusserer Parameter, getestet werden können. Dabei ist es von essentieller Bedeutung, die genannten Nachteile von Assays basierend auf ganzen lebenden Zellen zu vermeiden, d.h. die hohe Variabilität von Testergebnissen aufgrund der ständigen Veränderung lebender Organismen sowie die häufigen Zuordnungsschwierigkeiten von Ursache-Wirkungs-Beziehungen. Dieses ist begründet in dem komplexen Aufbau ganzer Zellen, die eine Vielzahl zusätzlicher biochemischer Komponenten beinhalten, welche am Signalübertragungsmechanismus eines Rezeptors möglicherweise nicht direkt beteiligt sind, aber in einem Testverfahren auch in ihrem Verhalten beeinflusst werden können, mit der Folge möglicher zusätzlicher Veränderungen beobachteter Testergebnisse.

Das Bedürfnis nach einem solchen Reagens wird erfindungsgemäss erfüllt durch die Bereitstellung eines bioanalytisches Reagens mit mindestens einem Vesikel, das aus einer lebenden Zelle erzeugt wird, umfassend mindestens einen Rezeptor, dadurch gekennzeichnet, dass ein von diesem Rezeptor in der verwendeten lebenden Zelle ausgelöster Mechanismus einer Signaltransduktion in besagtem. Vesikel als Bestandteil des bioanalytischen Reagens erhalten bleibt.

Ein wesentliches Kennzeichen einer Vielzahl bevorzugter Ausführungs-formen des erfindungsgemässen Reagens ist, dass ein Vesikel als Bestandteil dieses Reagens neben diesem Rezeptor auch weitere Zellprodukte bzw. Zellproteine umfasst, welche neben besagtem Rezeptor an dem genannten Mechanismus der Signaltransduktion beteiligt sind, beispielsweise in Form der Erhöhung oder Verringerung der Konzentration sekundärer Botenstoffe innerhalb des Vesikels. Vorzugsweise umfasst das Vesikel ausserdem weitere als Indikator geeignete Moleküle, welche ein in einem bioanalytischen Nachweisverfahren detektierbares Signal oder eine entsprechende detektierbare Signaländerung als Folge der Konzenztrationsänderung dieser sekundären Botenstoffe erzeugen.

In der WO 97/45534 wird ein Verfahren zur Kultivierung von Säugerzellen in Gegenwart von immobilisierten Vesikeln beschrieben, welche aus einem bestimmten Typ von Säugerzellen hergestellt wurden. Ähnlich wie dort beschrieben, wird auch in der vorliegenden Erfindung unter anderem Cytochalasin B zur Herstellung von nativen Vesikeln aus lebenden Säuger-zellen verwendet. In der WO 97/45534 werden jedoch keine Hinweise auf die Verwendung derartiger Vesikel in bioanalytischen Nachweisverfahren gegeben, insbesondere also nicht zur Untersuchung von Ligand-Rezeptor-Wechselwirkungen. Der Transduktionsmechanismus von gegebenenfalls mit den Vesikeln assoziierten Rezeptoren und dessen Beeinflussung durch das Kultivierungsverfahren liegt ausserhalb des Anwendungsgebietes, welches in der WO 97/45534 beschrieben wird, und wird darin in keiner Weise erörtert

Die Erzeugung von Vesikeln aus lebenden Zellen unter Zugabe von den Pilztoxinen Cytochalasin B oder D ist aus der Literatur bekannt (siehe z. B.: Henson J. H., "Relationships between the actin cytoskeleton and cell volume regulation", Microsc. Res. Tech., 47 (1999) 155 - 162; Brown S. S., Spudich J. A., "Mechanism of action of cytochalasin: evidence that it binds to actin filament ends", J. Cell. Biol., 88 (1981) 487 - 491; Atlas S. J., Lin S., "Dihydrocytochalasin B. Biological effects and binding to 3T3 cells", J. Cell. Biol., 76 (1978) 360 - 370).

Diese Methode der Präparation von sogenannten "nativen" Vesikeln ist sowohl anwendbar auf adhärente als auch auf in Suspension wachsende Zellen. Diese Pilztoxine wirken auf das Aktin-Zytoskelett ein. Das Zytoskelett ist ein dynamisches Netzwerk, das eine Reihe von essentiellen biologischen Funktionen in der Zelle wahrnimmt, wie z.B. Zellteilung, Regulation des Zellvolumens, Änderung der Zellform und Zellbewegung. Es wurde gezeigt, dass Cytochalasin B oder D an das Polymerisationsende von Aktinfilamenten binden und ihre Verlängerung durch die Blockierung der Anlagerung von weiteren globulären Aktinmonomeren verhindern.

Nach Zugabe von Cytochalasin runden sich die Zellen ab. Mikrofilamente ziehen sich zusammen und verdichten zu lokalen Aggregationen im Zellcortex. Das ansonsten durchgängige Aktin-Zytoskelett bekommt Lücken. Dieser Effekt führt dazu, dass das Endoplasma sich in diesen Regionen ausdehnt, unterstützt durch den cytoplasmatischen Druck. Als Folge kommt es an diesen Stellen zu Ausbuchtungen der Zellmembran. Diese Ausbuchtungen sind entweder blasen- oder füsschenartig. Sie schnüren sich, teilweise ohne zusätzliche äussere Einwirkung, als Vesikel ab oder können durch die Anwendung von Scherkräften von der Zelloberfläche abgelöst werden.

In einer jüngeren Publikation (Kask P., Palo K., Fay N., Brand L., Mets U., Ullmann D., Jungmann J., Pschorr J., Gall K., "Two-dimensional fluorescence intensity distribution analysis: theory and applications", Biophys. J., 78 (2000) 1703 - 1713) wird die Bindung fluoreszenter Liganden an einen menschlichen, hochaffinen Somatostatin-Rezeptor (SSTR-2) mittels Fluoreszenzmessungen mit aus Zellen hergestellten Vesikeln gezeigt (Schoeffter P., Perez J., Langenegger D., Schupbach E., Bobirnac I., Lubbert H., Bruns C., Hoyer D., "Characterization and distribution of somatostatin SS-1 and SRIF-1 binding sites in rat brain: identity with SSTR-2 receptors" , Eur. J. Pharmacol., 289 (1995) 163 - 173). Für diese Studie wurden Membranvesikel aus lebenden Zellen mit einem Glas-Homogenisierer hergestellt. Bei diesem Verfahren werden die Zellen aufgebrochen und mit grösster Wahrscheinlichkeit die Signaltransduktionskaskade, d. h. vor allem weitere für die Signaltransduktion wesentliche Zellproteine, zerstört. Neben Plasmamembranbasierenden Vesikeln werden dabei auch cytoplasmische Vesikel hergestellt. Eine nachträgliche Trennung dieser Vesikel unterschiedlicher Herkunft ist nicht möglich. - Im Spezialfall des Somatostatin-Rezeptors mag die Trennung der Vesikeltypen nicht entscheidend für den Ausgang eines Experiments sein, da Somatostatin-Rezeptoren durch Bindung mit Agonisten ins Vesikelinnere überführt und dort mittels Zugabe von Antikörpern lokalisiert werden können (Rocheville M., Lange D. C., Kumar U., Sasi R., Patel R. C., Patel Y. C., "Subtypes of the somatostatin receptor assemble as functional homo- and heterodimers", J. Biol. Chem,. 275 (2000) 7862 - 7869). Für Anwendungen mit anderen Rezeptoren in nur sehr niedriger natürlicher Konzentration ist demgegenüber eine Anreicherung des Anteils von Vesikeln aus der Plasmamembran wesentlich.

Das kontrollierte Verfahren zur Vesikelherstellung, gemäss unserer vorliegenden Erfindung, erhält im Gegensatz zu obigen Methoden die Komponenten der Signaltransduktionskaskade, d.h. insbesondere ausser einem Rezeptor auch die an der Signaltransduktion beteiligten Zellproteine, und deren Funktion intakt.

Der besagte Zell-Homogenisierungsprozess lässt sich durch die Zugabe von Protease-Inhibitoren beeinflussen. Die Protease-Inhibitoren verhindern in der Regel den enzymatischen Abbau der Membranrezeptoren durch freigesetzte Zell-Proteasen. Das Verfahren zur Herstellung des erfindungsgemässen bioanalytischen Reagens, unter Herstellung von Vesikeln mit Cytochalasin B, vermeidet die externe Zugabe von Protease-Inhibitoren. Dieses kann vorteilhaft sein, da zusätzliche Komponenten (in diesem Falle meist Gemische von Protease-Inhibitoren) in einer Testlösung möglicherweise die Rezeptor-Liganden-Wechselwirkung beeinflussen können.

Die vorliegende Erfindung erlaubt die Übertragung der traditionellen Assays und Nachweismethoden zur Untersuchung der Rezeptor-Liganden-Wechselwirkung und der nachfolgenden Signaltransduktion auf ein miniaturisiertes Format. Erfindungsgemäss werden die hergestellten Vesikel als Behältnisse für die Rezeptoren benutzt, wobei diese Behältnisse zusätzlich noch wesentliche Zellproteine enthalten können, die auch rekombinant aus derselben Zelle wie die zu erzeugenden Vesikel hergestellt werden können.

Diese Vesikel als von Zellen abgeleitete, aber zellunabhängige Behältnisse können zur Aufbewahrung eingefroren werden, so dass über einen längeren Zeitraum eine gleichbleibende Qualität der sequentiell benutzten Reagentien-Teilmengen eines solchen Herstellungs-Loses gewährleistet werden kann.

Die Vesikel umfassen beispielsweise entweder Behältnisse für cytosolische, rekombinant hergestellte Produkte (z. B. GFP, "Green Fluorescent Protein") oder werden beispielsweise eingesetzt als Träger für überexprimierte Rezeptoren (z. B. Serotoninrezeptor Typ 3, 5HT_{3A}). Erfindungsgemäss können die Vesikel auch für andere rekombinant hergestellte Produkte als Behältnisse dienen, wenn sie im Cytosol löslich sind oder Zelloberflächenrezeptoren umfassen.

Zur Erfüllung des Bedarfs an immer mehr Informationen über die intra- und extrazellulären biologischen Wechselwirkungen auf molekularer Ebene besteht ein Bedürfnis nach schnellen, parallelen und höchstempfindlichen analytischen Methoden mit minimalem Probenverbrauch.

Zur Bestimmung einer Vielzahl von Analyten sind gegenwärtig vor allem Verfahren verbreitet, in denen in sogenannten Mikrotiterplatten der Nachweis unterschiedlicher Analyten in diskreten Probenbehältnissen oder "Wells" dieser Platten erfolgt. Am weitesten verbreitet sind dabei Platten mit einem Raster von 8 x 12 Wells auf einer Grundfläche von typischerweise ca. 8 cm x 12 cm (siehe beispielsweise Corning Costar-Katalog Nr. 3370, 1999), wobei zur Füllung eines einzelnen Wells ein Volumen von einigen hundert Mikrolitern erforderlich ist. Für zahlreiche Anwendungen wäre es jedoch wünschenswert, das Probenvolumen deutlich zu verringern, nicht nur zur Verminderung des Bedarfs an gegebenenfalls nur in kleinen Mengen verfügbaren Proben und an Reagentien, sondern insbesondere auch zur Verringerung der Diffusionswege und damit der Verkürzung der Assay-Durchführungszeiten im Falle von Assays, bei denen biologische oder biochemische oder synthetische Erkennungselemente zur Erkennung eines oder mehrerer Analyten in einer Probe an einer Begrenzungswand der Probenbehältnisse immobilisiert sind.

Zur Verringerung der Probenvolumina und zur Erhöhung des Probendurch-satzes, vor allem für Screening-Verfahren, sind in den vergangenen Jahren einerseits, unter Beibehaltung der Grundmasse dieser Standard-Mikrotiter-platten, Platten höherer Dichte (16 x 24 Wells = 384 Wells und 32 x 48 Wells = 1536 Wells) entwickelt und auf den Markt gebracht worden. Dieser Ansatz erlaubt es, auf den etablierten Industriestandard angepasste Labor-roboter weitgehend beizubehalten (abgesehen von der erforderlichen höheren Dichte der Adressierpunkte zur Reagentienzugabe auf die Platten). Ein anderer Ansatz bestand darin, die klassische Plattengrundfläche aufzugeben und die Grösse der einzelnen Wells ausschliesslich nach den für eine bestimmte Anwendung notwendigen Probenvolumina auszurichten. Derartige Anordnungen wurden unter der Bezeichnung "Nanotiterplatten" bekannt, deren individuelle Probenbehältnis-Volumina teilweise nur noch einige Nanoliter betragen. Diese technische Lösung erfordert jedoch die Abkehr von den derzeit weit gebräuchlichen Laborrobotem, welche auf den klassischen Mikrotiterplattenstandard ausgelegt sind, und die Entwicklung einer neuen, für die miniaturisierten Formate optimierten Labor-Infra-struktur. Der damit verbundene zusätzliche Aufwand ist wahrscheinlich ein Hauptgrund dafür, dass sich diese neuen miniaturisierten Formate bisher am Markt noch nicht durchsetzen konnten.

In derartigen Verfahren zum Nachweis von Analyten in einer oder mehreren Proben sind in der Vergangenheit in zunehmendem Masse optische Methoden, beispielsweise basierend auf der Bestimmung der Änderung einer Absorption oder einer Lumineszenz, entwickelt worden, da solche Methoden berührungslos und ohne stärkere Rückwirkung auf die Probe selbst durch-geführt werden können. Mit dem Begriff "Lumineszenz" wird dabei in dieser Anmeldung die spontane Emission von Photonen im ultravioletten bis infraroten Bereich nach optischer oder nichtoptischer, wie beispielsweise elektrischer oder chemischer oder biochemischer oder thermischer Anregung, bezeichnet. Beispielsweise sind Chemilumineszenz, Biolumineszenz, Elektrolumineszenz und insbesondere Fluoreszenz und Phosphoreszenz unter dem Begriff "Lumineszenz" mit eingeschlossen.

Die klassischen Messmethoden, wie beispielsweise Absorptions- oder Fluoreszenzmessungen, beruhen im allgemeinen auf der direkten Beleuchtung eines Probevolumens in einem Probenbehältnis oder eines Messfeldes auf einer Innenwand eines Probenbehältnisses einer flüssigen Probe. Diese Anordnungen haben den Nachteil, dass neben dem Anregungsvolumen oder der Anregungsfläche, innerhalb derer ein Signal zum Nachweis eines Analyten erzeugt werden soll, im allgemeinen ein erheblicher Anteil der Umgebung von Anregungslicht erfasst wird, was zur nachteiligen Erzeugung von störenden Untergrundsignalen führen kann.

Mit zunehmender Miniaturisierung der Probenbehältnisse wird der Anteil störenden Umgebungslichts, insbesondere durch Reflexionen oder Lumineszenz von den Randflächen dieser Behältnisse, noch vergrössert, da mit der Verkleinerung des Beobachtungsvolumens der relative Anteil der Beiträge der Oberflächen am Gesamtsignal ansteigt. Zugleich verringert sich das erreichbare Probensignal proportional mit dem Probenvolumen.

Zur Verbesserung des Verhältnisses zwischen Messignal von möglicher-weise nur noch wenigen nachzuweisenden Analytmolekülen in einer Probe und dem störenden Umgebungssignal sind in der Vergangenheit im wesent-lichen zwei unterschiedliche Ansätze verfolgt worden. Der eine bestand darin, das Beobachtungsvolumen auf diese wenigen Moleküle einzuengen, der andere in der Beschränkung des Nachweises auf die Oberfläche, an der eine biologische oder biochemische Wechselwirkung stattfindet.

Der erste der beiden genannten Ansätze beruht auf dem Einsatz konfokaler Mikroskopie. Als Beispiel ist die von Eigen und Rigler entwickelte Fluoreszenzkorrelations-Spektroskopie (FCS) zu nennen, mit der der Nachweis einzelner Moleküle gelang. Mit dieser Technik wurden beispielsweise Signalproteine in einzelnen Zellen untersucht (Cluzel P., Surette M., Leibler S., "An ultrasensitive bacterial motor revealed by monitoring signaling proteins in single cell", Science, 287 (2000) 1652 - 1655). Da es sich bei derartigen Untersuchungen um die Bestimmung individueller Vorgänge an einzelnen Zellen oder Molekülen handelt, sind für eine quantitative analytische Aussage mit statistischer Relevanz jedoch eine Vielzahl von Einzelmessungen notwendig, was - trotz der hohen Empfindlichkeit dieser Methoden für den Nachweis einzelner Moleküle - gesamthaft betrachtet mit einem hohen Zeitaufwand verbunden ist.

Dieser Nachteil kann mit Hilfe des zweiten Ansatzes, durch einen räumlich selektiven Analytnachweis auf einer makroskopischen Wechselwirkungs-fläche, vermieden werden.

Diesem Ansatz folgend, sind zahlreiche Messanordnungen entwickelt worden, in denen der Nachweis des Analyten auf dessen Wechselwirkung mit dem evaneszenten Feld beruht, welches mit der Lichtleitung in einem optischen Wellenleiter verbunden ist, wobei auf der Oberfläche des Wellen-leiters biochemische oder biologische oder synthetische Erkennungs-elemente zur spezifischen Erkennung und Bindung der Analytmoleküle immobilisiert sind. Koppelt man eine Lichtwelle in einen optischen Wellenleiter ein, der von optisch dünneren Medien, d.h. Medien mit niedrigerem Brechungsindex, umgeben ist, so wird sie durch Totalreflexion an den Grenzflächen der wellenleitenden Schicht geführt. In die optisch dünneren Medien tritt dabei ein Bruchteil des geführten Lichts ein. Diesen Anteil bezeichnet man als evaneszentes oder quergedämpftes Feld. Die Stärke des evaneszenten Feldes ist sehr stark abhängig von der Dicke der wellenleitenden Schicht selbst sowie vom Verhältnis der Brechungsindices der wellenleitenden Schicht und der sie umgebenden Medien. Bei dünnen Wellenleitern, d. h. Schichtdicken von derselben oder niedrigerer Dicke als der zu führenden Wellenlänge, können diskrete Moden des geleiteten Lichts unterschieden werden. Derartige Verfahren haben den Vorteil, dass die Wechselwirkung mit dem Analyten auf die Eindringtiefe des evaneszenten Feldes ins angrenzende Medium, in der Grössenordnung von einigen hundert Nanometern, beschränkt ist und Störsignale aus der Tiefe des Mediums weitgehend vermieden werden können. Die ersten vorgeschlage-nen Messanordnungen mit optischen Wellenleitern beruhten auf hochmulti-modalen, selbsttragenden Einschichtwellenleitern, wie beispielsweise Fasern oder Plättchen aus transparentem Kunststoff oder Glas, mit Stärken von einigen hundert Mikrometern bis zu mehreren Millimetern.

Zur weiteren Verbesserung der Empfindlichkeit und gleichzeitig einfacheren Herstellung in Massenfabrikation wurden in den Folgejahren planare Dünn-schichtwellenleiter verwendet. Ein planarer Dünnschichtwellenleiter besteht im einfachsten Fall aus einem Dreischichtsystem: Trägermaterial (b), wellenleitende Schicht (a), angrenzendes Medium (bzw. zu üntersuchende Probe), wobei die wellenleitende Schicht den höchsten Brechungsindex besitzt. Zusätzliche Zwischenschichten können die Wirkung des planaren Wellenleiters noch verbessern.

Es sind verschiedene Verfahren für die Einkopplung von Anregungslicht in einen planaren Wellenleiter bekannt. Die am frühesten benutzten Verfahren beruhten auf Stimflächenkopplung oder Prismenkopplung, wobei zur Verminderung von Reflexionen infolge von Luftspalten im allgemeinen eine Flüssigkeit zwischen Prisma und Wellenleiter aufgebracht wird. Diese beiden Methoden sind vor allem in Verbindung mit Wellenleitern relativ grosser Schichtdicke, d. h. insbesondere selbsttragenden Wellenleitern, sowie bei einem Brechungsindex des Wellenleiters von deutlich unter 2 geeignet. Zur Einkopplung von Anregungslicht in sehr dünne, hoch-brechende wellenleitende Schichten ist demgegenüber die Verwendung von Koppelgittern eine wesentlich elegantere Methode.

Es können verschiedene Methoden zum Analytnachweis im evaneszenten Feld geführter Lichwellen in optischen Schichtwellenleitem unterschieden werden. Aufgrund des eingesetzten Messprinzips kann man beispielsweise zwischen Fluoreszenz- oder allgemeiner Lumineszenzmethoden auf der einen Seite und refraktiven Methoden andererseits unterscheiden. Hierbei können Verfahren zur Erzeugung einer Oberflächenplasmonenresonanz in einer dünnen Metallschicht auf einer dielektrischen Schicht mit niedrigerem Brechungsindex in die Gruppe der refraktiven Methoden mit einbezogen werden, sofern als Basis zur Bestimmung der Messgrösse der Resonanzwinkel des eingestrahlten Anregungslichts zur Erzeugung der Oberflächenplasmonenresonanz dient. Die Oberflächenplasmonenresonanz kann aber auch zur Verstärkung einer Lumineszenz oder zur Verbesserung des Signal-zu-Hintergrund-Verhältnisses in einer Lumineszenzmessung verwendet werden. Die Bedingungen zur Erzeugung einer Oberflächen-plasmonenresonanz sowie zur Kombination mit Lumineszenzmessungen sowie mit wellenleitenden Strukturen sind vielfach in der Literatur beschrieben, beispielsweise in den US-Patenten US-P 5,478,755, US-P 5,841,143, US-P 5,006,716 und US-P 4,649,280.

Bei den refraktiven Messmethoden wird die Änderung des sogenannten effektiven Brechungsindex aufgrund molekularer Adsorption oder Desorption auf dem Wellenleiter, zum Nachweis des Analyten benutzt. Diese Änderung des effektiven Brechungsindex wird, im Falle von Gitterkoppler-Sensoren, bestimmt aus der Änderung des Koppelwinkels für die Ein- oder Auskopplung von Licht in oder aus dem Gitterkoppler-Sensor, und im Falle von interferometrischen Sensoren aus der Änderung der Phasendifferenz zwischen dem in einem Sensorarm und einem Referenzarm des Interferometers geführten Messlichts.

Die genannten refraktiven Methoden haben den Vorteil, dass sie ohne Verwendung zusätzlicher Markierungsmoleküle, sogenannter molekularer Labels, eingesetzt werden können. Der Nachteil dieser labelfreien Methoden ist jedoch, dass die damit erzielbaren Nachweisgrenzen aufgrund der geringen Selektivität des Messprinzips, in Abhängigkeit von dem Molekulargewicht des Analyten auf pico- bis nanomolare Konzentrationsbereiche beschränkt sind, was für viele Anwendungen der modernen Bioanalytik, beispielsweise für diagnostische Applikationen, nicht ausreichend ist.

Zur Erreichung tieferer Nachweisgrenzen erscheinen lumineszenzbasierende Methoden aufgrund grösserer Selektivität der Signalerzeugung besser geeignet. Dabei ist die Lumineszenzanregung auf die Eindringtiefe des evaneszenten Feldes in das optisch dünnere Medium, also auf die unmittel-bare Umgebung des wellenleitenden Bereichs mit einer Eindringtiefe in der Grössenordnung von einigen hundert Nanometern ins Medium beschränkt. Dieses Prinzip wird evaneszente Lumineszenzanregung genannt.

Mittels hochbrechender Dünnschichtwellenleiter, in Kombination mit Lumineszenzdetektion, basierend auf einem nur einige hundert Nanometer dünnen wellenleitenden Film auf einem transparenten Trägermaterial, konnte in den letzten Jahren die Empfindlichkeit deutlich gesteigert werden. Beispielsweise wird in der WO 95/33197 eine Methode beschrieben, in der das Anregungslicht über ein Reliefgitter als diffraktives optisches Element in den wellenleitenden Film eingekoppelt wird. Die isotrop abgestrahlte Lumineszenz in der Eindringtiefe des evaneszenten Feldes befindlicher lumineszenzfähiger Substanzen wird mittels geeigneter Messvorrichtungen, wie zum Beispiel Photodioden, Photomultiplier oder CCD-Kameras, gemessen. Es ist auch möglich, den in den Wellenleiter rückgekoppelten Anteil der evaneszent angeregten Strahlung über ein diffraktives optisches Element, zum Beispiel ein Gitter, auszukoppeln und zu messen. Diese Methode ist zum Beispiel in der WO 95/33198 beschrieben. Dabei können Ein- und Auskoppelgitter auch identisch sein, da, aufgrund der Umkehrbar-keit des Lichtwegs, jedes Einkoppelgitter unter den gleichen Bedingungen wie für die Einkopplung auch als Auskoppelgitter verwendet werden kann.

Zur gleichzeitigen oder aufeinanderfolgenden Durchführung von aus-schliesslich lumineszenzbasierenden Mehrfachmessungen mit im wesent-lichen monomodalen, planaren anorganischen Wellenleitern sind, z. B. in der WO 96/35940, Vorrichtungen (Arrays) bekannt geworden, in denen auf einer Sensorplattform wenigstens zwei getrennte wellenleitende Bereiche angeordnet sind, so dass das in einem wellenleitenden Bereich geführte Anregungslicht von anderen wellenleitenden Bereichen getrennt ist. Mit einer solchen Anordnung ist es insbesondere auch möglich, unterschiedliche Analyten mittels verschiedener, in diskreten Messbereichen (d) immobilisierter Erkennungselemente, gleichzeitig in einer zugeführten Probe nachzuweisen.

Im Sinne der vorliegenden Erfindung sollen räumlich getrennte Messberei-che (d) durch die geschlossene Fläche definiert werden, die dort immobili-sierte biologische oder biochemische oder synthetische Erkennungselemente zur Erkennung eines Analyten aus einer flüssigen Probe einnehmen. Diese Flächen können dabei eine beliebige Geometrie, beispielsweise die Form von Punkten, Kreisen, Rechtecken, Dreiecken, Ellipsen oder Streifen, haben.

Für die Untersuchung von Rezeptor-Liganden-Wechselwirkungen, insbeson-dere der Funktionsfähigkeit eines vom Rezeptor gesteuerten Transduktions-mechanismus, besteht ein Bedürfnis nach einem derart ausgestalteten festen Träger, insbesondere nach einer Sensorplattform mit hoher Nachweis-empfindlichkeit, dass der Mechanismus der Signaltransduktion, insbeson-dere durch die Immobilisierung des Rezeptors auf einer festen Oberfläche, nicht beeinträchtigt wird. Verschiedene Ausführungsformen von festen Trägern bzw. Sensorplattformen werden im Rahmen dieser Erfindung bereitgestellt. Erster Gegenstand der Erfindung ist ein bioanalytisches Reagens mit mindestens einem Vesikel, das aus einer lebenden Zelle erzeugt wird; umfassend mindestens einen Rezeptor, dadurch gekennzeichnet, dass ein von diesem Rezeptor in der verwendeten lebenden Zelle ausgelöster Mechanismus einer Signaltransduktion in besagtem Vesikel als Bestandteil des bioanalytischen Reagens erhalten bleibt.

Der mit besagtem Vesikel assoziierte Rezeptor kann an die Vesikelmembran gebunden sein. Bevorzugt wird, wenn der Rezeptor in die Vesikelmembran integriert ist.

Dabei kann besagter Mechanismus der Signaltransduktion durch Einwirkung sowohl äusserer als auch vesikelinnerer Signale oder signalauslösende, gegebenenfalls von ausserhalb zugegebene, biologische oder biochemische oder synthetische Komponenten ausgelöst werden. Als "äussere oder vesikelinnere Signale" werden dabei beispielsweise Änderungen makroskopischer Eigenschaften, wie Änderungen von Ionenkonzentrationen im Medium oder im Vesikel, verstanden. Demgegenüber werden unter "signalauslösenden biologischen oder biochemischen oder synthetischen Komponenten" beispielsweise spezifisch an einen Rezeptor bindende Liganden verstanden.

Ein wesentliches Kennzeichen einer Vielzahl bevorzugter Ausfiihrungsfor-men des erfindungsgemässen Reagens ist, dass ein Vesikel als Bestandteil dieses Reagens neben dem mindestens einem Rezeptor auch weitere Zellprodukte bzw. Zellproteine umfasst, welche neben besagtem Rezeptor an dem besagten Mechanismus der Signaltransduktion beteiligt sind, beispielsweise in Form der Erhöhung oder Verringerung der Konzentration sekundärer Botenstoffe innerhalb des Vesikels.

Das besagte mindestens eine Vesikel als Bestandteil des erfindungsge-mässen bioanalytischen Reagens kann aus einer eukaryotischen Zelle oder aus einer Zelle eines nativen Gewebes erzeugt werden.

Es wird bevorzugt, dass das Innere des besagten mindestens einen Vesikels frei von Zellkernmaterial (chromosomaler DNA) ist, so dass innerhalb besagten Vesikels keine replikativen Prozesse auftreten. Dieses ist ein wichtiger Aspekt bei Applikationen mit "nativen" Vesikeln, die frei von Fremd-DNA sein sollen (z.B. beim Einbringen von Vesikeln als Carrier in einen anderen Zielorganismus).

Für viele Anwendungen des erfindungsgemässen bioanalytischen Reagens wird bevorzugt, dass das besagte mindestens eine Vesikel einen Durchmes-ser von 50 nm - 5000 nm hat. Besonders bevorzugt wird ein Durchmesser von 100 nm - 2000 nm.

Kennzeichen vieler Ausführungsformen des erfindungsgemässen bioanaly-tischen Reagens ist, dass besagter mindestens eine Rezeptor in besagtem mindestens einem Vesikel, als Bestandteil des bioanalytischen Reagens, in natürlicher Form vorliegt.

Für andere Anwendungen wird bevorzugt, dass besagter mindestens eine Rezeptor in besagtem mindestens einem Vesikel, als Bestandteil des bio-analytischen Reagens, in modifizierter Form vorliegt. Zum Beispiel kann besagter Rezeptor als Fusionsprotein vorliegen, beispielsweise durch Fusion mit einem fluoreszenten Protein wie GFP (Green Fluorescent Protein; Tsien R. Y., "The green fluorescent protein", Annu. Rev Biochem 67 (1998) 509-544) oder BFP (blue fluorescent protein) oder RFP (red fluorescent protein).

Für viele Ausführungsformen ist auch kennzeichnend, dass besagter mindestens ein Rezeptor in besagtem mindestens einem Vesikel als Bestandteil des bioanalytischen Reagens in rekombinant hergestellter Form vorliegt.

Ein wesentliches Kennzeichen des erfindungsgemässen bioanalytischen Reagens ist, dass eine in der Vesikel-erzeugenden lebenden Zelle vorhande-ne Bindungsfähigkeit des besagten mindestens einen Rezeptors, welcher mit dem Vesikel als Teil des bioanalytischen Reagens assoziiert ist, zu einem spezifischen Liganden, erhalten bleibt.

Der mindestens eine Rezeptor kann ausgewählt sein aus der Gruppe von signalübertragenden Rezeptoren, die von Plasmamembranrezeptoren, wie beispielsweise Ionenkanalrezeptoren, G-Protein-gekoppelten Rezeptoren (GPCR), Orphan-Rezeptoren, Enzym-gekoppelten Rezeptoren, wie z. B. Rezeptoren mit intrinsischer Tyrosine-Kinase Aktivität, Rezeptoren mit intrinsischer Serine/Threonine-Kinase Aktivität, des weiteren von Rezeptoren für Wachstumsfaktoren (Peptid-Hormon-Rezeptoren), Rezeptoren für chemotaktische Substanzen, wie beispielsweise der Klasse der Chemokine-Rezeptoren, und schliesslich intrazellulären Hormon- Rezeptoren, beispielsweise Steroidhormon-Rezeptoren, gebildet wird.

Ausführungsformen des erfindungsgemässen bioanalytischen Reagens sind dadurch gekennzeichnet, dass sich besagter mindestens eine Rezeptor in Kontakt mit der äusseren Membran des Vesikels befindet. Dann wird bevorzugt, dass die Flächendichte, bezogen auf die Oberfläche der äusseren Vesikelmembran, von mit der äusseren Membran eines Vesikels in Kontakt befindlichen Rezeptoren von gleicher Grössenordnung oder grösser als die entsprechende Dichte dieser Rezeptoren in der Vesikel-erzeugenden lebenden Zelle ist.

Es wird bevorzugt, dass besagtes mindestens eine Vesikel, als Bestandteil des erfindungsgemässen bioanalytischen Reagens, neben besagtem mindestens einem Rezeptor weitere biologische Verbindungen (Komponenten) aus der Gruppe umfasst, die von beispielsweise G-Proteinen und G-Protein-Regulatoren (z.B. rasGAP), Enzymen, wie beispielsweise Adenylatzyklasen, Phospholipasen, welche intrazelluläre sekundäre Botenstoffe (z.B. cAMP (zyklisches Adenosinmonophosphat), cGMP (zyklisches Guanosinmonophosphat), Diacylglycerol (DAG) oder Inositoltrisphosphat (IP3)) bilden, und Enzymen, wie beispielsweise Serin-, Threonin- und Tyrosin-Kinasen sowie Tyrosin-Phosphatasen, welche Proteine durch Phosphorylierung oder Dephosphorylierung aktivieren bzw. hemmen, gebildet wird.

Das erfindungsgemässe bioanalytische Reagens eignet sich auch zur Appli-kation in einem lebenden Organismus, beispielsweise um dort an einem spezifischen, vorbestimmten Ort bioanalytische Untersuchungen, z. B. mittels im Vesikel inkorporierter Indikatorstoffe, vorzunehmen. Beispielsweise für derartige Applikationen ist es von Vorteil, wenn mit der äusseren Membran des mindestens einen Vesikels biologische, biochemische oder synthetische Komponenten, wie beispielsweise Zelloberflächenproteine oder Zelloberflächenzucker assoziiert sind, welche dem Transport besagten Vesikels zu vorbestimmten Zielorten, wie beispielsweise Zellen und / oder Organen und / oder vorbestimmtem Gewebe in einem lebenden Organismus und / oder der Bindung an ein biologisches oder biochemisches oder synthetisches Erkennungselement, welches besagte biologische oder biochemische oder synthetische Komponenten spezifisch erkennt und bindet, dienen.

Zur Verminderung von unspezifischer Bindung eines Vesikels, als Teil eines erfindungsgemässen bioanalytischen Reagens, an eine mit dem Vesikel in Kontakt zu bringende Oberfläche, kann es vorteilhaft sein, wenn nach Erzeugung besagten Vesikels aus einer lebenden Zelle zusätzlich Lipide, welche beispielsweise hydrophile Polymere (wie z.B. Polyethylenglycole) enthalten, in die Vesikelmembran integriert werden. Vesikel mit oberflächenassoziierten Polymeren sind beispielsweise in der PCT/EP/00/04491 beschrieben.

Der besagte Mechanismus der Signaltransduktion, welcher in dem erfin-dungsgemässen bioanalytischen Reagens erhalten bleibt, kann aus der Grup-pe von Mechanismen stammen, die beispielsweise von Ionenleitung, G-Protein-Kopplung, Aktivierung oder Inhibition intravesikulärer Ionenkanäle, intravesikulärer Calciumfreisetzung, Proteinaktivierung durch enzymatische Phosphorylierung oder Dephosphorylierung (Kinase-Kaskaden; Phosphatasen) und Freisetzung bzw. enzymatischer Bildung von sekundären Botenstoffen, z.B. cAMP, cGMP oder Diacylglycerol (DAG), Inositoltrisphosphat (IP3), gebildet wird.

Der besagte Mechanismus der Signaltransduktion kann eine (sekundäre) Funktionsantwort des mit dem Vesikel assoziierten mindestens einen Rezeptors nach einer primären spezifischen Wechselwirkung besagten Rezeptors mit einem oder mehreren in einer Probe, welche mit besagtem Vesikel in Kontakt gebracht wird, enthaltenen natürlichen und / oder synthetischen Liganden umfassen.

Der Mechanismus der Signaltransduktion kann auch die Aktivierung eines Ionenkanals eines mit einem Vesikel, als Teil des besagten bioanalytischen Reagens, assoziierten Rezeptors umfassen.

Es ist auch möglich, dass besagter Mechanismus der Signaltransduktion die Bindung eines G-Proteins an einen mit einem Vesikel, als Teil des besagten bioanalytischen Reagens, assoziierten Rezeptors umfaßt.

Weiterhin kann besagter Mechanismus der Signaltransduktion die interne Freisetzung von Ionen, wie beispielsweise Ca²⁺, oder anderer Botenstoffe, wie beispielsweise cAMP oder cGMP umfassen.

Der Mechanismus der Signaltransduktion kann auch den enzymatischen Abbau eines Substrats durch ein vesikelassoziiertes Enzym in ein Produkt umfassen. Dabei kann sich ein besagtes Enzym im Vesikelinnern befinden oder mit der Vesikelmembran assoziiert sein.

Eine spezifische Ausführungsform des erfindungsgemässen bioanalytischen Reagens ist dadurch gekennzeichnet, dass eine (sekundäre) Funktionsant-wort als Teil des besagten Mechanismus der Signaltransduktion nach Wechselwirkung von einem oder mehreren in einer Probe, welche mit besagtem Vesikel in Kontakt gebracht wird, enthaltenen natürlichen und / oder synthetischen Liganden oder Cofaktoren mit natürlichen oder rekombinant hergestellten, mit dem Vesikel assoziierten, Proteinprodukten, erfolgt.

Es wird bevorzugte, dass besagtes mindestens eine Vesikel zusätzlich Komponenten zur Erzeugung eines experimentell detektierbaren Signals umfasst.

Diese zusätzlichen Komponenten zur Erzeugung eines experimentell detektierbaren Signals können mit in dem mindestens einen Vesikel als Bestandteil des besagten bioanalytischen Reagens assoziierten weiteren biologischen Verbindungen (Komponenten) assoziiert sein.

Dabei können besagte weitere biologische Verbindungen (Komponenten) aus der Gruppe stammen, die von beispielsweise G-Proteinen und G-Protein-Regulatoren (z.B. rasGAP), Enzymen, wie beispielsweise Adenylatzyklasen, Phospholipasen, welche intrazelluläre sekundäre Botenstoffe (z.B. cAMP (zyklisches Adenosinmonophosphat), cGMP (zyklisches Guanosinmonophosphat), Diacylglycerol (DAG) oder Inositoltrisphosphat (IP3)) bilden, und Enzymen, wie beispielsweise Serin-, Threonin- und Tyrosin-Kinasen sowie Tyrosin-Phosphatasen, welche Proteine durch Phosphorylierung oder Dephosphorylierung aktivieren bzw. hemmen, gebildet wird:

Die besagten zusätzlichen Komponenten zur Erzeugung eines experimentell detektierbaren Signals können auch mit einem Rezeptor assoziiert sein oder Bestandteile von mit dem Vesikel assoziierten Fusionsproteinen sein.

Besagte zusätzliche Komponenten zur Erzeugung eines experimentell detektierbaren Signals können ausgewählt sein aus der Gruppe von Komponenten, die von absorptiven und von lumineszenten Indikatoren, Lumineszenzlabeln, lumineszenten Nanopartikeln, von absorptiven und von lumineszenten Indikatorproteinen, wie beispielsweise BFP ("blue fluorescent protein"), GFP ("green fluorescent protein") oder RFP ("red fluorescent protein"), nicht-natürlichen lumineszenten (d.h. insbesondere fluoreszenten) Aminosäuren, radioaktiven Labeln, Spin-Labeln, wie beispielsweise NMR-Labeln oder ESR-Labeln, Ionenindikatoren, insbesondere pH- und Calcium-Indikatoren, oder potentialabhängigen Indikatoren, wie beispielsweise potentialabhängigen Lumineszenzlabeln, oder Redox-Komplexen gebildet wird.

Eine bevorzugte Ausführungsform des erfindungsgemässen bioanalytischen Reagens ist dadurch gekennzeichnet, dass besagte zusätzliche Komponenten zur Erzeugung eines experimentell detektierbaren Signals von derselben Zelle erzeugt werden, aus der das Vesikel hergestellt wird.

Eine andere mögliche Ausführungsform ist dadurch gekennzeichnet, dass die Zelle, aus der das Vesikel hergestellt wird, vor dessen Herstellung mit besagten zusätzlichen Komponenten zur Erzeugung eines experimentell detektierbaren Signals beladen wird.

Die besagten zusätzlichen Komponenten zur Erzeugung eines experimentell detektierbaren Signals können aber auch nach Ausbildung des Vesikels in dieses eingefügt werden.

Ein für den praktischen Einsatz sehr wichtiges und vorteilhaftes Kennzei-chen eines erfindungsgemässen bioanalytischen Reagens ist, dass die Funktionalität eines mit einem Vesikel als Teil des besagten bioanalytischen Reagens assoziierten Rezeptors bei Lagerung im tiefgefrorenen Zustand über einen Zeitraum von mindestens einer Woche, bevorzugt von min-destens einem Monat, besonders bevorzugt von mindestens einem Jahr erhalten bleibt. Genaue Bedingungen zum Einfrieren von Vesikeln als Bestandteil des erfindungsgemässen Reagens sind in Beispiel 3 beschrieben. Die "Erhaltung der Funktionalität" besagten Rezeptors soll dabei bedeuten, dass ein von besagtem Rezeptor auszulösender Mechanismus der Signal-transduktion in einem Vesikel auch nach dessen Lagerung unter den genannten Bedingungen im wieder aufgetauten Zustand erhalten ist.

Unter anderen Bedingungen, beispielsweise unter sterilen Bedingungen in gekühlter Pufferlösung, d.h. bei einer Temperatur unterhalb Raumtemperatur, beispielsweise bei 4°C, weist ein erfindungsgemässes bioanalytisches Reagens eine Lagerfähigkeit von mindestens einer Woche auf.

Ein weiterer Gegenstand der vorliegenden Erfindung ist ein Verfahren zur Herstellung eines bioanalytischen Reagens mit einem aus einer lebenden Zelle erzeugten Vesikel nach einer der vorgenannten Ausführungsformen, dadurch gekennzeichnet, dass besagtes Vesikel aus einer lebenden Zelle, umfassend mindestens einen Rezeptor, erzeugt wurde, und dass ein von besagtem Rezeptor in besagter lebender Zelle ausgelöster Mechanismus einer Signaltransduktion in besagtem Vesikel als Bestandteil des bioanalytischen Reagens erhalten bleibt.

Es wird bevorzugt, dass die Abschnürung besagten Vesikels von besagter lebender Zelle nach Zugabe von Cytochalasin B und / oder Cytochalasin D erfolgt.

Ausserdem wird bevorzugt, dass das erfindunggsgemässe Verfahren ohne Zugabe von Protease-Inhibitoren erfolgt. Bei anderen Verfahren, welche Zell-Proteasen freisetzen, besteht die Gefahr, dass trotz Zugabe eines Protease-Inhibitors oder von Gemischen von Protease-Inhibitoren ein gewisser Abbau von Rezeptorproteinen stattfinden kann. Ausserdem müssten Protease-Inhibitoren in zusätzlichen Reinigungsschritten entfernt werden.

Das erfindungsgemässe Verfahren kann die Anwendung von Scherkräften und / oder Zentrifügatiohsschritteh, beispielsweise unter Einwirkung eines Succrosegradienten, und / oder Chromatographieschritten, beispielsweise zur Auftrenung in Fraktionen unterschiedlicher Grössenverteilungen, und / oder von Filtrationsschritten und / oder von elektrophoretischen Methoden umfassen.

Mit Hilfe des erfindungsgemässen Verfahrens kann besagtes mindestens eine Vesikel aus einer eukaryotischen Zelle hergestellt werden. Es ist auch möglich, das besagte mindestens eine Vesikel aus einer Zelle eines nativen Gewebes herzustellen.

Von grosser Bedeutung ist eine solche Ausführungsform des erfindungsge-mässen Verfahren ist, welche dadurch kennzeichnet ist, dass das Innere eines nach diesem Verfahren hergestellten Vesikels frei von Zellkernmate-rial ist, so dass keine replikativen Prozesse auftreten.

Bevorzugt wird eine solche Ausführungsform des erfindungsgemässen Verfahrens, welche dadurch gekennzeichnet ist, dass ein nach diesem Verfahren hergestelltes Vesikel einen Durchmesser von 50 nm - 5000 nm, besonders bevorzugt von 100 - 2000 nm hat.

Für viele Anwendungen wird bevorzugt, dass ein Rezeptor in einem nach diesem Verfahren hergestellten Vesikel als Bestandteil des bioanalytischen Reagens in natürlicher Form vorliegt.

Für andere Anwendungen wird bevorzugt, dass ein Rezeptor in einem nach diesem Verfahren hergestellten Vesikel als Bestandteil des bioanalytischen Reagens in modifizierter Form vorliegt. Zum Beispiel kann besagter Rezeptor als Fusionsprotein vorliegen, beispielsweise durch Fusion mit einem fluoreszenten Protein wie GFP (green fluorescent protein) oder BFP (blue fluorescent protein) oder RFP (red fluorescent protein). Ein anderes Beispiel ist die Fusion zwischen einem GPCR und einem G-Protein.

Vielfach ist es auch vorteilhaft, wenn ein Rezeptor in einem nach diesem Verfahren hergestellten Vesikel in rekombinant hergestellter Form vorliegt.

Ein wichtiges Kennzeichen des erfindungsgemässen Verfahren ist, dass eine in der Vesikel-erzeugenden lebenden Zelle vorhandene "Bindungsfähigkeit des besagten mindestens einen Rezeptors, welcher mit dem Vesikel als Teil des bioanalytischen Reagens assoziiert ist, zu einem spezifischen Liganden, erhalten bleibt.

Der mindestens eine Rezeptor kann ausgewählt sein aus der Gruppe von signalübertragenden Rezeptoren, die von Plasmamembranrezeptoren, wie beispielsweise Ionenkanalrezeptoren, G-Protein-gekoppelten Rezeptoren (GPCR), Orphan-Rezeptoren, Enzym-gekoppelten Rezeptoren, wie z. B. Rezeptoren mit intrinsischer Tyrosine-Kinase Aktivität, Rezeptoren mit intrinsischer Serin/Threonine-Kinase-Aktivität, des weiteren Rezeptoren für Wachstumsfaktoren (Peptid-Hormon-Rezeptoren), Rezeptoren für chemotaktische Substanzen, wie der Klasse der Chemokine-Rezeptoren, und schliesslich intrazellulären Hormon-Rezeptoren, beispielsweise Steroidhormon-Rezeptoren, gebildet wird.

Bevorzugt sind solche Ausführungsformen des erfindungsgemässen Verfahrens, welche dadurch gekennzeichnet sind, dass die Flächendichte, bezogen auf die Oberfläche der äusseren Vesikelmembran, von mit der äusseren Membran eines Vesikels in Kontakt befindlichen Rezeptoren von gleicher Grössenordnung oder grösser als die entsprechende Dichte dieser Rezeptoren in der Vesikel-erzeugenden lebenden Zelle ist.

Weitere Ausführungsformen des erfindungsgemässen Verfahrens sind dadurch gekennzeichnet, dass besagtes mindestens eine nach diesem Verfahren hergestellte Vesikel neben besagtem mindestens einem Rezeptor weitere biologische Verbindungen (Komponenten) aus der Gruppe umfasst, die von beispielsweise G-Proteinen und G-Protein-Regulatoren (z.B. rasGAP), Enzymen, wie beispielsweise Adenylatzyklasen, Phospholipasen, welche intrazelluläre sekundäre Botenstoffe (z.B. cAMP (zyklisches Adenosinmonophosphat), cGMP (Zyklisches Guanosinmonophosphat), Diacylglycerol (DAG) oder Inositoltrisphosphat (IP3)) bilden, und Enzymen, wie beispielsweise Serin-, Threonin- und Tyrosin-Kinasen sowie Tyrosin-Phosphatasen, welche Proteine durch Phosphorylierung oder Dephosphorylierung aktivieren bzw. hemmen, gebildet wird.

Kennzeichen weiterer Ausführungsformen des Verfahrens ist, dass mit der äusseren Membran des mindestens einen nach diesem Verfahren hergestell-ten Vesikels biologische, biochemische oder synthetische Komponenten, wie beispielsweise Zelloberflächenproteine oder Zelloberflächenzucker assoziiert sind, welche dem Transport besagten Vesikels zu vorbestimmten Zielorten, wie beispielsweise Zellen und / oder Organen und / oder vorbestimmtem Gewebe in einem lebenden Organismus und / oder der Bindung an ein biologisches oder biochemisches oder synthetisches Erkennungselement, welches besagte biologische oder biochemische oder synthetische Komponenten spezifisch erkennt und bindet, dienen.

Zur Verminderung von unspezifischer Bindung eines Vesikels, als Teil eines erfindungsgemässen bioanalytischen Reagens, an eine mit dem Vesikel in Kontakt zu bringende Oberfläche, kann es vorteilhaft sein, wenn nach Erzeugung besagten Vesikels aus einer lebenden Zelle zusätzlich Lipide, welche beispielsweise hydrophile Polymere (wie z.B. Polyethylenglycole) enthalten, in die Vesikelmembran integriert werden. Vesikel mit oberflächenassozüerten Polymeren sind beispielsweise in der PCT/EP/00/04491 beschrieben.

Von grosser Bedeutung sind auch solche Ausführungsformen des erfin-dungsgemässen Verfahrens, welche dadurch gekennzeichnet sind, dass besagtes mindestens eine nach diesem Verfahren hergestellte Vesikel zusätzlich Komponenten zur Erzeugung eines experimentell detektierbaren Signals umfasst.

Diese zusätzlichen Komponenten zur Erzeugung eines experimentell detektierbaren Signals können mit in dem mindestens einen Vesikel als Bestandteil des besagten bioanalytischen Reagens assoziierten weiteren biologischen Verbindungen (Komponenten) umfasst sein. Dabei können besagte weitere biologische Verbindungen (Komponenten) aus der Gruppe stammen, die von beispielsweise G-Proteinen und G-Protein-Regulatoren (z.B. rasGAP), Enzymen, wie beispielsweise Adenylatzyklasen, Phospholipasen, welche intrazelluläre sekundäre Botenstoffe (z.B. cAMP (zyklisches Adenosinmonophosphat), cGMP (Zyklisches Guanosinmonophosphat), Diacylglycerol (DAG) oder Inositoltrisphosphat (IP3)) bilden, und Enzymen, wie beispielsweise Serin-, Threonin- und Tyrosin-Kinasen sowie Tyrosin-Phosphatasen, welche Proteine durch Phosphorylierung oder Dephosphorylierung aktivieren bzw. hemmen, gebildet wird.

Die besagten zusätzlichen Komponenten zur Erzeugung eines experimentell detektierbaren Signals können auch mit einem Rezeptor assoziiert sein oder Bestandteile von mit dem Vesikel assoziierten Fusionsproteinen sein.

Besagte zusätzliche Komponenten zur Erzeugung eines experimentell detektierbaren Signals können ausgewählt sein aus der Gruppe von Komponenten, die von absorptiven und von lumineszenten Indikatoren, Lumineszenzlabeln, lumineszenten Nanopartikeln, von absorptiven und von lumineszenten Indikatorproteinen, wie beispielsweise BFP ("blue fluorescent protein"), GFP ("green fluorescent protein") oder RFP ("red fluorescent protein"), nicht-natürlichen lumineszenten (d.h. insbesondere fluoreszenten) Aminosäuren, radioaktiven Labels, Spin-Labeln, wie beispielsweise NMR-Labeln oder ESR-Labeln, Ionenindikatoren, insbesondere pH- und Calcium-Indikatoren, oder potentialabhängigen Indikatoren, wie beispielsweise potentialabhängigen Lumineszenzlabeln, oder Redox-Komplexen gebildet wird

Nach dem erfindungsgemässen Verfahren können besagte zusätzlichen Komponenten zur Erzeugung eines experimentell detektierbaren Signals von derselben Zelle erzeugt werden, aus der das Vesikel hergestellt wird.

Es ist auch möglich, die Zelle, aus der das Vesikel hergestellt wird, vor dessen Herstellung mit besagten zusätzlichen Komponenten zur Erzeugung eines experimentell detektierbaren Signals zu beladen.

Diese zusätzlichen Komponenten zur Erzeugung eines experimentell detektierbaren Signals können aber auch nach Ausbildung des Vesikels in dieses eingefügt werden.

Weiterer Gegenstand der Erfindung ist ein bioanalytisches Nachweisver-fahren mit einem erfindungsgemässen bioanalytischen Reagens nach einer der vorgenannten Ausführungsformen, wobei besagtes Nachweisverfahren ausgewählt ist aus der Gruppe, die beispielsweise von optischen Nachweisverfahren, wie beispielsweise refraktometrischen Verfahren, Oberflächenplasmonenresonanz, optischen Absorptionsmessungen (z. B. Internen Reflexionsmessungen mit einem hochrefraktiven Material in Kombination mit infrarotspektroskopischen Messungen) oder Lumineszenzdetektion (z. B. Fluoreszenz-Korrelations-Spektroskopie), Detektion von energie- oder Ladungstransfer, Massenspektroskopie, elektrischen oder elektrochemischen Nachweisverfahren, wie beispielsweise Elektrophysiologie, Patch-Clamp-Techniken, Impedanzmessung, elektronischen Resonanzmessungen, wie beispielsweise Elektonenspinresonanz oder Kernspinresonanz, gravimetrischen (z. B. elektrischen Schwingquarzmessungen), radioaktiven Verfahren oder elektrophoretischen Messungen gebildet wird.

Ein Teil der möglichen Ausführungsformen des erfindungsgemässen bioanalytisches Nachweisverfahrens ist dadurch gekennzeichnet, dass dieses in homogener Lösung ausgeführt wird.

Eine spezielle Gruppe von Ausführungsformen des erfindungsgemässen bioanalytischen Nachweisverfahrens mit einem erfindungsgemässen bioanalytischen Reagens betrifft spezifische Patch-clamp-Verfahren, welche in Einfach-Ausführung und in paralleler Mehrfachausführung durchgeführt werden können. Ein bioanalytisches Nachweisverfahren gemäss dieser Ausführungsform ist dadurch gekennzeichnet, dass dieses Nachweisverfahren mit Hilfe einer Messanordnung mit mindestens 2 Elektroden und separierten, für die Aufnahme von Flüssigkeiten geeigneten Kompartimenten durchgeführt wird, wobei sich zwischen zwei, einander gegenüber befindlichen, in je mindestens ein Kompartiment hineinreichenden oder ein solches berührenden und beliebig geformten Elektroden ein fester Träger (vorzugsweise als elektrisch isolierende Trennwand) befindet, der mindestens eine Apertur enthält und jeweils mindestens 2 Kompartimente voneinander trennt.

Der genannte Träger stellt eine zwischen den Elektroden angeordnete Trennwand aus elektrisch isolierendem Material dar. Wie oben genannt, weist der Träger eine Apertur auf sowie eine Oberfläche, an der Membranen von Vesikeln aus einem erfindungsgemässen bioanalytischen Reagens fixiert werden können. Der Träger muss nicht aus einem Stück bestehen, sondern er kann z. B. aufgebaut sein aus einem Halter, auf dem das für die Membranbindung und Membranpositionierung eigentlich relevante Material befestigt oder in den dieses Material eingelassen ist, wobei dieses Material für die Bindung bzw. Positionierung der Membranen mindestens eine Apertur aufweist. Die Apertur kann ausserdem von einer ringförmigen, spitz zulaufenden Erhebung (typischerweise mit Höhe im Submikrometerbereich und der Apertur im Zentrum) umgeben sein. Damit kann auf einem ansonsten im wesentlichen planaren Träger eine Mikropipetten-ähnliche Öffnung erzeugt werden.

Mit einer solchen spezifischen Anordnung wird es ermöglicht, dass sich bei Existenz einer Spannungsdifferenz über der Messanordnung, vermittelt durch die mindestens zwei Elektroden, ein inhomogenes elektrisches Feld um die Apertur aufbaut, das mit Annäherung an die Apertur betragsmässig grösser wird und nahe der Apertur befindliche, aus einer lebenden Zelle erzeugte Vesikel, aus einem erfindungsgemässen bioanalytischen Reagens, elektrophoretisch auf diese zubewegen kann.

Es wird damit auch ermöglicht, solche Vesikel durch einen hydrodynamischen oder elektrokinetischen Fluss oder durch andere mechanische Manipulation (z. B. mittels optischer Pinzetten, Kraftmikroskop oder Mikromanipulator) über die Apertur zu positionieren.

Die Fixierung der Membranen kann z.B. auf elektrostatischen Wechselwirkungen zwischen einer beispielsweise negativ geladenen Membranoberfläche und einer positiv geladenen Trägeroberfläche beruhen. Falls die Trägeroberfläche als solche nicht die gewünschte Ladung aufweist, so kann sie entsprechend modifiziert werden.

Es wird bevorzugt, dass besagte Messanordnung auf einer Seite oder auf beiden Seiten des Trägers Mittel aufweist, die eine Flüssigkeitszugabe und / oder eine Flüssigkeitspeicherung und / oder einen Flüssigkeitsaustausch und / oder die Zugabe von aus einer lebenden Zelle erzeugten Vesikeln, aus einem erfindungsgemässen bioanalytischen Reagens, zwischen Träger und Elektrode ermöglichen.

Ausserdem wird bevorzugt, dass die mindestens eine Apertur besagter Messanordnung einen solchen Durchmesser aufweist, dass sich bei Existenz einer Spannungsdifferenz über der Messanordnung, vermittelt durch die mindestens zwei Elektroden, ein inhomogenes elektrisches Feld um die Apertur aufbaut, das mit Annäherung an die Apertur betragsmässig grösser wird und nahe der Apertur aus einer lebenden Zelle erzeugte Vesikel, aus einem erfindungsgemässen bioanalytischen Reagens, elektrophoretisch auf diese zubewegen kann.

Vorteilhaft ist auch, wenn die mindestens eine Apertur besagter Messanordnung einen solchen Durchmesser aufweist, dass sich aus einer lebenden Zelle erzeugte Vesikel, aus einem erfindungsgemässen bioanalytischen Reagens, durch einen hydrodynamischen oder elektrokinetischen Fluss oder durch andere mechanische Manipulation (z.B. mittels optischer Pinzetten, Kraftmikroskop oder Mikromanipulator) über oder in der Apertur positionieren lassen.

Viele Ausführungsformen dieser speziellen Gruppe erfindungsgemässer bioanalytischer Nachweisverfahren sind dadurch gekennzeichnet, das der Träger besagter Messanordnung eine elektrisch geladene Oberfläche aufweist, die attraktiv für aus einer lebenden Zelle erzeugte Vesikel, aus einem erfindungsgemässen bioanalytischen Reagens, ist, oder dass auf seiner Oberfläche eine Haftvermittlungsschicht zur Bindung besagter Vesikel aufgebracht ist.

Das erfindungsgemässe bioanalytische Nachweisverfahren kann durchgeführt werden, indem aus einer lebenden Zelle erzeugte Vesikel, aus einem erfindungsgemässen bioanalytischen Reagens, in ein vorher mit Puffer gefülltes oder ungefülltes Compartiment zwischen Trennwand bzw. Träger und Elektrode gegeben werden und durch eine an die Elektroden angelegte elektische Spannungsdifferenz auf die Aperturöffnung bewegt werden oder / und durch hydrodynamischen oder elektrokinetischen Fluss oder / und mechanisch (z. B. durch optische Pinzette, Kraftmikroskop oder Mikromanipulator) auf die Aperturöffnung positioniert werden.

Insbesondere ist es möglich, dass aus einer lebenden Zelle erzeugte Vesikel, aus einem erfindungsgemässen bioanalytischen Reagens, auf besagte Aperturöffnung positioniert werden, die Vesikelmembranen mit der Oberfläche des Trägers eine elektrisch dichte Verbindung über der Aperturöffnung eingehen und eine Aufzeichnung des Membranwiderstandes ermöglichen. Bei diesem Vorgang können die Vesikel in ihrer Form erhalten bleiben oder mit der Oberfläche des Trägers fusionieren und so eine Apertur-überspannende planare Membran bilden. Dabei kann mit dem erfindungsgemässen Verfahren ein guter Signal-Rauschabstand erreicht werden.

Das Verfahren ermöglicht es auch, dass zu mindestens einem Kompartiment künstliche Lipidvesikel, mit einem grösseren Durchmesser als dem Durchmesser besagter Apertur, gegeben werden, mit dem Ziel, damit auf der Oberfläche des Trägers eine Apertur-überspannende, planare Lipiddoppel-schicht zu erzeugen, und dass anschliessend aus lebenden Zelle erzeugte Vesikel, aus einem erfindungsgemässen bioanalytischen Reagens, zu besagtem Kompartiment hinzugegeben werden, mit dem Ziel, diese mit der erzeugten planaren Lipidmembran zu fusionieren und damit Rezeptoren, welche mit diesen aus lebenden Zellen erzeugten Vesikeln assoziiert sind, elektrischen oder optischen Messungen zugänglich zu machen.

Ausserdem schafft das Verfahren die Möglichkeit, dass Membranproteine nach der Positionierung eines aus einer lebenden Zelle erzeugten Vesikels über einer Apertur in dieses eingebaut werden.

Es wird bevorzugt, dass ein über einer Apertur befindliches aus einer lebenden Zelle erzeugtes Vesikel, oder eine aus diesem Vesikel erzeugte Apertur-überspannende planare Membran optischen Messungen, insbesondere Fluoreszenzmessungen, oder simultanen optischen und elektrischen zugänglich ist und diese daran ausgeführt werden.

Eine besondere Variante dieser auf Patch-clamp-Techniken basierenden erfindungsgemässen bioanalytischen Nachweisverfahren ist dadurch gekennzeichnet, dass eine Messanordnung oder ein Messsystem mit mehreren Aperturen auf einem Träger verwendet wird, und dass Messungen über mindestens zwei Aperturen sequentiell und/oder parallel erfolgen.

Insbesondere können auch eine Vielzahl von aus lebenden Zellen erzeugte Vesikels, aus einem erfindungsgemässen bioanalytischen Reagens, in einem Array auf einen festen, elektrisch isolierenden Träger angeordnet sein, wobei dieses Array von Vesikeln mit einem Array von Patch-Clamp-Pipetten gleicher geometrischer Anordnung (welches z.B. mikromechanisch positioniert werden kann) wie das Vesikel-Array in elektrisch isolierenden Kontakt gebracht wird, um eine gleichzeitige Durchführung von voneinander unabhängigen elektrischen Messungen, oder simultanen elektrischen und optischen Messungen, an einer Vielzahl einzelner Vesikel zu ermöglichen.

Weitere Ausführungsformen von derartigen Messanordnungen, insbesondere auch mit einem "Patch Clamp-Array",und damit auszuführende analytische Nachweisverfahren, die sich für ein bioanalytisches Nachweisverfahren mit einem erfindungsgemässen bioanalytischen Reagens eignen, sind in der WO 99/31503 beschrieben. Ihre besondere Verwendung im Zusammenhang mit einem erfindungsgemässen Nachweisverfahren mit einem erfindungs-gemässen bioanalytischen Reagens ist ebenfalls Bestandteil dieser Erfindung.

Eine Vielzahl möglicher Ausführungsformen eines erfindungsgemässen bioanalytischen Nachweisverfahrens ist dadurch gekennzeichnet, dass das mindestens eine aus einer lebenden Zelle erzeugte Vesikel umfassend mindestens einen Rezeptor, aus einem erfindungsgemässen bioanalytischen Reagens, auf einem festen Träger immobilisiert wird.

Vorteilhafterweise ist ein aus einer lebenden Zelle hergestelltes Vesikel aus einem erfindungsgemässen bioanalytischen Form nach seiner immobilisierung auf einem festen Träger insbesondere auch massenspektroskopischen Untersuchungen zugänglich.

Diese Ausführungsformen eines erfindungsgemässen bioanalytischen Nachweisverfahrens sind dadurch gekennzeichnet, dass ein von besagtem Rezeptor in besagter lebenden Zelle ausgelöster Mechanismus einer Signaltransduktion in einem aus der Zelle erzeugten Vesikel nach dessen Immobilisierung erhalten bleibt.

Insbesondere Gegenstand der Erfindung ist daher ein bioanalytisches Nachweisverfahren mit mindestens einem auf der Oberfläche eines festen Trägers immobilisiertem, aus einer lebenden Zelle erzeugtem Vesikel, umfassend mindestens einen Rezeptor, aus einem erfindungsgemässen bioanalytischen Reagens, welches dadurch gekennzeichnet ist, dass ein von besagtem Rezeptor in besagter lebenden Zelle ausgelöster Mechanismus einer Signaltransduktion in besagtem Vesikel nach der Immobilisierung erhalten bleibt.

Es wird bevorzugt, dass Vesikel, umfassend jeweils mindestens einen Rezeptor, in diskreten Messbereichen (d), mit jeweils ein oder mehr Vesikeln, auf der Oberfläche besagten festen Trägers immobilisiert sind.

Weiterhin wird bevorzugt, dass in einer Vielzahl von Messbereichen Vesikel mit wenigstens zwei unterschiedlichen Arten von Rezeptoren immobilisiert sind, wobei innerhalb eines einzelnen Messbereichs vorzugsweise jeweils Vesikel mit einer einheitlichen Art von Rezeptoren immobilisiert sind.

Die Immobilisierung des mindestens einen aus einer lebenden Zelle erzeugten Vesikels auf der Oberfläche besagten festen Trägers kann beispielsweise mittels kovalenter Bindung oder mittels physikalischer Adsorption (elektrostatischer oder van-der-Waals-Wechselwirkung oder hydrophiler oder hydrophober Wechselwirkung oder einer Kombination dieser Wechselwirkungen) erfolgen.

Es wird bevorzugt, dass zwischen der Oberfläche besagten festen Trägers und dem mindestens einen darauf immobilisierten Vesikel eine Haftvermittlungsschicht aufgebracht ist. Dabei ist erfindungsgemäss die Haftvermittlungsschicht so gestaltet, dass auch nach der Immobilisierung von aus einer lebenden Zelle erzeugten Vesikeln als Bestandteil eines bioanalytischen Reagens, umfassend mindestens einen Rezeptor, auf besagter Haftvermittlungsschicht ein von besagtem Rezeptor in besagter lebenden Zelle ausgelöster Mechanismus einer Signaltransduktion in besagtem Vesikel erhalten bleibt.

Es wird bevorzugt, dass die Haftvermittlungsschicht eine Stärke von vorzugsweise weniger als 200 nm, besonders bevorzugt von weniger als 20 nm hat.

Die Haftvermittlungsschicht kann eine chemische Verbindung aus der Gruppe Silane, Epoxide, funktionalisierte, geladene oder polare Polymere und "selbstorganisierte funktionalisierte Mono- oder Mehrfachschichten" umfassen.

Eine bevorzugte Ausführungsform ist dadurch gekennzeichnet, dass die Haftvermittlungsschicht eine monomolekulare Schicht aus vorwiegend einer Sorte von Proteinen, wie beispielsweise Serumalbuminen oder Streptavidin, oder modifizierten Proteinen, wie z.B. biotinyliertes Serumalbumin, umfasst.

Kennzeichen einer anderen bevorzugten Ausführungsform ist, dass die Haftvermittlungsschicht selbstorganisierte, Alkan-endständige Monoschich-ten aus vorwiegend einer Sorte von chemischen oder biochemischen Molekülen umfasst.

Besonders bevorzugt ist eine Ausführungsform, welche dadurch gekenn-zeichnet ist, dass die Haftvermittlungsschicht in Form einer Doppelschicht ausgebildet ist, umfassend eine erste Alkan-endständige, selbsorganisierte Ankerschicht und eine zweite Schicht, welche über Selbstorganisation ("self assembly") von synthetischen oder natürlichen Lipiden gebildet wird.

Die Immobilisierung des mindestens einen aus einer lebenden Zelle erzeugten Vesikels auf der Oberfläche besagten festen Trägers kann beispielsweise mittels kovalenter Bindung oder mittels physikalischer Adsorption (elektrostatischer oder van-der-Waals-Wechselwirkung oder hydrophiler oder hydrophober Wechselwirkung oder einer Kombination dieser Wechselwirkungen) erfolgen.

Eine besondere Ausführungsform des erfindungsgemässen bioanalytischen Nachweisverfahrens, mit einer besonders spezifischen Art der Immobilisierung der Vesikel ist dadurch gekennzeichnet, dass mit der Haftvermittlungsschicht biologische oder biochemische oder synthetische Erkennungselemente assoziiert sind, welche ein aus einer lebenden Zelle erzeugtes Vesikel mit oberflächenassozüerten biologischen oder biochemischen oder synthetischen Komponenten zur spezifischen Erkennung und Bindung, als Teil der entsprechenden, vorangehend beschriebenen spezifischen Ausführungsform eines erfindungsgemässen bioanalytischen Reagens, erkennen und binden. Beispielsweise können diese spezifischen Wechselwirkungen zur Erkennung und Bindung der Vesikel an deren Erkennungselemente auf der Haftvermittlungsschicht auf Wechselwirkungen mit Biotin / Streptavidin, sogenannten "Histidin-Tags" (Ref. : Schmid E. L., Keller T. A., Dienes Z., Vogel H., "Reversible oriented surface immobilization of functional proteins on oxide surfaces", Anal Chem 69 (1997)1979-1985; Sigal G. B., Bamdad C., Barberis A., Strominger J., Whitesides G. M., "A self-assembled monolayer for the binding and study of histidine-tagged proteins by surface plasmon resonance", Anal Chem 68 (1996) 490-497), Zuckern oder Peptidaffinitäts-Wechselwirkungen beruhen, wobei ein beliebiger der jeweils zwei Bindungspartner mit der Vesikeloberfläche assoziiert ist und der andere Partner auf der Oberfläche besagter Haftvermittlungsschicht verankert ist.

Eine andere Ausführungsform des erfindungsgemässen bioanalytischen Nachweisverfahrens ist dadurch gekennzeichnet, dass mindestens ein Ligand für einen Rezeptor, welcher an ein aus einer lebenden Zelle erzeugtes Vesikel, aus einem erfindungesgemässen bioanalytischen Reagens nach einer der genannten Ausführungsformen, gebunden ist, auf der Oberfläche eines festen Trägers, gegebenenfalls über ein Spacer-Molekül, immobilisiert ist.

Dabei wird bevorzugt, dass in einer Vielzahl von Messbereichen (d) wenigstens zwei unterschiedliche Liganden für Rezeptoren, welche an ein aus lebenden Zellen erzeugte Vesikel, aus einem erfindungsgemässen bioanalytischen Reagens, gebunden sind, wobei innerhalb eines einzelnen Messbereichs vorzugsweise jeweils eine einheitliche Art von Liganden immobilisiert ist.

Besagte Liganden können auf der Oberfläche des festen Trägers mittels kovalenter Bindung oder mittels physikalischer Adsorption (z.B. elektrostatischer oder van-der-Waals-Wechselwirkung oder hydrophiler oder hydrophober Wechselwirkung oder einer Kombination dieser Wechselwirkungen) immobilisiert sein.

Bevorzugt wird, dass zwischen der Oberfläche des festen Trägers und besagten darauf immobilisierten Liganden eine Haftvermittlungsschicht aufgebracht ist.

Für die Auswahl der Haftvermittlungsschicht zur Immobilisierung besagter Liganden auf dem festen Träger gelten die gleichen Bevorzugungen, wie sie vorangehend für eine Haftvermittlngsschicht zur Immobilisierung von aus einer lebenden Zelle erzeugten Vesikeln aus einem erfindungsgemässen bioanalytischen Reagens genannt wurden.

Eine besonders bevorzugte Ausführungsform eines erfindungsgemässen bioanalytischen Nachweisverfahrens ist dadurch gekennzeichnet, dass Bereiche zwischen den räumlich getrennten Messbereichen, mit darin immobilisierten, aus lebenden Zellen erzeugten Vesikeln (aus einem bioanalytischen Reagens nach einer der genannten Ausführungsformen) oder mit darin immobilisierten Liganden für Rezeptoren, welche an aus lebenden Zellen erzeugte Vesikel (aus einem bioanalytischen Reagens nach einer der genannten Ausführungsformen) gebunden sind, oder / und dass Bereiche innerhalb dieser Messbereiche, zwischen den genannten darin immobilisierten Verbindungen, zur Minimierung unspezifischer Bindung von Analyten oder deren Nachweissubstanzen "passiviert werden", d.h. dass zwischen den räumlich getrennten Messbereichen (d) oder / und dass innerhalb dieser Messbereiche (d) zwischen den genannten darin immobilisierten Verbindungen gegenüber dem Analyten "chemisch neutrale" Verbindungen aufgebracht sind, vorzugsweise beispielsweise bestehend aus den Gruppen, die von Albuminen, Casein, Detergentien - wie beispielsweise Tween 20 -, Detergentien-Lipid-Mischungen (aus synthetischen oder / und natürlichen Lipiden), synthetischen und natürlichen Lipiden oder auch hydrophilen Polymeren, wie beispielsweise Polyethylenglycolen oder Dextranen, gebildet werden.

Es ist auch möglich eine (zur Immobilisierung der biologischen oder biochemischen oder synthetischen Erkennungselemente) aktivierte Oberfläche (beispielsweise umfassend Poly-L-Lysin oder funktionalisierte Silane, beispielsweise mit Aldehyd- oder Epoxygruppen) zu passivieren, beispielsweise (im Falle von Aldehyd- oder Epoxygruppen) mittels Zugabe von reduzierenden Reagentien, wie beispielsweise Natriumborhydrat.

Das Material der Oberfläche besagten festen Trägers mit immobilisierten, aus lebenden Zellen erzeugten Vesikeln (aus einem erfindungsgemässen bioanalytischen Reagens nach einer der genannte Ausführungsformen) oder mit darauf immobilisierten Liganden für Rezeptoren, welche an aus lebenden Zellen erzeugte Vesikel (aus einem erfindungsgemässen bioanalytischen Reagens nach einer der genannte Ausführungsformen) gebunden sind, kann ein Material aus der Gruppe umfassen, die beispielsweise von form-, spritz- oder fräsbaren Kunststoffen, Kohlenstoffverbindungen, Metallen, wie beispielsweise Gold, Silber, Kupfer, Metalloxiden oder Silikaten, wie zum Beispiel Glas, Quarz oder Keramiken, oder Silizium oder Germanium oder ZnSE oder einer Mischung dieser Materialien gebildet wird.

Dabei kann besagter fester Träger in einer Vielzahl unterschiedlicher Ausführungsformen ausgebildet sein. Beispielsweise kann es sich hierbei um ein Glas- oder Mikroskop-Plättchen handeln. Es kann sich hierbei auch um eine Mikrotiter-Platte handeln, wie sie z.B. für Screening-Assays (zum Test, beispielsweise mittels klassischer Fluoreszenzmethoden oder mittels Fluoreszenz-Korrelationsspektroskopie, einer Vielzahl von Verbindungen) gebräuchlich ist.

Es wird bevorzugt, dass die Oberfläche besagten festen Trägers im wesentlichen planar ist.

Eine Gruppe bevorzugter Ausführungsformen des bioanalytischen Nachweisverfahrens ist dadurch gekennzeichnet, dass es sich bei besagtem festen Träger um eine optische oder elektronische Sensorplattform handelt.

Dabei wird bevorzugt, dass besagter fester Träger mindestens in einem Wellenlängenbereich im ultravioletten bis infraroten Spektrum transparent ist und er vorzugsweise ein Material aus der Gruppe umfasst, die beispielsweise von form-, spritz- oder fräsbaren Kunststoffen, Kohlenstoffverbindungen, Metallen, Metalloxiden oder Silikaten, wie zum Beispiel Glas, Quarz oder Keramiken, oder einer Mischung dieser Materialien gebildet wird.

Bevorzugt wird dabei, dass es sich bei besagtem festen Träger um einen als Sensorplattform dienenden optischen Wellenleiter handelt. Besonders bevorzugt wird eine solche Ausführungsform des Nachweisverfahrens, welche dadurch gekennzeichnet ist, dass es sich bei besagtem festen Träger um einen als Sensorplattform dienenden optischen Dünnschichtwellenleiter handelt, mit einer ersten optisch transparenten Schicht (a) mit Brechungsindex n₁ auf einer zweiten optisch transparenten Schicht (b) mit Brechungsindex n₂, wobei n₁ > n₂ ist.

Beispielsweise zur gleichzeitigen Untersuchung mehrerer Proben und / oder Bestimmung mehrerer Analyten in einer oder mehreren Proben ist es von Vorteil, wenn die Sensorplattform als fester Träger aufgeteilt ist in zwei oder mehr diskrete wellenleitende Bereiche.

Das Material der zweiten optisch transparenten Schicht (b) der Sensorplattform als festem Träger kann ein Material aus der Gruppe umfassen, welche von Silikaten, wie beispielsweise Glas oder Quarz, oder transparenten form-, spritz- oder fräsbaren, insbesondere thermoplastischen Kunststoffen, wie beispielsweise Polycarbonaten, Polyimiden, Polymethylmethacrylaten oder Polystyrolen gebildet wird.

Es wird bevorzugt, dass der Brechungsindex der ersten optisch transparenten Schicht (a) der Sensorplattform als festem Träger grösser als 1.8 ist.

Ausserdem wird bevorzugt, dass die erste optisch transparente Schicht (a) der Sensorplattform als festem Träger TiO₂., ZnO, Nb₂O₅, Ta₂O₅, HfO₂, oder ZrO₂, bevorzugt aus TiO₂, Ta₂O₅ oder Nb₂O₅ umfasst.

Die Dicke der ersten optisch transparenten Schicht (a) beträgt vorteilhaft 40 bis 300 nm, besonders bevorzugt 100 bis 200 nm.

Eine weitere Ausführungsform des erfindungsgemässen bioanalytischen Nachweisverfahrens ist dadurch gekennzeichnet, dass sich zwischen den optisch transparenten Schichten (a) und (b) und in Kontakt mit Schicht (a) eine weitere optisch transparente Schicht (b') mit niedrigerem Brechungsindex als dem der Schicht (a) und einer Stärke von 5 nni - 10000 nm, vorzugsweise von 10 nm - 1000 mm befindet. Diese Zwischenschicht (b') kann beispielsweise der Verbesserung der Haftung der Schicht (a) auf der Schicht (b) oder die Verminderung des Einflusses von Oberflächenrauüigkeiten der Schicht (b) dienen. Aufgabe der Schicht (b') kann jedoch beispielsweise auch der Verminderung des Eindringens des evaneszenten Feldes eines in der Schicht (a) geführten Lichts in die Schicht (b) dienen, beispielsweise zur Verminderung einer unerwünschten Lumineszenzanregung in der Schicht (b).

Eine bevorzugte Ausführungsform des erfindungsgemässen , bioanalytischen Nachweisverfahrens ist dadurch gekennzeichnet, dass die Einkopplung von Anregungslicht in die optisch transparente Schicht (a) zu den Messbereichen (d) auf der Sensorplattform als Träger, über ein oder mehrere optische Einkoppelelemente aus der Gruppe erfolgt, die von Prismenkopplern, evaneszenten Kopplern mit zusammengebrachten optischen Wellenleitern mit überlappenden evaneszenten Feldern, Stirnflächenkopplern mit vor einer Stirnseite der wellenleitenden Schicht angeordneten fokussierenden Linsen, vorzugsweise,Zylinderlinsen, und Gitterkopplern gebildet wird.

Besonders bevorzugt wird dabei, dass die Einkopplung des Anregungslichts zu den Messbereichen (d) mit Hilfe von einer oder mehrere Gitterstrukturen (c) erfolgt, die in der optisch transparenten Schicht (a) ausgeprägt sind.

Gegenstand der Erfindung in allgemeinerer Form ist ein bioanalytisches Nachweisverfahren nach einer der vorgenannten Ausführungsformen, welches dadurch gekennzeichnet ist, dass eine oder mehrere flüssige Proben, umfassend aus lebenden Zellen erzeugte Vesikel (aus einem erfindungsgemässen bioanalytischen Reagens), mit damit assoziierten Rezeptoren, mit den in einem oder mehreren Messbereichen immobilisierten Liganden für diese Rezeptoren in Kontakt gebracht werden und eine Signaländerung als Folge einer Bindung der mit besagten Vesikeln assoziierten Rezeptoren an ihre immobilisierten Liganden gemessen wird.

Bevorzugt wird dabei, dass die Signaltransduktion von Rezeptoren, welche mit aus lebenden Zellen erzeugten Vesikeln (aus einem erfindungsgemässen bioanalytischen Reagens) assoziiert sind, nach Bindung dieser Rezeptoren an ihre immobilisierten Liganden gemessen wird, wobei diese Signaltransduktion beispielsweise durch Bindung weiterer Liganden an mit besagten Vesikeln assoziierte Rezeptoren oder andere induzierende Einwirkungen ausgelöst werden kann.

Beispielsweise kann in einer Vielzahl von Messbereichen eine einheitliche Art von Liganden immobilisiert sein. Sofern diese Messbereiche individuell oder mehrere dieser Messbereiche gemeinsam, beispielsweise innerhalb von mit dem festen Träger als Grundplatte ausgebildeten Probenbehältnissen (siehe hierzu auch weiter unten), fluidisch adressierbar sind, ergeben sich für die industrielle Forschung und Entwicklung interessante Screening-Methoden, welche auf der Verwendung eines erfindungsgemässen bioanalytischen Reagens beruhen. Beispielsweise können zu unterschiedlichen Messbereichen mit darin immobilisierten gleichartigen Liganden unterschiedliche, aus verschiedenen lebenden Zellen erzeugte Vesikel, mit gegebenenfalls unterschiedlichen damit assoziierten Rezeptoren und gegebenenfalls unterschiedlichen weiteren biologischen Verbindungen (Komponenten), hinzugegeben werden und das unterschiedliche Bindungsverhalten zu den immobilisierten Liganden in den diskreten Messbereichen untersucht werden.

Eine weitere bevorzugte Ausführungsform dieses erfindungsgemässen bioanalytischen Nachweisverfahrens ist dadurch gekennzeichnet, dass die Bindung von Rezeptoren, welche mit aus lebenden Zellen erzeugten Vesikeln (aus einem erfindungsgemässen bioanalytischen Reagens) assoziiert sind, an besagte immobilisierte Liganden in Kompetition zur Bindung dieser mit besagten Vesikeln assoziierten Rezeptoren an in Lösung befindliche Liganden stattfindet.

Sofern diese Messbereiche individuell oder mehrere dieser Messbereiche gemeinsam, beispielsweise innerhalb von mit dem festen Träger als Grundplatte ausgebildeten Probenbehältnissen (siehe hierzu auch weiter unten), fluidisch adressierbar sind, ist es beispielsweise auch möglich, zu individuellen Probenbghältnissen unterschiedliche Konzentrationen von aus lebenden Zellen erzeugten Vesikeln in einem erfindungsgemässen bioanalytischen Reagens hinzuzugeben und die Kompetition zwischen der Bindung der damit assoziierten Rezeptoren zu den immobilisierten Liganden und in Lösung befindlichen Liganden zu untersuchen. In einer anderen Variante ist es auch möglich, unterschiedliche Oberflächenkonzentrationen in Messbereichen immobilisierter Liganden zu erzeugen, hierzu eine einheitliche Konzentration von Vesikeln aus einem erfindungsgemässen bioanalytischen Reagens hinzuzugeben und dann wiederum die Kompetition zwischen der Bindung der damit assoziierten Rezeptoren zu den immobilisierten Liganden und in Lösung befindlichen Liganden zu untersuchen.

Für den Fachmann ist offensichtlich, dass sich aus den genannten Varianten erfindungsgemässer bioanalytischer Nachweisverfahren eine Vielzahl weiterer Möglichkeiten durch deren Kombination ergibt.

Während sich die genannten Varianten mit auf einem festen Träger immobilisierten Liganden und diesen in einer Probe zugeführten, aus lebenden Zellen erzeugten Vesikeln aus einem erfindungsgemässen bioanalytischen Reagens, besonders gut eignen für Nachweise, welche auf einer Änderung der Massenbelegung des festen Trägers beruhen (wie z. B. refraktive Methoden basierend auf der Änderung des effektiven Brechungsindex an der Oberfläche eines optischen Wellenleiters, siehe unten), ist es für andere Anwendungen vorteilhaft, aus lebenden Zellen erzeugte Vesikel, aus einem erfindungsgemässen bioanalytischen Reagens, auf einem festen Träger zu immobilisieren und diesen dann eine oder mehrere Proben (gegebenenfalls individuell adressierbar für verschiedene Messbereiche) mit darin nachzuweisenden Liganden zuzuführen. Für den Fachmann ist offensichtlich, dass sich aus dieser Umkehrung der Assay-Architektur zu den vorgenannten Varianten analoge Ausführungsmöglichkeiten des erfindungsgemässen bioanalytischen Nachweisverfahrens ergeben.

Eine grosse Gruppe von Ausführungsformen eines erfindungsgemässen bioanalytischen Nachweisverfahrens ist also dadurch gekennzeichnet, dass eine oder mehrere flüssige Proben mit den in einem oder mehreren Messbereichen immobilisierten, aus lebenden Zellen erzeugten Vesikeln (aus einem erfindungsgemässen bioanalytischen Reagens), mit damit assoziierten Rezeptoren, in Kontakt gebracht werden und eine Signaländerung als Folge der Bindung von Liganden oder einer anderen induzierenden Einwirkung auf besagte Rezeptoren gemessen wird.

Eine bevorzugte Ausführungsform ist dadurch gekennzeichnet, dass eine oder mehrere flüssige Proben mit den in einem oder mehreren Messbereichen immobilisierten, aus lebenden Zellen erzeugten Vesikeln (aus einem erfindungsgemässen bioanalytischen Reagens), mit damit assoziierten Rezeptoren, in Kontakt gebracht werden und die Signaltransduktion besagter Rezeptoren als Folge der Bindung von Liganden oder einer anderen induzierenden Einwirkung auf besagte Rezeptoren gemessen wird.

Kennzeichen einer speziellen Ausführungsform ist, dass die Bindung von Liganden aus einer zugegebenen flüssigen Probe an Rezeptoren, welche mit den immobilisierten, aus lebenden Zellen erzeugten Vesikeln (aus einem erfindungsgemässen bioanalytischen Reagens) assoziiert sind, in Kompetition zur Bindung dieser Liganden an in Lösung befindliche, gegebenenfalls mit Vesikeln assoziierte Rezeptoren stattfindet.

Eine besonders bevorzugte Ausführungsform eines erfindungsgemässen bioanalytischen Nachweisverfahrens ist dadurch gekennzeichnet, dass eine oder mehrere flüssige Proben, umfassend aus lebenden Zellen erzeugte Vesikel (aus einem erfindungsgemässen bioanalytischen Reagens), mit damit assoziierten Rezeptoren, mit den in einem oder mehreren Messbereichen immobilisierten Liganden für diese Rezeptoren in Kontakt gebracht werden, Anregungslicht von einer oder mehreren Lichtquellen gleicher oder unterschiedlicher Wellenlänge über eine oder mehrere Gitterstrukturen (c) zu den Messbereichen (d) eingekoppelt wird und die Änderung von ein oder mehr von den Messbereichen (d) ausgehenden optischen Signalen, als Folge einer Bindung der mit besagten Vesikeln assoziierten Rezeptoren an ihre immobilisierten Liganden, gemessen wird.

Eine andere besonders bevorzugte Ausführungsform ist dadurch gekennzeichnet, dass eine oder mehrere flüssige Proben mit den in einem oder mehreren Messbereichen (d) immobilisierten Vesikeln mit damit assoziierten Rezeptoren in Kontakt gebracht werden, Anregungslicht von einer oder mehreren Lichtquellen gleicher oder unterschiedlicher Wellenlänge über eine oder mehrere Gitterstrukturen (c) zu den Messbereichen (d) eingekoppelt wird und die Änderung von ein oder mehr von den Messbereichen (d) ausgehenden optischen Signalen, als Folge der Bindung von Liganden an besagte Rezeptoren oder von anderen induzierenden Einwirkungen auf besagte Rezeptoren, gemessen wird.

Hieraus ergeben sich weitere mögliche, unterschiedliche Ausfühmngs-formen des erfindungsgemässen bioanalytischen Nachweisverfahrens. Eine Gruppe von Ausführungsformen ist dadurch gekennzeichnet, dass besagte Änderungen von optischen Signalen aus den Messbereichen (d) hervorgerufen sind durch Änderungen des effektiven Brechungsindex im Nahfeld der optisch transparenten Schicht (a) in diesen Messbereichen und bei der jeweiligen Anregungswellenlänge gemessen werden.

Eine andere, bevorzugte Gruppe von möglichen Ausführungsformen des erfindungsgemässen bioanalytischen Nachweisverfahrens ist dadurch gekennzeichnet, dass es sich bei besagten Änderungen von optischen Signalen aus den Messbereichen (d) um Änderungen von ein oder mehreren Lumineszenzen gleicher oder unterschiedlicher Wellenlänge handelt, welche in besagten Messbereichen im Nahfeld der optisch transparenten Schicht (a) angeregt wurden und bei jeweils von der zugehörigen Anregungswellen-länge unterschiedlichen Wellenlängen gemessen werden.

Es wird bevorzugt, dass die einen oder mehreren Lumineszenzen und / oder Bestimmungen von Lichtsignalen bei der Anregungswellenlänge polarisationsselektiv vorgenommen werden, wobei vorzugsweise die einen oder mehreren Lumineszenzen bei einer anderen Polarisation als der des Anregungslichts gemessen werden.

Gegenstand der Erfindung in wiederum allgemeinerer Form ist ein bioanalytisches Nachweisverfahren nach einer der vorgenannten Ausführungsformen zur gleichzeitigen oder sequentiellen, quantitativen oder / und qualitativen Bestimmung eines oder mehrerer Analyten aus der Gruppe von Rezeptoren oder Liganden, Chelatoren oder "Histidin-tag-Komponenten", Enzymen, Enzymcofaktoren oder Inhibitoren.

Das erfindungsgemässe bioanalytische Nachweisverfahren nach einer der vorgenannten Ausführungsformen ist dadurch gekennzeichnet, dass die zu untersuchenden Proben beispielsweise wässrige Lösungen oder Oberflächenwasser oder Boden- oder Pflanzenextrakte oder Bio- oder Syntheseprozessbrühen sind oder aus biologischen Gewebeteilen entnommen sind oder aus Lebensmitteln, Geruchs- oder Aromastoffen oder aus Kosmetikstoffen stammen.

Weiterer Gegenstand der Erfindung ist ein fester Träger mit mindestens einem auf einer Oberfläche immobilisiertem, aus einer lebenden Zelle erzeugtem Vesikel aus einem erfindungsgemässen bioanalytischen Reagens nach einer der vorgenannten Ausführungsformen, umfassend mindestens einen Rezeptor, dadurch gekennzeichnet, dass ein von besagtem Rezeptor in besagter lebenden Zelle ausgelöster Mechanismus einer Signaltransduktion in besagtem Vesikel nach der Immobilisierung erhalten bleibt.

Eine bevorzugte Ausführungsform des erfindungsgemässen festen Trägers ist dadurch gekennzeichnet, dass Vesikel, umfassend jeweils mindestens einen Rezeptor, in diskreten Messbereichen (d) mit jeweils ein oder mehr Vesikeln immobilisiert sind.

Dabei wird bevorzugt, dass in einer Vielzahl von Messbereichen (d) Vesikel mit wenigstens zwei unterschiedlichen Arten von Rezeptoren immobilisiert sind, wobei innerhalb eines einzelnen Messbereichs vorzugsweise jeweils Vesikel mit einer einheitlichen Art von Rezeptoren immobilisiert sind.

Die Immobilisierung des mindestens einen aus einer lebenden Zelle erzeugten Vesikels auf der Oberfläche besagten festen Trägers kann beispielsweise mittels kovalenter Bindung oder mittels physikalischer Adsorption (elektrostatischer oder van-der-Waals-Wechselwirkung oder hydrophiler oder hydrophober Wechselwirkung oder einer Kombination dieser Wechselwirkungen) erfolgen.

Es wird bevorzugt, dass zwischen der Oberfläche besagten festen Trägers und dem mindestens einen darauf immobilisierten Vesikel eine Haftvermittlungsschicht aufgebracht ist. Dabei ist erfindungsgemäss die Haftvermittlungsschicht so gestaltet, dass auch nach der Immobilisierung von aus einer lebenden Zelle erzeugten Vesikeln als Bestandteil eines bioanalytischen Reagens, umfassend mindestens einen Rezeptor, auf besagter Haftvermittlungsschicht ein von besagtem Rezeptor in besagter lebenden Zelle ausgelöster Mechanismus einer Signaltransduktion in besagtem Vesikel erhalten bleibt.

Es wird bevorzugt, dass die Haftvermittlungsschicht eine Stärke von vorzugsweise weniger als 200 nm, besonders bevorzugt von weniger als 20 nm hat.

Die Haftvermittlungsschicht kann eine chemische Verbindung aus der Gruppe Silane, Epoxide, funktionalisierte, geladene oder polare Polymere und "selbstorganisierte funktionalisierte Mono- oder Mehrfachschichten" umfassen.

Eine bevorzugte Ausführungsform ist dadurch gekennzeichnet, dass die Haftvermittlungsschicht eine monomolekulare Schicht aus vorwiegend einer Sorte von Proteinen, wie beispielsweise Serumalbuminen oder Streptavidin, oder modifizierten Proteinen, wie z.b. biotinyliertes Serumalbumin, umfasst.

Kennzeichen einer anderen bevorzugten Ausführungsform ist, dass die Haftvermittlungsschicht selbstorganisierte, Alkan-endständige Mono-schichten aus vorwiegend einer Sorte von chemischen oder biochemischen Molekülen umfasst.

Besonders bevorzugt ist eine Ausführungsform, welche dadurch gekennzeichnet ist, dass die Haftvermittlungsschicht in Form einer Doppelschicht ausgebildet ist, umfassend eine erste Alkan-endständige, selbsorganisierte Ankerschicht und eine zweite Schicht, welche über Selbstorganisation ("self assembly") von synthetischen oder natürlichen Lipiden gebildet wird.

Die Immobilisierung des mindestens einen aus einer lebenden Zelle erzeugten Vesikels auf der Oberfläche besagten festen Trägers kann beispielsweise mittels kovalenter Bindung oder mittels physikalischer Adsorption (elektrostatischer oder van-der-Waals-Wechselwirkung oder hydrophiler oder hydrophober Wechselwirkung oder einer Kombination dieser Wechselwirkungen) erfolgen.

Eine besondere Ausführungsform des erfindungsgemässen festen Trägers mit einer besonders spezifischen Art der Immobilisierung der Vesikel ist dadurch gekennzeichnet, dass mit der Haftvermittlungsschicht biologische oder biochemische oder synthetische Erkennungselemente assoziiert sind, welche ein Vesikel mit oberffächenassozüerten biologischen oder biochemischen oder synthetischen Komponenten zur spezifischen Erkennung und Bindung, als Teil der entsprechenden, vorangehend beschriebenen spezifischen Ausführungsform eines erfindungsgemässen bioanalytischen Reagens, erkennen und binden. Beispielsweise können diese spezifischen Wechselwirkungen zur Erkennung und Bindung der Vesikel an deren Erkennungselemente auf der Haftvermittlungsschicht auf Wechselwirkungen mit Biotin / Streptavidin, sogenannten "Histidin-Tags", Zuckern oder Peptidaffinitäts-Wechselwirkungen beruhen, wobei ein beliebiger der jeweils zwei Bindungspartner mit der Vesikeloberfläche assoziiert ist und der andere Partner auf der Oberfläche besagter Haftvermittlungsschicht verankert ist.

Eine andere Ausführungsform des erfindungsgemässen festen Trägers ist dadurch gekennzeichnet, dass mindestens ein Ligand für einen Rezeptor, welcher an ein aus einer lebenden Zelle erzeugtes Vesikel, aus einem erfindungesgemässen bioanalytischen Reagens nach einer der genannten Ausführungsformen, gebunden ist, auf der Oberfläche dieses festen Trägers, gegebenenfalls über ein Spacer-Molekül, immobilisiert ist.

Dabei wird bevorzugt, dass in einer Vielzahl von Messbereichen (d) wenigstens zwei unterschiedliche Liganden für Rezeptoren, welche an ein aus lebenden Zellen erzeugte Vesikel, aus einem erfindungsgemässen bioanalytischen Reagens, gebunden sind, wobei innerhalb eines einzelnen Messbereichs vorzugsweise jeweils eine einheitliche Art von Liganden immobilisiert ist.

Besagte Liganden können auf der Oberfläche des festen Trägers mittels kovalenter Bindung oder mittels physikalischer Adsorption (z.B. elektrostatischer oder van-der-Waals-Wechselwirkung oder hydrophiler oder hydrophober Wechselwirkung oder einer Kombination dieser Wechselwirkungen) immobilisiert sein.

Bevorzugt wird, dass zwischen der Oberfläche des festen Trägers und besagten darauf immobilisierten Liganden eine Haftvermittlungsschicht aufgebracht ist.

Für die Auswahl der Haftvermittlungsschicht zur Immobilisierung besagter Liganden auf dem festen Träger gelten die gleichen Bevorzugungen, wie sie vorangehend für eine Haftvermittlungsschicht zur Immobilisierung von aus einer lebenden Zelle erzeugten Vesikeln aus einem erfindungsgemässen bioanalytischen Reagens genannt wurden.

Eine besonders bevorzugte Ausführungsform eines erfindungsgemässen festen Trägers ist dadurch gekennzeichnet, dass Bereiche zwischen den räumlich getrennten Messbereichen, mit darin immobilisierten, aus lebenden Zellen erzeugten Vesikeln (aus einem bioanalytischen Reagens nach einer der genannten Ausführungsformen) oder mit darin immobilisierten Liganden für Rezeptoren, welche an aus lebenden Zellen erzeugte Vesikel (aus einem bioanalytischen Reagens nach einer der genannten Ausführungsformen) gebunden sind, oder / und dass Bereiche innerhalb dieser Messbereiche, zwischen den genannten darin immobilisierten Verbindungen, zur Minimierung unspezifischer Bindung von Analyten oder deren Nachweissubstanzen "passiviert werden", d.h. dass zwischen den räumlich getrennten Messbereichen (d) oder / und dass innerhalb dieser Messbereiche (d) zwischen den genannten darin immobilisierten Verbindungen gegenüber dem Analyten "chemisch neutrale" Verbindungen aufgebracht sind, vorzugsweise beispielsweise bestehend aus den Gruppen, die von Albuminen, Casein, Detergentien - wie beispielsweise Tween 20 -, Detergentien-Lipid-Mischungen (aus synthetischen oder / und natürlichen Lipiden), synthetischen und natürlichen Lipiden oder auch hydrophilen Polymeren, wie beispielsweise Polyethylenglycolen oder Dextranen, gebildet werden.

Es ist auch möglich eine (zur Immobilisierung der biologischen oder biochemischen oder synthetischen Erkennungselemente) aktivierte Oberfläche (beispielsweise umfassend Poly-L-Lysin oder funktionalisierte Silane, beispielsweise mit Aldehyd- oder Epoxygruppen) zu passivieren, beispielsweise (im Falle von Aldehyd- oder Epoxygruppen) mittels Zugabe von reduzierenden Reagentien, wie beispielsweise Natriumborhydrat.

Das Material der Oberfläche besagten festen Trägers mit immobilisierten, aus lebenden Zellen erzeugten Vesikeln (aus einem erfindungsgemässen bioanalytischen Reagens nach einer der genannte Ausführungsformen) oder mit darauf immobilisierten Liganden für Rezeptoren, welche an aus lebenden Zellen erzeugte Vesikel (aus einem erfindungsgemässen bioanalytischen Reagens nach einer der genannte Ausführungsformen) gebunden sind, kann ein Material aus der Gruppe umfassen, die beispielsweise von form-, spritz- oder fräsbaren Kunststoffen, Kohlenstoffverbindungen, Metallen, wie beispielsweise Gold, Silber, Kupfer, Metalloxiden oder Silikaten, wie zum Beispiel Glas, Quarz oder Keramiken, oder Silizium oder Germanium oder ZnSE oder einer Mischung dieser Materialien gebildet wird.

Dabei kann besagter fester Träger in einer Vielzahl unterschiedlicher Ausführungsformen ausgebildet sein. Beispielsweise kann es sich hierbei um ein Glas- oder Mikroskop-Plättchen handeln. Es kann sich hierbei auch um eine Mikrotiter-Platte handeln, wie sie z.B. für Screening-Assays (zum Test, beispielsweise mittels klassischer Fluoreszenzmethoden oder mittels Fluoreszenz-Korrelationsspektroskopie, einer Vielzahl von Verbindungen) gebräuchlich ist.

Es wird bevorzugt, dass die Oberfläche besagten festen Trägers im wesentlichen planar ist.

Eine Gruppe bevorzugter Ausführungsformen eines erfindungsgemässen festen Trägers ist dadurch gekennzeichnet, dass es sich hierbei um eine optische oder elektronische Sensorplattform handelt.

Gegenstand der Erfindung ist daher auch eine Sensorplattform als fester Träger nach einer der vorgenannten Ausführungsformen, dadurch gekennzeichnet, dass besagter fester Träger mindestens in einem Wellenlängenbereich im ultravioletten bis infraroten Spektrum transparent ist und er vorzugsweise ein Material aus der Gruppe umfasst, die beispielsweise von form-, spritz- oder fräsbaren Kunststoffen, Kohlenstoffverbindungen, Metallen, Metalloxiden oder Silikaten, wie zum Beispiel Glas, Quarz oder Keramiken, oder Silizium oder Germanium oder ZNSe oder einer Mischung dieser Materialien gebildet wird.

Bevorzugt wird dabei eine solche Sensorplattform als fester Träger, welche dadurch gekennzeichnet ist, dass es sich hierbei um einen als Sensorplattform dienenden optischen Wellenleiter handelt.

Vorzugsweise ist die Sensorplattform als fester Träger dadurch gekennzeichnet, dass es sich hierbei um einen als Sensorplattform dienenden optischen Dünnschichtwellenleiter handelt, mit einer ersten optisch transparenten Schicht (a) mit Brechungsindex n₁ auf einer zweiten optisch transparenten Schicht (b) mit Brechungsindex n₂, wobei n₁ > n₂ ist.

Von Vorteil ist für viele Anwendungen eine solche Ausführunggsform einer erfindungsgemässen Sensorplattform als fester Träger, welche aufgeteilt ist in zwei oder mehr diskrete wellenleitende Bereiche.

Das Material der zweiten optisch transparenten Schicht (b) kann ein Material aus der Gruppe umfassen, welche von Silikaten, wie beispielsweise Glas oder Quarz, oder transparenten form-, spritz- oder fräsbaren, insbesondere thermoplastischen Kunststoffen, wie beispielsweise Polycarbonaten, Polyimiden, Polymethylmethacrylaten oder Polystyrolen gebildet wird.

Es wird bevorzugt, dass der Brechungsindex der ersten optisch transparenten Schicht (a) grösser als 1.8 ist.

Bevorzugt wird für eine Reihe von Anwendungen eine Ausführungsform der erfindungsgemässen Sensorplattform als fester Träger, welche dadurch gekennzeichnet ist, dass die erste optisch transparente Schicht (a) TiO₂, ZnO, Nb₂O₅, Ta₂O₅, HfO₂, oder ZrO₂, bevorzugt TiO₂, Ta₂O₅ oder Nb₂O₅ umfasst.

Die Dicke der ersten optisch transparenten Schicht (a) beträgt vorteilhaft 40 bis 300 nm, bevorzugt 100 bis 200 nm.

Eine weitere bevorzugte Ausführungsform der erfindungsgemässen Sensorplattform als fester Träger ist dadurch gekennzeichnet, dass sich zwischen den optisch transparenten Schichten (a) und (b) und in Kontakt mit Schicht (a) eine weitere optisch transparente Schicht (b') mit niedrigerem Brechungsindex als dem der Schicht (a) und einer Stärke von 5 nm - 10000 nm, vorzugsweise von 10 nm - 1000 nm, befindet.

Es wird bevorzugt, dass die Einkopplung von Anregungslicht in die optisch transparente Schicht (a) zu den Messbereichen (d) über ein oder mehrere optische Einkoppelelemente aus der Gruppe erfolgt, die von Prismenkopplern, evaneszenten Kopplern mit zusammengebrachten optischen Wellenleitern mit überlappenden evaneszenten Feldern, Stirnflächenkopplern mit vor einer Stirnseite der wellenleitenden Schicht angeordneten fokussierenden Linsen, vorzugsweise Zylinderlinsen, und Gitterkopplern gebildet wird. Besonders bevorzugt wird, dass die Einkopplung des Anregungslichts zu den Messbereichen (d) mit Hilfe von einer oder mehreren Gitterstrukturen (c) erfolgt, die in der optisch transparenten Schicht (a) ausgeprägt sind.

Eine Weiterentwicklung einer erfindungsgemässen Sensorplattformen nach einer der vorgenannten Ausftihrungsformen ist dadurch gekennzeichnet, dass diese zusätzlich ein oder mehrere Probenbehältnisse umfasst, deren Boden von besagter Sensorplattform gebildet wird, wobei besagte Probenbehältnisse mindestens im Bereich der einen oder mehreren Messbereiche in Richtung der Sensorplattform geöffnet sind, wobei besagte Probenbehältnisse auf der von der Sensorplattform abgewandten Seite offen oder bis auf Einlass- und / oder Auslassöffnungen geschlossen sein können.

Eine Vielzahl weiterer Ausführungsformen von Sensorplattformen, welche sich in Kombination mit einem erfindungsgenmässen bioanalytischen Reagens und für den Einsatz in einem erfindungsgemässen bioanalytiscehn Nachweisverfahren eignen, sind ausführlich beispielsweise in den Patenten US 5,822,472, US 5,959,292 und US 6,078,705 sowie in den Patentanmeldungen WO 96/35940, WO 97/37211, WO 98/08077, WO 99/58963 und PCT/EP 00/07529 beschrieben. Die darin beschriebenen Ausführungsformen von Sensorplattformen und Verfahren zum Nachweis eines oder mehrerer Analyten, sowie die darin beschriebenen optischen System und analytischen Systeme, sind als Bestandteil von erfindungsgemässen Sensorplattformen als festen Trägem mit einem erfindungsgemässen bioanalytischen Reagens sowie als Bestandteil von damit ausgeführten erfindungsgemässen bioanalytischen Nachweisverfahren ebenfalls Gegenstand der vorliegenden Erfindung.

Ein weiterer Gegenstand der vorliegenden Erfindung ist die Verwendung eines Vesikels aus einem erfindungsgemässen bioanalytischen Reagens nach einer der vorgenannten Ausführungsformen und / oder eines erfindungsgemässen festen Trägers mit mindestens einem darauf immobilisierten Vesikel nach einer der vorgenannten Ausführungsformen zur Aufkonzentrierung von Membranrezeptoren oder zur Aufkonzentrierung von eine immunologische Antwort auslösenden Proteinen (wie z.B. Antigenen) in einer zwei- oder dreidimensionalen Phase, welche beispielsweise nachfolgend in lebende Organismen eingebracht werden können, beispielsweise zur Stimulierung von Prozessen der Immunabwehr.

Weiterer Gegenstand der Erfindung ist die Verwendung eines Vesikels als Bestandteil eines erfindungsgemässen bioanalytischen Reagens nach einer der vorgenannten Ausführungsformen als Behältnis therapeutischer, diagnostischer, photosensitiver oder anderer biologisch wirksamer Substanzen zur Verabreichung in einem lebenden Organismus.

Die vorliegende Erfindung umfasst auch die Verwendung eines erfindungsgemässen bioanalytischen Reagens nach einer der vorgenannten Ausführungsformen und / oder eines erfindungsgemässen festen Trägers mit mindestens einem darauf immobilisierten Vesikel nach einer der vorgenannten Ausführungsformen und / oder eines erfindungsgemässen bioanalytischen Nachweisverfahrens nach einer der vorgenannten. Ausführungsformen zur Untersuchung von Rezeptor-Liganden-Wechselwirkungen, insbesondere zur Bestimmung der Bindungsstärke und kinetischer Parameter dieser Wechselwirkungen zwischen einem Rezeptor und seinem Liganden oder zur Bestimmung der Kanalaktivität eines Ionenkanalrezeptors nach Ligandenbindung oder anderer induzierender Einwirkungen auf besagten Rezeptor oder der enzymatischen Aktivität von mit einem Vesikel als Bestandteil eines erfindungsgemässen bioanalytischen Reagens assoziierten Enzymen oder zum Nachweis sekundärer Botenstoffe nach Ligandenbindung an einen Rezeptor und einer dadurch ausgelösten Signaltransduktion oder zur Untersuchung von Protein-Protein-Wechselwirkungen oder zum Nachweis von Proteinkinasen.

Ausserdem umfasst die vorliegende Erfindung die Verwendung eines erfindungsgemässen bioanalytischen Reagens nach einer der vorgenannten Ausführungsformen und / oder eines erfindungsgemässen festen Trägers mit mindestens einem darauf immobilisierten Vesikel nach einer der vorgenannten Ausführungsformen und / oder eines erfindungsgemässen bioanalytischen Nachweisverfahrens nach einer der vorgenannten Ausführungsformen zu quantitativen oder / und qualitativen Analysen zur Bestimmung chemischer, biochemischer oder biologischer Analyten in Screeningverfahren in der Pharmaforschung, der Kombinatorischen Chemie, der Klinischen und Präklinischen Entwicklung, zu Echtzeitbindungsstudien und zur Bestimmung kinetischer Parameter im Affinitätsscreening und in der Forschung, zu qualitativen und quantitativen Analytbestimmungen, insbesondere für die DNA- und RNA-Analytik, für die Erstellung von Toxizitätsstudien sowie für die Bestimmung von Expressionsprofilen sowie zum Nachweis von Antikörpern, Antigenen, Pathogenen oder Bakterien in der pharmazeutischen Produktentwicklung und -forschung, der Human- und Veterinärdiagnostik, der Agrochemischen Produktentwicklung und -forschung, der symptomatischen und präsymptomatischen Pflanzendiagnostik, zur Patientenstratifikation in der pharmazeutischen Produktentwicklung und für die therapeutische Medikamentenauswahl, zum Nachweis von Pathogenen, Schadstoffen und Erregern, insbesondere von Salmonellen, Prionen und Bakterien, in der Lebensmittel- und Umweltanalytik sowie zur Analyse und Qualitätskontrolle von Geruchs- und Geschmackstoffen, sowie Aromastoffen.

### Ausführungsbeispiele

Die vorliegenden Beispiele beschreiben die Herstellung eines erfindungsgemässen bioanalytischen Reagens mit mindestens einem Vesikel, das aus einer lebenden Zelle erzeugt wurde, umfassend mindestens einen Rezeptor, dadurch gekennzeichnet, dass ein von diesem Rezeptor in der verwendeten lebenden Zelle ausgelöster Mechanismus einer Signaltransduktion in besagtem Vesikel als Bestandteil des bioanalytischen Reagens erhalten bleibt.

In diesen Beispielen wird gezeigt,
- dass Membranrezeptoren von Membranen lebender Zellen auf Membranen "nativer" Vesikel ohne Funktionalitätsverlust transferiert werden können
- dass Komponenten aus dem Lumen der vesikelbildenden Zelle auf die gebildeten, "nativen" Vesikel ohne einen Austausch mit umgebendem Medium übertragen werden können
- dass für Sekundärantworten notwendige Lumineszenzindikatoren in "native" Vesikel eingeschleust werden können
- und dass die übertragenen Komponenten einschliesslich der Indikatoren stabil im Inneren der "nativen" Vesikel eingeschlossen bleiben.

### Beispiel 1: Herstellung von "nativen" Vesikeln als Bestandteil eines erfindungsgemässen bioanalytisches Reagens und Visualisierung der hergestellten Vesikel

### 1.1. Herstellung von Vesikeln definierter Grösse

Zur Herstellung von Vesikeln ("Vesikulierungsprozess") wurden adhärent wachsende HEK293 (human embryonic kidney) Zellen in jeweils 15 ml DMEM/F12 (Dulbecco's modified Eagle medium, Gibco BRL Life Technologies) in 75 ml T-Flaschen (TPP, Schweiz) kultiviert. Das Medium wurde dabei mit 2.2% fötalem Kälberserum (Gibco BRL Life Technologies) versetzt. Die Zellkulturen wurden in einem Inkubator aufbewahrt (37°C, 5% CO₂).

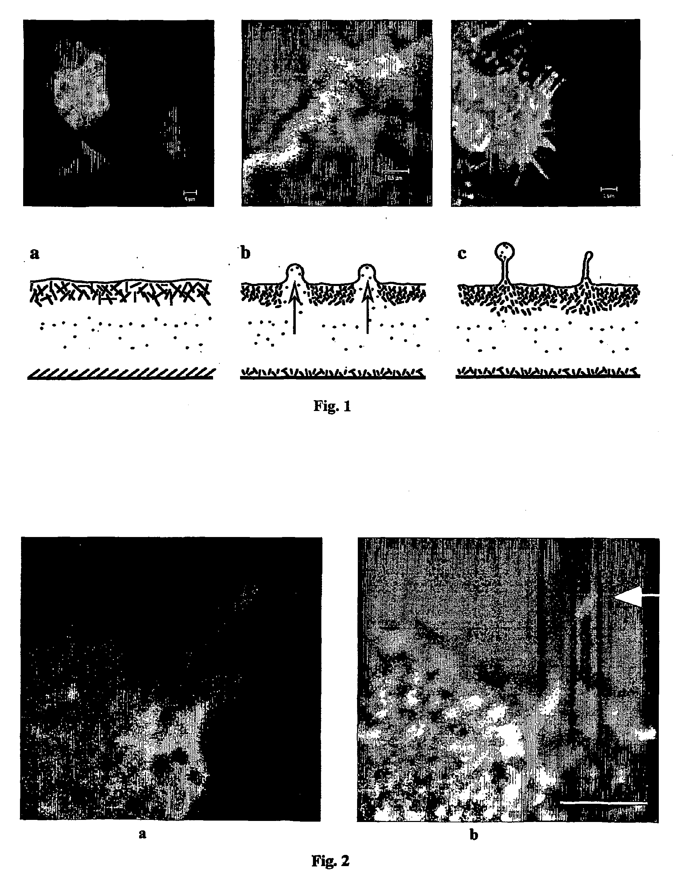

Zum Sichtbarmachen des cytoplasmatischen Zellinhalts während des Vesikulierungsprozesses wurden HEK293-Zellen mit *Aequorea victoria* GFP (Green Fluorescent Protein) kodierenden Plasmid-DNA (Clontech; Palo Alto, CA, USA) auf Basis der gängigen Calciumphosphat-DNA-Copräzipitation (Jordan M., Schallhorn A., Wurm F. M-: "Transfecting mammalian cells: optimization of critical parameters affecting calcium-phosphate precipitate formation", Nucleic Acids Res. 24 (1996) 596-601) transfiziert. Zwanzig Stunden nach der Transfektion war die grüne Fluoreszenz des GFP im gesamten Zellinhalt, nach Anregung bei 488 nm, in einem konfokalen Fluorreszenzmikroskop sichtbar. Diese Kontrolle wurde vor allem angewendet, um nachzuweisen, dass während des Vesikulierungsprozesses der Zell- und Vesikelinhalt intakt bleibt, d. h. dass es zu keinem Kontakt bzw. zu keiner Vermischung mit dem umgebenden äusseren Zellmedium kommt (siehe Figur 1).

Zur Vesikulierung wurde das im Kulturmedium enthaltene Serum durch Dekantierung oder, im Falle von Suspensionszellen, durch Zentrifugation (Rotor SS34 Sorvall, bei 120 g; Zentrifugationsdauer 5 Minuten) entfernt und gegen serumfreies DEMEM-Medium ausgetauscht. Die Zellkonzentration lag zwischen 1 x 10⁷ und 5 x 10⁷ Zellen pro Milliliter.

Cytochalasin B oder D (Sigma-Aldrich) (Stammlösung mit einer Konzentration von 2 mg/ml in DMSO) wurde zu vorgewärmtem DMEM-Medium in einer Endkonzentration von 20 µg/ml zugegeben. Der Vesikulationsprozess war abhängig von Zellalter und Zelllinie und betrug zwischen 5 und 60 Minuten. Anschliessend wurden Scherkräfte appliziert (1 Min. Vortexen), um möglichst vollständig an der Zelloberfläche verbleibende Vesikel abzulösen. Diese Suspension wurde dann durch einen sterilen Spritzenfilter filtriert (5 µm Porengrösse; Acrodisc), um verbleibende Zellkörper von den Vesikeln abzutrennen. Die Vesikelsuspension wurde anschliessend durch Zentrifugation aufkonzentriert (Rotor SS 34, Sorvall, bei 5400 UpM (Umdrehungen pro Minute; 20 Min.).

Das erhaltene Vesikelpellet wurde dann in 500 µl Phosphatpuffer (pH 7.5) resuspendiert und auf einen vorgeformten Succrosegradienten geladen. Dieser Gradient bestand aus drei Sucroseschichten mit abnehmender Dichte (von unten nach oben 2 M, 1.5 M, 1.3 M Succrose in deionisiertem, sterilem Wasser). Nach 90-minütiger Zentrifugation bei 25000 UpM in einem Schwingrotor (TST 60.4 bei 84.840 g; Sorvall; Kontron Ultrazentrifuge) waren drei deutlich separierte Banden sichtbar. Diese Banden enthielten Vesikel von verschiedender Grösse. Die Vesikelmembranen wurden durch Anfärbung mit Octadecyl Rhodamin B Chlorid (R18) (Molecular Probes Inc., USA) sichtbar gemacht, um mit Hilfe dieser Fluoreszenzmarkierung die Grösse der Vesikel in einem konfokalen Fluoreszenzmikroskop (Anregung: 560 nm / Emission: 590 nm) auszumessen. Die oberste Bande enthielt Vesikel mit einer Grösse von 100 - 300 nm Durchmesser, die mittlere Bande solche mit einem Durchmesser von 500 - 800 nm und die unterste Bande Vesikel mit einer Grössenverteilung zwischen 1µm und 3 µm.

Damit waren die so hergestellten "nativen" Vesikel kleiner als beispielsweise rote Blutkörperchen (3 - 5 µm). Die Vesikel aus den beiden oberen Banden waren sogar kleiner als Mitochondrien.

Wie nachfolgend gezeigt wird, enthalten die "nativen Vesikel" Teile des Endoplasmatischen Reticulums. Aufgrund ihrer Grösse jedoch sind der Zellkern und Mitochondrien (bei Vesikeln mit einem Durchmessr von weniger als 800 nm) von der Inkorporation in die Vesikel ausgeschlossen. Im Falle von G-Protein-gekoppelten Rezeptoren (GPCR) folgt aus dieser Grössenverteilung beispielsweise, dass die Vesikel nur das Endoplasmatische Reticulum, als allerdings wichtigstes Reservoir intrazellulärer Calciumionen und als essentiellen Bestandteil der GPCR-Signaltransduktionskaskade, enthalten können.

Für Experimente zur Untersuchung der nach der Präparation verbliebenen Fähigkeit der Rezeptoren zur Ligandenbindung wurden Vesikel der obersten Bande verwendet, während für Nachweise der Freisetzung von sekundären Botenstoffen, wie Ca²⁺ und cAMP, Vesikel der beiden unteren Fraktionen eingesetzt wurden, da sie in einem grösseren Ausmass mit geeigneten Fluoreszenzindikatoren beladen werden können.

Der Prozess der Bildung von Zellausstülpungen und Zellabschnürungen bei der Vesikelbildung ist in dem unterhalb der Fluoreszenzmikroskop-Aufnahmen befindlichen Diagramm (Figur 1) schematisch illustriert: (a) Normales Aktin-Filamentnetz im Zellkortex. (b) Die Aktinfilamente ziehen sich einige Minuten nach Applikation von Cytochalasin B/D (Konzentration 20 µg/ml) an gewissen Stellen zurück, und das Endoplasma der Zelle kann sich lokal ausdehnen, was (c) Zellvorstülpungen und Abschnürungen zur Folge hat.

### 1.2. Nachweis des Endoplasmatischen Reticulums in "nativen" Vesikeln

Zum Nachweis des Endoplasmatischen Reticulums in nativen Vesikeln, als Hauptvoraussetzung zur Freisetzung von sekundärem Ca²⁺ nach Aktivierung der Signaltransduktionskaskade von G-Protein-gekoppelten Rezeptoren - wurde das grün fluoreszierende Protein (GFP) von *Aequorea victoria* auf der Ebene der kodierenden DNA mit einer Peptidsignalsequenz (MRLCIPQVLLALFLSMLTAPGEG) versehen, die das GFP während seiner Synthese in der Zelle in das Endoplasmatische Reticulum leitet. Dieser molekularbiologische Eingriff hatte keinen nachteiligen Einfluss auf die Vitalität der kultivierten HEK293-Zellen. Die Überlappung der GFP-Fluoreszenz mit den geometrischen Abmessungen des Endoplasmatischen Reticulums wurde mittels des kommerziellen lipophilen ER-Farbstoffs (1,1'-Dihexadecyl-3,3,3',3'-tetramethylindocarbocyanine perchlorat (DiIC16(3); Molecular Probes) in ganzen Zellen überprüft. Anhand der Fluoreszenz der GFP-Moleküle konnten Teile des Endoplamatischen Reticulums in nativen Vesikeln mit Hilfe konfokaler Bildschnitte mikroskopisch nachgewiesen werden (Figur 2).

### 1.3. Herstellung und Nachweis von "nativen" Vesikeln mit (A) inkorporiertem, fluoreszenzmarkiertem G-Protein sowie (B) mit inkorporierten Indikatorfarbstoffen

**(A)** Es wurden "native" Vesikel mit der rekombinanten G-alpha-Untereinheit eines G-Proteins (G_{α}) hergestellt. Um das Vorhandensein dieses Proteins in lebenden HEK293-Zellen mit Hilfe des konfokalen Fluoreszenzmikroskops nachweisen zu können, wurde das G-alpha-15 Protein (G_{α15}) auf der Ebene der kodierenden DNA mit dem *Aequorea victoria* GFP fusioniert. Dazu wurde die für EGFP (Enhanced Green Fluorescent Protein; Clontech) kodierende DNA in die G_{αq} Untereinheit eingesetzt. Das endstandene Fusionsprotein wurde mit Hilfe der oben genannten Calciumphosphat-Präzipitationsmethode in HEK293-Zellen transfiziert. 24 Stunden nach der Transfektion war das grün markierte G_{α}-Protein im Cytoplasma nachweisbar (wie in Abbildung la zu Beispiel 1.1). Mit Hilfe konfokaler Mikroskopie konnten die rekombinant exprimierten Proteinen in der Nähe der Zellmembran lokalisiert werden.
   Nach Zugabe von Cytochalasin B (2 mM) zu den G-Protein exprimierenden HEK293-Zellen schnürten sich Plasmamembran-Vesikel ab, die das grün fluoreszierende G_{α} Fusionsprotein inkorporiert hatten.
**(B)** Es wurden "native" Vesikel mit inkorporierten, chemischen Fluoreszenzindikatoren hergegestellt. Dazu wurden HEK-Zellen mit den Calcium-sensitiven Indikatoren (Fura-Rot, C47H52N4O24S, 149732-62-7 Glycine, N-[2-[(acetyloxy)methoxy]-2-oxoethyl]-N-[5-[2-[2-[bis[2-[(acetyloxy)methoxy]-2-oxoethyl]amino]- 5-methylphenoxy]ethoxy]-2-[(5-oxo-2-thioxo-4-imidazolidinylidene) methyl]-6-benzofuranyl]-, (acetyloxy)methyl ester und Fluo-3/AM (C36H45Cl2N7O13; 121714-22-5 Glycine, N-[4-[6-[(acetyloxy)methoxy]- 2,7-dichloro-3-oxo-3H-xanthen-9-yl]-2-[2-[2-[bis[2-[(acetyloxy) methoxy]-2-oxyethyl]amino]-5-methylphenoxy]ethoxy]phenyl]-N-[2-[(acetyloxy)methoxy]- 2-oxyethyl]-, (acetyloxy)methyl ester; Molecular Probes) beladen. Pro Milliliter Zellnährkulturmedium (DMEM/F12: Dulbecco's modified Eagle Medium F12) wurden 12.5µg Fluo-3/AM und 24.5µg Fura-Rot zugegeben. Die ionensensitiven Indikatorfarbstoffe waren zuvor in DMSO gelöst worden. Die Ladungszeit der Acetoxymethylester betrug 60 Minuten bei 37°C. Zugabe von Cytochalasin führte zur Abschnürung von Vesikeln mit deutlicher Indikatorbeladung. Beide Indikatoren waren im Fluoreszenzmikroskop klar sichtbar. Über die Analyse der gemessenen Fluoreszenzintensitäten wurde die Konzentration an freien Ca²⁺ - Ionen im Innern der Vesikel wie auch die Konzentration an freien Ca²⁺ - Ionen im Zytosol der Ursprungszelle gemessen. Beide waren vergleichbar und betrugen 130 nM.

### 1.4. Herstellung von Vesikeln mit inkorporierten, rekombinanten Fusionsproteinen aus G-Proteinen und fluoreszenzmarkierten Proteinen sowie inkorporierten Indikatorfarbstoffen

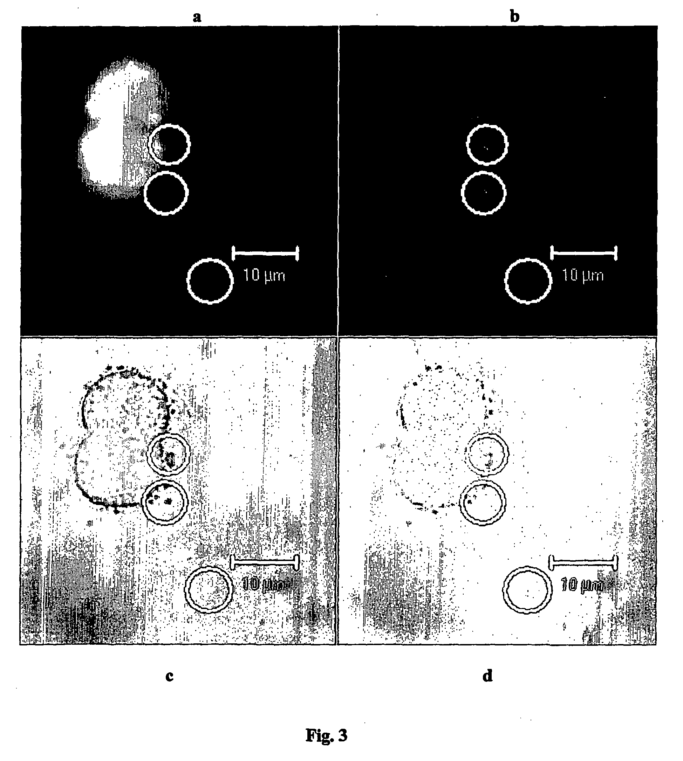

Auf DNA Ebene wurde die für EGFP (Enhanced Green Fluorescent Protein; Clontech) kodierende in die G_{αq}- Untereinheit eingesetzt. Das endstandene Fusionsprotein wurde mit Hilfe der oben genannten Calciumphosphat-Präzipitationsmethode in HEK293-Zellen transfiziert. 24 Stunden nach der Transfektion war das grün markierte G_{α}-Protein im Cytoplasma nachweisbar und zeigte neben dem Auftreten diffuser Bereiche häufig eine Tendenz zur Ausbildung von Aggregaten (0.5 - 1 µm im Durchmesser) nahe der Plasmamembran. Nach Zugabe von Cytochalasin B (2 mM) zu den G-Protein exprimierenden HEK293-Zellen schnürten sich Plasmamembran-Vesikel ab, die das grün fluoreszierende G_{α} -Protein inkorporiert hatten (Abbildung 3). Die Zellen wurden mit Calcium-sensitiven Indikatoren (Fura-Rot und Fluo-3/AM) beladen. Pro Milliliter Zellnährkulturmedium (DMEM/F12: Dulbecco's modified Eagle Medium F12) wurden 12.5µg Fluo-3/AM und 24.5µg Fura-Rot zugegeben. Die ionensensitiven Indikatorfarbstoffe waren zuvor in DMSO gelöst worden. Die Ladungszeit der Acetoxymethylester betrug 60 Min. bei 37°C. Zugabe von Cytochalasin führte zur Abschnürung von indikatorbeladenen Vesikeln. Beide Indikatoren waren im Vesikellumen durch das Fluoreszenzmikroskop deutlich sichtbar (Figur 3). Die Analyse der gemessenen Fluoreszenzintensitäten ergab eine Konzentration an freien Ca²⁺ - Ionen von 130 nM, wie er auch bei der Bestimmung des freien Ca²⁺ - Ionen im Zytosol einer Zelle gemessen wird.

### Beispiel 2: Herstellung von signaltransduktionsfähigen "nativen" Vesikeln mit inkorporierten Rezeptoren und deren Nachweis

### 2.1. Inkorporation von Ionenkanal-Rezeptoren in "native" Vesikel unter Erhaltung von deren Funktionalität, am Beispiel des 5HT_{3A}-Rezeptors

Im den folgenden Ausführungsbeispielen wird der 5HT₃ Serotonin-Rezeptor als repräsentativer ligandengesteuerter Ionenkanal verwendet. In der Literatur werden zwei verschiedene Formen des 5HT₃ Rezeptors beschrieben, der 5HT_{3A} und der 5HT_{3B} Rezeptor (Davies P. A., Pistis M., Hanna M. C., Peters J. A., Lambert J. J., Hales T. G., Kirkness E. F., "The 5-HT3B subunit is a major determinant of serotonin-receptor function", Nature 397 (1999) 359-363). In den folgenden Ausführungsbeispielen wird nur der 5HT_{3A} Rezeptor verwendet.

Verschiedene rekombinante Konstrukte des Serotonin (5HT_{3A}) Rezeptors wurden in HEK293-Zellen exprimiert, unter Kontrolle des menschlichen Cytomegalo-Virus-Gen-Promoters. Transiente Expression des vollen Wildtyp-Rezeptors wurde erreicht durch Co-Transfektion des eukaryotischen Expressionsvektor CMVβ (Clontech, Palo Alto, CA) mit den Rezeptor kodierender cDNA und / oder mit entsprechender cDNA für cytosolisches GFP, um Zellen zu identifizieren, die gleichzeitig den Serotonin-Rezeptor (5-HT_{3A} R) exprimierten.

16 bis 20 Stunden vor der Transfektion wurden HEK293-Zellen (10⁵ Zellen/ml) in 6-Well-Platten oder, im Falle von Proben für eine spätere Untersuchung im konfokalen Fluoreszenzmikroskop, auf sterilen Deckglässchen (Durchmesser 22 mm) in 6-Well-Platten, ausgesät. Die Zellen wurden transfiziert mittels *Effectene* (Qiagen, Hilden, Deutschland) entsprechend der Herstelleranleitung. Nach vier Stunden Transfektion in befeuchteter Atmosphäre (5% CO₂, 37°C) wurde das Transfektionsmedium durch frisches Zellkulturmedium ausgetauscht.

Die Herstellung der Vesikel aus den Zellen erfolgte 48 Stunden nach der Transfektion in ähnlicher Weise, wie es in Beispiel 1.1 beschrieben ist. Dazu wurden die Zellen in zwei 6-Well-Platten unter leichtem Rühren ein bis zwei Minuten lang einer Trypsin-EDTA-Lösung (Sigma) (0.5 - 1 ml pro Well) ausgesetzt. Der Well-Inhalt wurde dann 5 Min. lang zentrifugiert (1200 UpM, Rotor SLA-600, Sorvall, Newtown, USA). Der Überstand wurde verworfen und das erhaltene Pellet in 10 ml in PBS-Puffer (10 mM Phosphatpufferlösung mit Na₂HPO₄, K₂HPO₄, 138 mM NaCL, 2.7 mM KCl, pH 7.4) unter Vortexen resuspendiert. Die Erzeugung der Vesikel aus dem Zell-Präparat wurde ausgelöst durch Zugabe von Cytochalasin aus einer Stammlösung in DMSO (4 mg/ml) bis zu einer Endkonzentration von 20 µg/ml. Zur Abtrennung der als Folge abgeschnürten Vesikel von den Zellen wurden wiederum mittels Vortexen (2 Min.) Scherkräfte appliziert, bevor abschliessend die Suspension nochmals suspendiert und dann der Überstand mit den darin enthaltenen Vesikeln durch einen Spritzenfilter filtriert wurde (1.2 µm Porengrösse, Acrodisc).

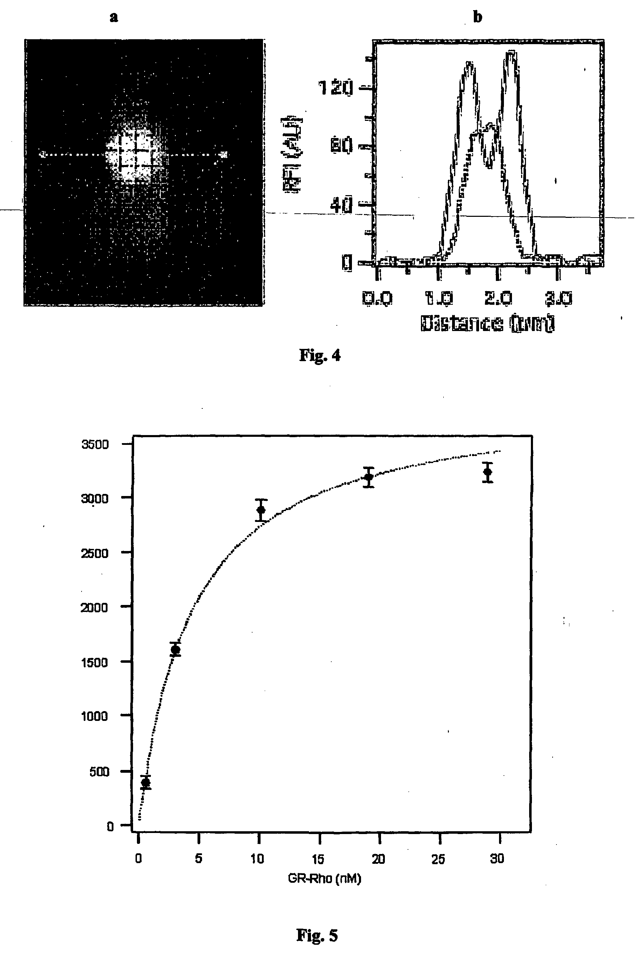

Entsprechend obiger Beschreibung wurden "native" Vesikel mit darin enthaltenem, exprimiertem Rezeptor hergestellt. Die Anwesenheit des 5HT_{3A}-Rezeptors in der äusseren Vesikelmembran wurde durch Markierung dieses Rezeptors mit rezeptorspezifischem Liganden GR-Cy5 (= 1,2,3,9-Tetrahydro-3-[(5-Methyl-1*H*-Imidazol-4-yl)methyl]-9-(3-Amino-(N-Cy5-amide)-propyl)-4*H-*Carbazol-4-on) nachgewiesen. Intensitätsprofile der Fluoreszenz von fluoreszenzmarkierten Rezeptoren und von GFP, auf die Vesikel übertragen aus dem GFP-markierten cytosolischen Zellinhalt, beweisen eindeutig die Existenz des Rezeptors in der "nativen" Vesikelmembran und somit seine Herkunft aus der Plasmamembran der vesikelbildenden Säugerzelle. Die Anwesenheit von GFP im "nativen" Vesikel weist eindeutig die cytosolische Herkunft aus der Zelle nach (Abbildung 4).

### 2.1.1 Nachweis der Ligandenbindungsfähigkeit des 5HT_{3A}-Rezeptors in "nativen" Vesikeln in Lösung

Die Fähigkeit von 5HT_{3A} Rezeptoren zur spezifischen Ligandenbindung, als deren "primäre" Funktionalität, nach Inkorporation der Rezeptoren in "native" Vesikel wurde mittels eines kompetitiven radiologischen Bindungsassays untersucht (Tairi A. P., Hovius R., Pick H., Blasey H., Bernard A., Surprenant A., Lundstrom K., Vogel H., "Ligand binding to the serotonin 5HT3 receptor studied with a novel fluorescent ligand", Biochemistry 37 (1998) 15850-15864 ; Wohland T., Friedrich K., Hovius R., Vogel H., "Study of ligand-receptor interactions by fluorescence correlation spectroscopy with different fluorophores: evidence that the homopentameric 5-hydroxytryptamine type 3As receptor binds only one ligand", Biochemistry 38 (1999) 8671-8681).

Dazu wurden Proben des besagten Reagens mit den in Lösung befindlichen Vesikeln für 60 Minuten bei Raumtemperatur mit 1.5 nM des Tritium-gelabelten Liganden [³H]-GR65630 in 240 µl HEPES-Puffer (10 mM HEPES, pH 7.4) (HEPES: 4-(2-hydroxyethyl)piperazine-1-ethanesulfonic acid) in 96-Well-MultiScreen-Platten (Millipore, F-Molsheim) inkubiert. Die Inkubation wurde durch schnelles Filtern und anschliessendes 3-faches Waschen mit jeweils 300 µl eiskaltem HEPES-Puffer abgeschlossen. Die Filter wurden in Szintillationsgefässe transferiert und in jeweils 1 ml Ultima Gold TM (Packard, meridan, USA) aufgenommen. Die Radioaktivität wurde mit einem TriCarb 2200CA Flüssigkeits-Szintillationszähler gemessen. Das Ausmass unspezifischer Bindung an den Rezeptor und an die Vesikel wurde in Anwesenheit eines hohen Überschusses von Quipazin (1 µM) abgeschätzt.. Die Dissoziationskonstante K_{d} wurde bei 6 verschiedenen Konzentrationen des Liganden [³H]-GR65630 aus Scatchard-Plots bestimmt. Das Bindungsverhalten ("Pharmakologie") des Rezeptors wurde durch Kompetition von verschiedenen pharmakologisch aktiven Substanzen mit der Bindung des radioaktiv markierten Referenzliganden [³H]-GR65630 in Form von IC₅₀ Werten (Konzentration bei 50% Inhibierung) ermittelt. Alle Experimente wurden zweifach durchgeführt. Zu Vergleichszwecken wurden Experimente unter vergleichbaren Bedingungen auch an den ganzen Mutterzellen durchgeführt. Die folgende Tabelle fasst die experimentell bestimmten Dissoziationskonstanen zusammen.

| | | Rezeptor in Mutterzelle | Rezeptor in "nativen" Vesikeln |
|---|---|---|---|
| | | pKᵢ | pKᵢ |
| *Antagonist* | | | |
| | GR-H | 10.2 ± 0.1 | 9.7 ± 0.1 |
| | Granisetron | 9.2 ± 0.1 | 9.5 ± 0.1 |
| | Ondansetron | 8.5 ± 0.1 | 8.6 ± 0.1 |
| *Agonist* | | | |
| | Quipazine | 9.3 ± 0.1 | 9.4 ± 0.2 |
| | mCPBG | 8.2 ± 0.1 | 8.3 ± 0.1 |
| | 5HT | 7.5 ± 0.1 | 7.7 ± 0.1 |
| | PBG | 6.5 ± 0.1 | 6.6 ± 0.1 |
| | [³H]-GR65630 | 9.0 ± 0.1 | 9.4 ± 0.1 |

### Erklärung der Kurzbezeichnungen:

### Antagonist

| | |
|---|---|
| GR-H | 1,2,3,9-tetrahydro-3-[(5-methyl-1H-imidazol-4-yl)methyl)]-9-(3-aminopropyl)-4H-carbazol-4-one |
| | |
| Granisetron : | endo-N-(9-methyl-9-azabicyclo[3.3.1]non-3-yl)-1-methyl-1H-indazole-3-carboxamide hydrochloride |
| Ondansetron : | 1,2,3,9-tetrahydro-9-methyl-3-[(2-methyl-1H-imidazol-1-yl)methyl]-4H-carbazol-4-one |
| | |
| Agonist: | |
| Quipazine : | 2-(1-Piperazinyl)quinoline |
| m-CPBG : | m-Chlorophenylbiguanide |
| 5-HT : | 5-Hydroxytryptamine |
| PBG: | Phenylbiguanide |
| | |
| [³H]-GR65630 | siehe: Kilpatrick G. J., Jones G.J., Tyers M. B., (1988) "The distribution of specific binding of the 5-HT3 receptor ligand [3H]-GR65630 in rat brain using quantitative autoradiography", Neuroscience Letters 94 (1988) 156-160. |

### 2.1.2 Nachweis der Ligandenbindungsfähigkeit des 5HT_{3A}-Rezeptors in oberflächenimmobilisierten "nativen" Vesikeln

Die Ligandenbindungexperimente an oberflächenimmobilisierten Vesikel wurden mit dem fluoreszenzmarkierten Liganden GR-Cy5 durchgeführt. Für diese Experimente wurde ein homologes Serotoninrezeptor-Konstrukt 5HT_{3A} verwendet, das mit einem am Ende einer Untereinheit fusionierten EGFP (Enhanced Green Fluorescent Protein, Clontech) exprimiert wurde. Die hergestellten "nativen" Vesikel wurden auf Deckgläsern mittels physikalischer Adsorption der Vesikel auf den Gläsern immobilisiert, durch zweistündige oder Übernacht-Inkubation von typischerweise 400 µl einer nach obiger Methode hergestellten Lösung einer Vesikelpräparation in 6-Well-Platten mit Deckgläsern an deren Boden bei 4°C. Dann wurden die Deckgläser mit den darauf immobilisierten Vesikeln aus den Platten entfernt und in eine offene Zelle eingesetzt, welche dann mit 200- 300 µl PBS-Puffer gefüllt wurde.

Die Affinität eines fluoreszenten Liganden zu den Vesikel-gebundenen 5HT_{3A}-Rezeptoren, in einem Experiment mit einer Vielzahl von Vesikeln, wurde untersucht, indem eine Serie von Deckgläsern jeweils 2 Stunden lang mit Vesikeln in Lösung und ansteigenden Konzentrationen von fluoreszenten Liganden bei Raumtemperatur inkubiert wurde. Eine zweite Serie von Deckgläsern wurde unter gleichen Bedingungen, zur Abschätzung des Ausmasses von unspezifischer Bindung der fluoreszenten Liganden an die Vesikel, ebenfalls mit Vesikeln in Lösung und gleichen, ansteigenden Konzentrationen von fluoreszenten Liganden sowie zusätzlich 5 µM Quipazin, als Kompetitor in hohem Überschuss (> 150-fach), inkubiert.

Die Affinität des fluoreszenten Liganden zu an einzelne, diskrete Vesikel gebundene Rezeptoren wurde bestimmt durch sequentielle Zugabe ansteigender Konzentrationen des fluoreszenten Liganden auf ein- und dasselbe Deckglas mit darauf durch Übemacht-Inkubation immobilisierten Vesikeln. Die Durchführung der Untersuchungen erfolgte in analoger Weise wie oben beschrieben für eine Vielzahl von Vesikeln.

Die Fluoreszenzintensitäten des Liganden wurden mit einem konfokalen Fluoreszenzmikroskop (Zeiss, Laser Scanning Microscope LSM510), unter Verwendung eines geeigneten Filtersatzes, gemessen. Das Fluoreszenzsignal des zusätzlich in die Vesikel inkorporierten GFP wurde jeweils benutzt, um vor einer Liganden-Zugabe das Mikroskop auf den Arbeitsabstand der Ebene zu justieren, in der sich die Vesikel befanden. Die Fluoreszenzsignale der Liganden (rot) wurden über die Fluoreszenzsignale des EGFP (grün) entsprechend der Anzahl von aktiven Rezeptoren pro Vesikel referenziert. Die dargestellten Bilder wurden aus den mit einem Photomultiplier erfassten Signalen des Fluoreszenzmikroskops erzeugt. Für die Bildauswertung wurden sogenannte "Regions of Interest" (ROIs) der Bilder festgelegt, deren Abmessungen den auszumessenden Arealen angepasst wurden.

Die Resultate dieses Experimentes zeigen, dass Vesikel unterschiedliche Zahlen an assoziierten 5HT_{3A}-Rezeptoren, mit der Folge deutlich unterschiedlicher Fluoreszenzintensitäten von verschiedenen Vesikeln, aufweisen, aber die Bindungskonstanten identische Werte ergaben, welche mit Literaturwerten übereinstimmen, welche mit isolierten Rezeptoren oder an vollständigen Zelle bestimmt wurden (Tairi A. P., Hovius R., Pick H., Blasey H., Bernard A., Surprenant A., Lundstrom K., Vogel H., "Ligand binding to the serotonin 5HT3 receptor studied with a novel fluorescent ligand", Biochemistry 37 (1998) 15850-15864 ; Wohland T., Friedrich K., Hovius R., Vogel H., "Study of ligand-receptor interactions by fluorescence correlation spectroscopy with different fluorophores: evidence that the homopentameric 5-hydroxytryptamine type 3As receptor binds only one ligand", Biochemistry 38 (1999) 8671-8681).

Als Beispiel sind in Figur 5 die gemessenen Fluoreszenzintensitäten als Funktion ansteigender Konzentrationen von GR-Rho (1,2,3,9-tetrahydro-3-[(5-methyl-1*H*-imidazol-4-yl)methyl]-9-(3-amino-(N-rhodamineB-thiocarbamoyl)-propyl)-4*H-*carbazol-4-on), als fluoreszentem Liganden dargestellt (nach Subtraktion der beobachteten Fluoreszenzintensität Fo in Abwesenheit von GR-Rho). Aus dem Fit der Messdaten mit der Gleichung einer Langmuir-Isotherme ergab sich ein Wert von K_{d} = (4.2 +/- 1.7) nM für die Dissoziationskonstante des Systems bestehend aus dem HT₃-Rezeptor und dem GR-Rho-Liganden (Figur 5).

### 2.1.3. Funktionsfähigkeit der vollständigen Signaltransduktionkaskade eines in "native" Vesikel inkorporierten Rezeptors: Induktion der Ionenkanalaktivität (Sekundärfunktion) des 5HT_{3A}-Rezeptors nach spezifischer Ligandenbindung (Primärfunktion).

Es wurde überraschend festgestellt, dass mit einem erfindungsgemässen bioanalytischen Reagens die vollständige, über einen Rezeptor vermittelte Signaltransduktion erhalten bleibt.

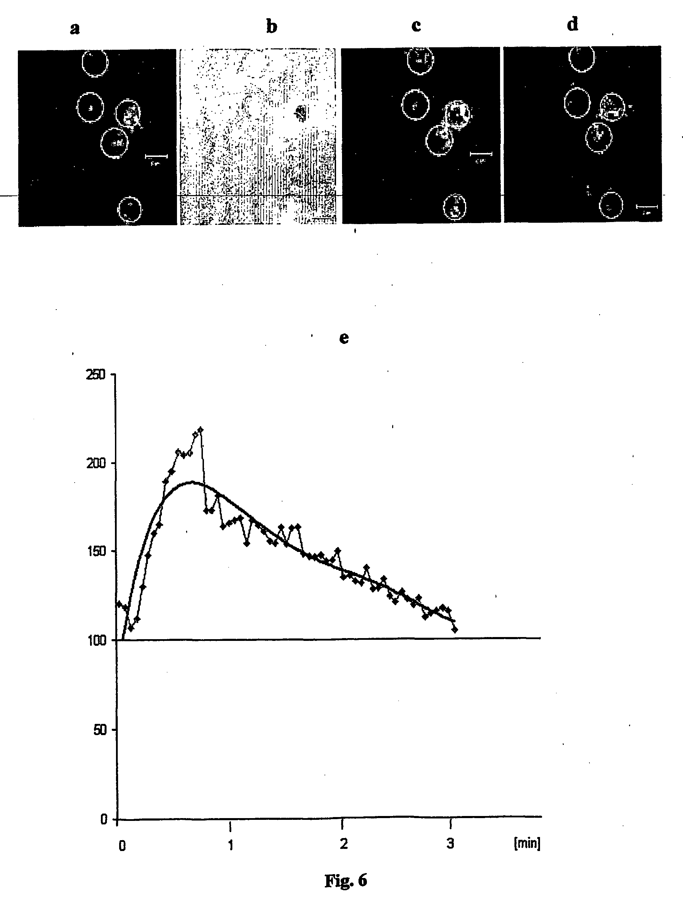

Figur 6 demonstriert die Fähigkeit der Vesikel mit inkorporiertem 5HT_{3A}-Serotoninrezeptor, die Konzentration intrazellulären Calciums zu steuern. Dabei wurden die Ursprungs-HEK-Zellen, aus denen unter Zugabe von Cytochalasin die Vesikel nach den oben beschriebenen Verfahren herausgelöst werden, mit dem Acetoxymethylester des Fluo-3 als Calciumindikator beladen (im allgemeinen 20µg Fluo-3 in 500 µl DMEM/F12 Medium). Die hergestellten Vesikel enthielten neben dem 5HT_{3A}-Rezeptor den besagten fluoreszenten Calciumindikator und Teile des Endoplasmatischen Reticulums.

Fluoreszenzanregung des Fluo-3 erfolgte bei 488 nm. Die Emission wurde bei 500 - 530 nm gemessen. Die mit Fluo-3 beladenen Vesikel wurden dann mit 350 µM m-Chlorophenylbiguanidehydrochlorid (mCPG), einem rezeptorspezifischen Agonisten, stimuliert. Die Stimulation erfolgte durch Zugabe des Agonisten in das Vesikelmedium (t = 0 sec). Bereits weniger als eine Minute nach der Zugabe des Agonisten wurde das maximale Fluoreszenzsignal, das durch Binden des Calciums an den Calciumindikator Fluo-3 entstand, gemessen. Der Wert betrug 35µM freier Calciumionen im Vesikelinnern, das heisst in dem vom Vesikel umschlossenen Zytosol. (Mit Hilfe des Calcium-Indikators Fluo-3 werden freie Calciumionen nur in dem aus der Ursprungszelle übertragenen Zytosol (Vesikellumen) nachgewiesen. Calciumionen im endoplasmatischen Reticulum oder in anderen gegebenenfalls im Vesikel mit eingeschlossenen Zellorganellen oder Teilen von Zellorganellen werden von diesem Nachweis nicht erfasst.

Das Ansteigen des Fluoreszenzsignals bis zu seinem Maximalwert nach weniger als einer Minute und die darauffolgende Abnahme des Fluoreszenzsignals zum Anfangswert nach weiteren 2 Minuten, also ingesamt 3 Minuten nach Agonistenzugabe, ist ein Anzeichen für die intakte Funktionsweise des vollständigen Mechanismus der Signaltransduktionskaskade innerhalb des Vesikels, das heisst, für die Fähigkeit des Vesikels, die Konzentration freier Calciumionen zu "puffern". Dazu gehören neben der Zeitspanne zum Schliessen des Ionenkanals des Rezeptors und serotonintypischen Desensitisierungsmechanismen das Einsetzen von Natrium-Calcium-Austauschern.

### 2.2. Inkorporation von G-Protein-gekoppelten Rezeptoren in "native" Vesikel unter Erhaltung von deren Funktionalität, am Beispiel des NK1-Rezeptors

Ein wichtiger Vertreter der membranständigen, G-Protein-gekoppelten Rezeptoren (GPCR) ist der NK1-Rezeptor. Er spielt eine wichtige immunologische Rolle in der Aktivierung von Astrozyten im zentralen Nervensystem durch Substanz P, einem Tachykinin. Tachykinine sind Neuropeptide, die sowohl im peripheren als auch zentralen Nervensystem aktiv sind, und bei Entzündungsprozessen, Schmerzempfindlichkeit und einigen autonomen Reflexen eine Rolle spielen.

Es gibt Hinweise, dass GPCR Membranriefelungsprozesse induzieren (Okamoto H., Takuwa N., Yokomizo T., Sugimoto N., Sakurada S., Shigematsu H., Takuwa Y., "Inhibitory Regulation of Rac Activation, Membrane Ruffling, and Cell Migration by the G Protein-Coupled Sphingosine-1-Phosphate Receptor EDG5 but Not EDG1 or EDG3", Mol Cell Biol 20 (2000) 9247-9261). Dieses im Vergleich zum beschriebenen 5HT3-Rezeptor unterschiedliche Verhalten könnte den Einbau in die nativen Vesikel als nachteilig Reagens beeinflussen. Im folgenden wird gezeigt, dass dies nicht der Fall ist.

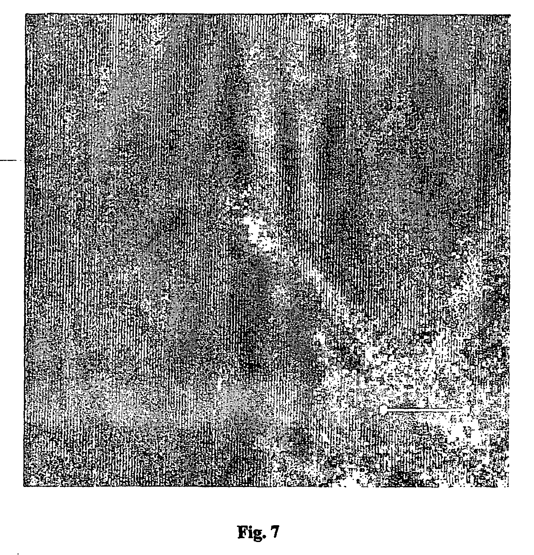

Der NK1 Rezeptor wurde auf der Ebene der kodierenden DNA am Carboxy-terminalen Ende mit der für EGFP (Clontech Palo Alto, Ca, USA) kodierenden DNA fusioniert und mittels der Calcium-Phosphat/DNA-Kopräzipitationsmethode (Jordan M., Schallhorn A., Wurm F. M., "Transfecting mammalian cells: optimization of critical parameters affecting calcium-phosphate precipitate formation"; Nucleic Acids Res 24 (1996) 596-601) in HEK293-Zellen transfiziert. 24 Stunden nach der Transfektion waren grün markierte NK1-Rezeptoren in der Plasmamembran der HEK293- Zellen nachweisbar. Dann wurde Cytochalasin B (10 µg/ml) zu den exprimierenden Zellen gegeben und die Vesikelbildung mittels konfokaler Mikroskopie verfolgt. Figur 7 demonstriert die Inkorporation von NK1-GFP-Fusionsproteinen in "native" Vesikel. Am Beispiel des NK1-Rezeptors wird somit der Nachweis geführt, dass G-Protein gekoppelte Rezeptoren in native Vesikel eingebaut werden.

### 3. Lagerungsfähigkeit von nativen Vesikeln

Frisch präparierte "native" Vesikel konnten in Phosphatpuffer (PBS: 150 mM Natriumphosphat, 150 mM NaCl, pH 7.2± 0.1 (25°C) oder DMEM/F12-Medium unter sterilen Bedingungen bei 4°C ohne Qualitätsveränderung bis zu einer Woche gelagert werden.

Zum Einfrieren wurden "native" Vesikel in DMEM-Medium mit 2.2 % Serum (Fetal Bovine Serum) und 10 % Dimethylsulfoxid (DMSO) resuspendiert. Diese Vesikelsuspension wurde in flüssigem Stickstoff schockgefroren. Gefrorene Vesikel konnten bis zu 6 Monate bei - 80°C ohne Qualitätsverlust gelagert werden. Verglichen mit einer frisch präparierten Vesikel-Fraktion zeigten gefrorene "native" Vesikel nach dem Auftauen keine signifikanten Veränderungen in Grösse oder Morphologie. Sie zeigten auch keine verstärkte Tendenz zu Aggregationsbildungen.

### Bestimmungen zur Stabilität von gefrorenen Vesikeln:

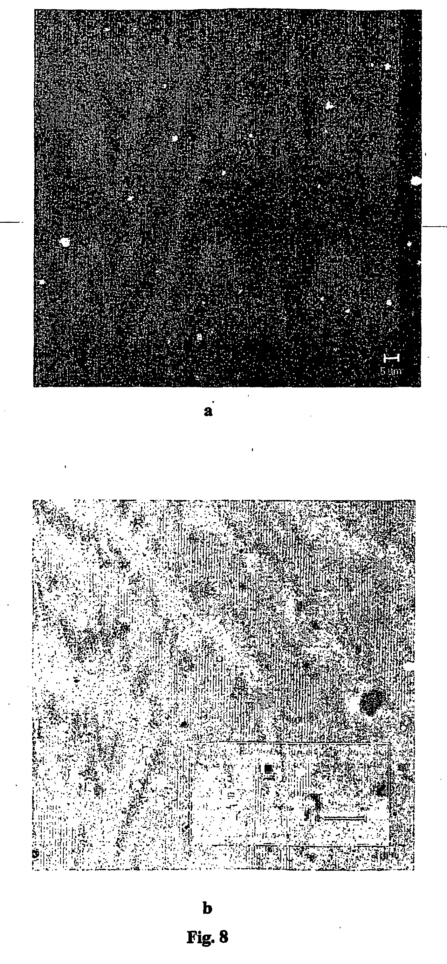

Vesikel wurden von HEK293 Zellen hergestellt, die in ihrem Cytoplasma das GFP von *Aquorea victoria* exprimierten. Ein frei im Cytoplasma gelöstes, fluoreszierendes Protein sollte als Nachweis dafür dienen, dass die Vesikel den Einfrier- und Auftauzyklus unbeschadet überstehen und nicht platzen bzw. auslaufen. Abbildung 8 zeigt als Beispiel Mikroskop-aufnahmen von Vesikeln nach 30-tägiger Lagerung bei -80°C. Eine Beschädigung oder Veränderung der Morphologie der Vesikel wurde nicht beobachtet.

### Beschreibung der Figuren:

**Figur 1****:** Prozess der Abschnürung von Vesikeln mit Übertragung von Cytoplasma von der Zelle in die Vesikel: Mit einem konfokalen Fluoreszenzmikroskop erzeugte Aufnahmen von HEK293-Zellen, welche nach Transfektion mit *Aequorea* GFP eine grüne Fluoreszenz im Cytoplasma aufweisen (Anregung 488 nm / Emission 510 nm) ; darunter graphische Illustrationen des Prozesses. **(a)** Normales Aktin-Filamentnetz im Zellkortex vor der Zugabe von Cytochalasin. **(b)** Nach der Applikation von Cytochalasin B beginnen die Zellen sich abzurunden und bilden **(c)** blasen- oder füsschenartige Ausstülpungen.
**Figur 2****:** Konfokale Aufnahme (Anregung: 488 nm; Emission: 510 nm), welche die Inkorporierung von Teilen des endoplasmatischen Reticulums in "native" Vesikel illustriert (**(a)** Bild bei der Fluoreszenzwellenlänge; **(b)** Überlagerung des Bildes bei der Fluoreszenz-wellenlänge mit dem Transmissionsbild). Das endoplasmatische Reticulum wurde durch die Expression von rekombinantem EGFP (Enhanced GFP ; Clontech, Palo Alto, CA, USA) in HEK293-Zellen sichtbar gemacht. Hierbei wurde das EGFP mit einer Signalsequenz versehen, die das fluoreszierende Protein während seiner Expression in das Lumen des endoplasmatischen Retikulums leitet und damit "anfärbt".- Der Pfeil markiert ein Vesikel, das nach Zugabe von Cytochalsin B [10 µg/ml von einer lebenden HEK293-Zelle angeschnürt wurde. Der Grössenbalken entspricht einer Länge von 5 µm.
**Figur 3****:** Konfokale Abbildung einer sich teilenden und zur Vesikelbildung behandelten HEK-Zelle, die heterolog GFP-gelabeltes G_{α}-Protein exprimiert. Die Zelle war zuvor mit dem Calcium-Indikatorfarbstoff Fura-Rot behandelt worden. Im Uhrzeigersinn, links oben beginnend, sind zu sehen: **(a)** Fura-Rot-Signal, **(b)** GFP-G_{α}-Signal, **(c)** Transmissionsbild, **(d)** Überlagerung der Aufnahmen **(a)** und **(c).** Das Fura-Rot-Signal (Bild **(a))** wurde mit einem Hochpassfilter (Kantenfilter mit Transmission für λ > 650 nm) aufgenommen. Bild **(b)** zeigt die Fluoreszenzemission des GFP zwischen 500 und 530 nm. Das Transmissionsbild entspricht einer NORMARSKI Abbildung. Für alle gezeigten Aufnahmen erfolgte die Fluoreszenzanregung bei 488 nm. - Mit Kreisen markiert sind "native", sich abschnürende wie auch bereits abgekoppelte Vesikel. Man beachte, dass die Vesikel sowohl das rekombinant hergestellte Produkt (G_{α}-Protein) als auch den Calciumindikator mit sich führen.
**Figur 4****: (a)** Fluoreszenz eines aus einer lebenden HEK293-Zelle erzeugten Vesikels mit aus der Ursprungszelle übertragenem, GFP-markiertem cytosolischem Zellinhalt (grüne Fluoreszenz im Vesikelinnern) und in der Vesikelmembran inkorporiertem 5HT3-Rezeptor, markiert mit GR-Cy5 (rote Fluoreszenz aus dem äusseren Bereich des Vesikels). **(b)** Linienprofile der grünen und roten Fluoreszenz.
**Figur 5****:** Bindungskurve eines fluoreszenten Liganden an einzelne diskrete Vesikel (n = 3), bestimmt durch sequentielle Zugabe ansteigender Konzentrationen des fluoreszenten Liganden auf ein- und dasselbe Deckglas mit darauf durch Übemacht-Inkubation immobilisierten Vesikeln:
   Fluoreszenzintensitäten F als Funktion ansteigender Konzentrationen von GR-Rho, als fluoreszentem Liganden des in "native" Vesikel inkorporierten 5HT₃-Rezeptors aus HEK293-Zellen dargestellt (nach Subtraktion der beobachteten Fluoreszenzintensität F₀ in Abwesenheit von GR-Rho). Aus dem Fit der Messdaten mit der Gleichung einer Langmuir-Bindungsisotherme ergibt sich ein Wert von K_{d} = (4.2 +/- 1.7) nM für die Dissoziationskonstante des Systems bestehend aus dem HT₃-Rezeptor und dem GR-Rho-Liganden.
**Figur 6****:** Demonstration der Fähigkeit des in Vesikel inkorporierten 5-HT₃-Serotoninrezeptors zur Regulation der Konzentration intravesikulärer Calciumionen, visualisiert anhand der Fluoreszenz des Calciumindikators Fluo-3 (Anregung: 488 nm; Emission: 500 - 530 nm). Stimulation der mit Fluo-3 beladenen Vesikel durch Zugabe von 350 µM m-Chlorophenylbiguanidhydrochlorid (mCPG), als einem rezeptorspezifischen Agonisten.
   Konfokale Fehlfarbenaufnahmen vor, t=0 **(a, b),** 45 sec **(c)** und 3 min **(d)** nach Zugabe des Serotoninagonisten aufgenommen. Die Momentaufnahme **(b)** stellt die analysierten Bildausschnitte (Kreise) auf Hintergrund des Transmissionsbildes, überlagert mit der nicht mit Fehlfarben kodierten Fluoreszenz des Calciumindikators, dar. Man beachte, dass die Fluoreszenzsignale aufgrund der hohen Verstärkung eine grössere Fläche als die Vesikel einnehmen.
   **(e)** Zeitlicher Verlauf des Fluorezenzsignals eines Vesikels, das die stärkste Ca-Antwort zeigte. Die Stimulation erfolgte durch Zugabe des Agonisten (mCPG) in das Vesikelmedium (t = 0 sec). Bereits nach weniger als einer Minute wurde das maximale Fluoreszenzsignal, das durch Binden des Calciums an den Calciumindikator Fluo-3 entstand, gemessen.
**Figur 7****:** Ein sich von einer HEK293 Zelle loslösendes "natives" Vesikel, welche Zelle den mit GFP markierten NK1 Rezeptor exprimiert. Hierbei wurde auf der Ebene der kodierenden DNA das NK1 Rezeptorprotein mit dem Aequorea victoria GFP fusioniert.
**Figur 8****:** Konfokale Aufnahmen, die die Qualität von "nativen" Vesikeln nach 30-tägiger Lagerung bei -80°C und anschliessendem Auftauen illustrieren. "Native" Vesikel wurden durch Cytochalasin B-Behandlung von HEK293 Zellen hergestellt, die transient transfiziertes, rekombinantes EGFP (Clontech; Palo Alto, Ca, USA) im Zytosol exprimieren. **(a)** Fluoreszenzaufnahme (Anregung 488 nm; Emission 510 nm); **(b)** Kombination von Transmissionsbild und Fluoreszenzaufnahme. Der Bildausschnitt unten rechts zeigt eine stärkere Vergrösserung von intakten "nativen" Vesikeln.

## Patentansprüche

1. Verfahren zur Herstellung eines bioanalytischen Reagens mit einem aus einer lebenden Zelle erzeugten Plasmamembran-Vesikel umfassend mindestens einen Rezeptor und neben dem mindestens einen Rezeptor weitere Zellprodukte bzw. Zellproteine, welche an einem von diesem Rezeptor in der verwendeten lebenden Zelle ausgelösten Mechanismus einer Signaltransduktion beteiligt sind, wobei es bei dem Vesikulierungsprozess zu keiner Vermischung des Zell- und Vesikelinhalts mit dem umgebenden äußeren Zellmedium kommt und ein von besagtem Rezeptor in besagter lebender Zelle ausgelöster Mechanismus einer Signaltransduktion in besagtem Plasmamembran-Vesikel als Bestandteil des bioanalytischen Reagens erhalten bleibt, **dadurch gekennzeichnet, dass** die Abschnürung besagten Plasmamembran-Vesikels von besagter lebender Zelle nach Zugabe von Cytochalasin B und/oder Cytochalasin D zu vorgewärmtem DMEM-Medium erfolgt und weiterhin umfassend die Anwendung von Scherkräften vorzugsweise mittels Vortexen.

2. Verfahren nach Anspruch 1, **dadurch gekennzeichnet, dass** dieses die Anwendung von Zentrifugationsschritten und / oder Chromatographieschritten und / oder von Filtrationsschritten und / oder von elektrophoretischen Methoden umfasst.

3. Verfahren nach einem der Ansprüche 1 oder 2, **dadurch kennzeichnet, dass** das Innere eines nach diesem Verfahren hergestellten Plasmamembran-Vesikels frei von Zellkernmaterial ist, so dass keine replikativen Prozesse auftreten.

4. Verfahren nach einem der Ansprüche 1-3, **dadurch gekennzeichnet, dass** eine in der Plasmamembran-Vesikel-erzeugenden lebenden Zelle vorhandene Bindungsfähigkeit des besagten mindestens einen Rezeptors, welcher mit dem Plasmamembran-Vesikel als Teil des bioanalytischen Reagens assoziiert ist, zu einem spezifischen Liganden, erhalten bleibt.

5. Verfahren nach einem der Ansprüche 1-4, **dadurch gekennzeichnet, dass** der mindestens eine Rezeptor ausgewählt ist aus der Gruppe von signalübertragenden Rezeptoren, die von Plasmamembranrezeptoren und intrazellulären Hormon-Rezeptoren gebildet wird.

6. Verfahren nach einem der Ansprüche 1-5, **dadurch gekennzeichnet, dass** besagtes mindestens eine nach diesem Verfahren hergestellte Plasmamembran-Vesikel neben besagtem mindestens einem Rezeptor weitere biologische Komponenten aus der Gruppe umfasst, die von G-Proteinen und G-Protein-Regulatoren, Enzymen, welche intrazelluläre sekundäre Botenstoffe bilden, und Enzymen, welche Proteine durch Phosphorylierung oder Dephosphorylierung aktivieren bzw. hemmen, gebildet wird.

7. Verfahren nach einem der Ansprüche 1-6, **dadurch gekennzeichnet, dass** mit der äusseren Membran des mindestens einen nach diesem Verfahren hergestellten Plasmamembran-Vesikels biologische, biochemische oder synthetische Komponenten assoziiert sind, welche dem Transport besagten Vesikels zu Zellen und / oder Organen und / oder vorbestimmtem Gewebe in einem lebenden Organismus als vorbestimmten Zielorten und / oder der Bindung an ein biologisches oder biochemisches oder synthetisches Erkennungselement, welches besagte biologische oder biochemische oder synthetische Komponenten spezifisch erkennt und bindet, dienen.

8. Verfahren nach einem der Ansprüche 1-7, **dadurch gekennzeichnet, dass** besagtes mindestens eine nach diesem Verfahren hergestellte Plasmamembran-Vesikel zusätzlich Komponenten zur Erzeugung eines experimentell detektierbaren Signals umfasst.

9. Verfahren zur Herstellung eines bioanalytischen Reagens mit einem aus einer lebenden Zelle erzeugten Plasmamembran-Vesikel nach einem der Ansprüche 1-8, **dadurch gekennzeichnet, dass** besagtes Plasmamembran-Vesikel mit einem künstlichen Lipidvesikel zu einem Mischvesikel fusioniert wird.

10. Verfahren nach Anspruch 9, **dadurch gekennzeichnet, dass** besagtes Mischvesikel im Vergleich zu dem aus einer lebenden Zelle erzeugten Plasmamembran-Vesikel wesentlich vergrößert wird.

11. Verfahren nach einem der Ansprüche 9-10, **dadurch gekennzeichnet, dass** besagtes Mischvesikel im Vergleich zu dem aus einer lebenden Zelle erzeugten Plasmamembran-Vesikel zusätzliche natürliche und / oder synthetische Lipide und / oder auch zusätzliche Proteine mit zusätzlichen Funktionalitäten enthält.

12. Bioanalytisches In-vitro-Nachweisverfahren mit einem bioanalytischen Reagens hergestellt nach einem der Ansprüche 1 bis 11 mit mindestens einem Plasmamembran-Vesikel, das aus einer lebenden Zelle erzeugt wird, umfassend mindestens einen Rezeptor, wobei besagtes mindestens eine Plasmamembran-Vesikel neben dem mindestens einen Rezeptor weitere Zellprodukte bzw. Zellproteine umfasst, welche an einem von diesem Rezeptor in der verwendeten lebenden Zelle ausgelösten Mechanismus einer Signaltransduktion beteiligt sind, wobei besagte Plasmamembran-Vesikel keine Beimengungen infolge von Mischungen mit dem umgebenden äußeren Zellmedium während des Vesikulierungsprozesses aufweisen und ein von besagtem Rezeptor ausgelöster Mechanismus einer Signaltransduktion in besagtem Plasmamembran-Vesikel als Bestandteil des bioanalytischen Reagens erhalten bleibt, zur gleichzeitigen oder sequentiellen, quantitativen oder / und qualitativen Bestimmung eines oder mehrerer Analyten aus der Gruppe von Rezeptoren oder Liganden, Chelatoren oder "Histidin-tag-Komponenten", Enzymen, Enzymcofaktoren oder Inhibitoren, wobei besagtes Nachweisverfahren ausgewählt ist aus der Gruppe, die beispielsweise von optischen Nachweisverfahren, Detektion von Energie- oder Ladungstransfer, Massenspektroskopie, elektrischen oder elektrochemischen Nachweisverfahren, elektronischen Resonanzmessungen, radioaktiven Verfahren oder elektrophoretischen Messungen gebildet wird.

13. Bioanalytisches Nachweisverfahren nach Anspruch 12, **dadurch gekennzeichnet, dass** dieses in homogener Lösung ausgeführt wird.

14. Bioanalytisches Nachweisverfahren nach Anspruch 12, **dadurch gekennzeichnet, dass** dieses Nachweisverfahren mit Hilfe einer Messanordnung mit mindestens 2 Elektroden und separierten, für die Aufnahme von Flüssigkeiten geeigneten Kompartimenten durchgeführt wird, wobei sich zwischen zwei, einander gegenüber befindlichen, in je mindestens ein Kompartiment hineinreichenden oder ein solches berührenden und beliebig geformten Elektroden ein fester Träger befindet, der mindestens eine Apertur enthält und jeweils mindestens 2 Kompartimente voneinander trennt.

15. Bioanalytisches Nachweisverfahren nach Anspruch 14, **dadurch gekennzeichnet, dass** besagte Messanordnung auf einer Seite oder auf beiden Seiten des Trägers Mittel aufweist, die eine Flüssigkeitszugabe und / oder eine Flüssigkeitsspeicherung und / oder einen Flüssigkeitsaustausch und / oder die Zugabe vom Plasmamembran-Vesikel zwischen Träger und Elektrode ermöglichen.

16. Bioanalytisches Nachweisverfahren nach einem der Ansprüche 14-15, **dadurch gekennzeichnet, dass** die mindestens eine Apertur besagter Messanordnung einen solchen Durchmesser aufweist, dass sich bei Existenz einer Spannungsdifferenz über der Messanordnung, vermittelt durch die mindestens zwei Elektroden, ein inhomogenes elektrisches Feld um die Apertur aufbaut, das mit Annäherung an die Apertur betragsmäßig größer wird und nahe der Apertur das Plasmamembran-Vesikel elektrophoretisch auf diese zubewegen kann.

17. Bioanalytisches Nachweisverfahren nach einem der Ansprüche 14-15, **dadurch gekennzeichnet, dass** die mindestens eine Apertur besagter Messanordnung einen solchen Durchmesser aufweist, dass sich das Plasmamembran-Vesikel durch einen hydrodynamischen oder elektrokinetischen Fluss oder durch andere mechanische Manipulation über oder in der Apertur positionieren lassen.

18. Bioanalytisches Nachweisverfahren nach einem der Ansprüche 14-17, **dadurch gekennzeichnet, dass** der Träger besagter Messanordnung eine elektrisch geladene Oberfläche aufweist, die attraktiv für das Plasmamembran-Vesikel ist, oder dass auf seiner Oberfläche eine Haftvermittlungsschicht zur Bindung besagter Plasmamembran-Vesikel aufgebracht ist.

19. Bioanalytisches Nachweisverfahren nach einem der Ansprüche 14-18, **dadurch gekennzeichnet, dass** die Plasmamembran-Vesikel in ein vorher mit Puffer gefülltes oder ungefülltes Compartiment zwischen Trennwand bzw. Träger und Elektrode gegeben werden und durch eine an die Elektroden angelegte elektrische Spannungsdifferenz auf die Aperturöffnung bewegt werden oder / und durch hydrodynamischen oder elektrokinetischen Fluss oder / und mechanisch auf die Aperturöffnung positioniert werden.

20. Bioanalytisches Nachweisverfahren nach einem der Ansprüche 14-19, **dadurch gekennzeichnet, dass** die Plasmamembran-Vesikel auf besagte Aperturöffnung positioniert werden, die Vesikelmembranen mit der Oberfläche des Trägers eine elektrisch dichte Verbindung über der Aperturöffnung eingehen und eine Aufzeichnung des Membranwiderstandes ermöglichen.

21. Bioanalytisches Nachweisverfahren nach einem der Ansprüche 14-20, **dadurch gekennzeichnet, dass** zu mindestens einem Kompartiment künstliche Lipidvesikel, mit einem größeren Durchmesser als dem Durchmesser besagter Apertur, gegeben werden, mit dem Ziel, damit auf der Oberfläche des Trägers eine Apertur-überspannende, planare Lipiddoppelschicht zu erzeugen, und dass anschließend die Plasmamembran-Vesikel zu besagtem Kompartiment hinzugegeben werden mit dem Ziel, diese mit der erzeugten planaren Lipidmembran zu fusionieren und damit Rezeptoren, welche mit diesen aus lebenden Zellen erzeugten Plasmamembran-Vesikeln assoziiert sind, elektrischen oder optischen Messungen zugänglich zu machen.

22. Bioanalytisches Nachweisverfahren nach einem der Ansprüche 14-21, **dadurch gekennzeichnet, dass** Membranproteine nach der Positionierung eines der Plasmamembran-Vesikels über einer Apertur in dieses eingebaut werden.

23. Bioanalytisches Nachweisverfahren nach einem der Ansprüche 14-22, **dadurch gekennzeichnet, dass** das über einer Apertur befindliche Plasmamembran-Vesikel, oder eine aus diesem Plasmamembran-Vesikel erzeugte Apertur-überspannende planare Membran optischen Messungen, insbesondere Fluoreszenzmessungen, oder simultanen optischen und elektrischen Messungen zugänglich ist und diese daran ausgeführt werden.

24. Bioanalytisches Nachweisverfahren nach einem der Ansprüche 14-23, **dadurch gekennzeichnet, dass** eine Messanordnung oder ein Messsystem mit mehreren Aperturen auf einem Träger verwendet wird, und dass Messungen über mindestens zwei Aperturen sequentiell und/oder parallel erfolgen.

25. Bioanalytisches Nachweisverfahren nach einem der Ansprüche 14-23, **dadurch gekennzeichnet, dass** eine Vielzahl von der aus lebenden Zellen erzeugte Plasmamembran-Vesikel in einem Array auf einen festen, elektrisch isolierenden Träger angeordnet sind, wobei dieses Array von Plasmamembran-Vesikeln mit einem Array von Patch-Clamp-Pipetten gleicher geometrischer Anordnung wie das Plasmamembran-Vesikel-Array in elektrisch isolierenden Kontakt gebracht wird, um eine gleichzeitige Durchführung von voneinander unabhängigen elektrischen Messungen, oder simultanen elektrischen und optischen Messungen, an einer Vielzahl einzelner Plasmamembran-Vesikel zu ermöglichen.

26. Bioanalytisches Nachweisverfahren nach Anspruch 12, **dadurch gekennzeichnet, dass** mindestens ein der Plasmamembran-Vesikel auf der Oberfläche eines festen Trägers immobilisiert wird.

27. Bioanalytisches Nachweisverfahren nach Anspruch 26 **dadurch gekennzeichnet, dass** ein von besagtem Rezeptor in besagter lebender Zelle ausgelöster Mechanismus einer Signaltransduktion in besagtem Plasmamembran-Vesikel nach der Immobilisierung erhalten bleibt.

28. Bioanalytisches Nachweisverfahren nach Anspruch 26, **dadurch gekennzeichnet, dass** das Plasmamembran-Vesikel in diskreten Messbereichen (d), mit jeweils ein oder mehr Vesikeln, auf der Oberfläche besagten festen Trägers immobilisiert sind.

29. Bioanalytisches Nachweisverfahren nach Anspruch 28, **dadurch gekennzeichnet, dass** in einer Vielzahl von Messbereichen (d) Plasmamembran-Vesikel mit wenigstens zwei unterschiedlichen Arten von Rezeptoren immobilisiert sind, wobei innerhalb eines einzelnen Messbereichs vorzugsweise jeweils Vesikel mit einer einheitlichen Art von Rezeptoren immobilisiert sind.

30. Bioanalytisches Nachweisverfahren nach einem der Ansprüche 26-29, **dadurch gekennzeichnet, dass** die Immobilisierung des Plasmamembran-Vesikels auf der Oberfläche besagten festen Trägers mittels kovalenter Bindung oder mittels physikalischer Adsorption erfolgt.

31. Bioanalytisches Nachweisverfahren nach einem der Ansprüche 26-30, **dadurch gekennzeichnet, dass** zwischen der Oberfläche besagten festen Trägers und dem mindestens einen darauf immobilisierten Plasmamembran-Vesikel eine Haftvermittlungsschicht aufgebracht ist.

32. Bioanalytisches Nachweisverfahren nach Anspruch 31, **dadurch gekennzeichnet, dass** die Haftvermittlungsschicht eine chemische Verbindung aus der Gruppe Silane, Epoxide, funktionalisierte, geladene oder polare Polymere und "selbstorganisierte funktionalisierte Mono- oder Mehrfachschichten" umfasst.

33. Bioanalytisches Nachweisverfahren nach Anspruch 31, **dadurch gekennzeichnet, dass** die Haftvermittlungsschicht eine monomolekulare Schicht aus vorwiegend einer Sorte von Proteinen oder modifizierten Proteinen umfasst.

34. Bioanalytisches Nachweisverfahren nach Anspruch 33, **dadurch gekennzeichnet, dass** die Haftvermittlungsschicht selbstorganisierte, Alkan-endständige Monoschichten aus vorwiegend einer Sorte von chemischen oder biochemischen Molekülen umfasst.

35. Bioanalytisches Nachweisverfahren nach einem der Ansprüche 31-34, **dadurch gekennzeichnet, dass** mit der äusseren Membran des mindestens einen Plasmamembran-Vesikels biologische, biochemische oder synthetische Komponenten assoziiert sind, welche dem Transport besagten Plasmamembran-Vesikels zu Zellen und / oder Organen und / oder vorbestimmtem Gewebe in einem lebenden Organismus als vorbestimmten Zielorten und / oder der Bindung an ein biologisches oder biochemisches oder synthetisches Erkennungselement, welches besagte biologische oder biochemische oder synthetische Komponenten spezifisch erkennt und bindet, dienen, und diese biologischen oder biochemischen oder synthetischen Komponenten zur spezifischen Erkennung und Bindung mit der Haftvermittlungsschicht biologische oder biochemische oder synthetische Erkennungselemente assoziiert sind, welche das Plasmamembran-Vesikel erkennen und binden.

36. Bioanalytisches Nachweisverfahren nach Anspruch 12, **dadurch gekennzeichnet, dass** mindestens ein Ligand für einen Rezeptor, welcher an ein Plasmamembran-Vesikel gebunden ist, auf der Oberfläche eines festen Trägers, gegebenenfalls über ein Spacer-Molekül, immobilisiert ist.

37. Bioanalytisches Nachweisverfahren nach Anspruch 36, **dadurch gekennzeichnet, dass** in einer Vielzahl von Messbereichen (d) wenigstens zwei unterschiedliche Liganden für Rezeptoren, welche an ein Plasmamembran-Vesikel, gebunden sind, wobei innerhalb eines einzelnen Messbereichs vorzugsweise jeweils eine einheitliche Art von Liganden immobilisiert ist.

38. Bioanalytisches Nachweisverfahren nach einem der Ansprüche 36-37, **dadurch gekennzeichnet, dass** besagte Liganden auf der Oberfläche des festen Trägers mittels kovalenter Bindung oder mittels physikalischer Adsorption immobilisiert sind.

39. Bioanalytisches Nachweisverfahren nach einem der Ansprüche 36-37, **dadurch gekennzeichnet, dass** zwischen der Oberfläche des festen Trägers und besagten darauf immobilisierten Liganden eine Haftvermittlungsschicht aufgebracht ist.

40. Bioanalytisches Nachweisverfahren nach einem der Ansprüche 28-39, **dadurch gekennzeichnet, dass** Bereiche zwischen den räumlich getrennten Messbereichen, mit den darin immobilisierten Plasmamembran-Vesikeln oder mit darin immobilisierten Liganden für Rezeptoren, welche an das Plasmamembran-Vesikel gebunden sind, oder / und dass Bereiche innerhalb dieser Messbereiche, zwischen den genannten darin immobilisierten Verbindungen, zur Minimierung unspezifischer Bindung von Analyten oder deren Nachweissubstanzen "passiviert werden", d.h. dass zwischen den räumlich getrennten Messbereichen (d) oder / und dass innerhalb dieser Messbereiche (d) zwischen den genannten darin immobilisierten Verbindungen gegenüber den Analyten "chemisch neutrale" Verbindungen aufgebracht sind, vorzugsweise bestehend aus den Gruppen, die von Albuminen, Casein, Detergentien, Detergentien-Lipid-Mischungen, synthetischen und natürlichen Lipiden oder auch hydrophilen Polymeren gebildet werden.

41. Bioanalytisches Nachweisverfahren nach einem der Ansprüche 14-40, **dadurch gekennzeichnet, dass** das Material der Oberfläche besagten festen Trägers mit den immobilisierten Plasmamembran-Vesikeln oder mit den darauf immobilisierten Liganden für Rezeptoren, welche an das Plasmamembran-Vesikel gebunden sind, ein Material aus der Gruppe umfasst, die von form-, spritz- oder fräsbaren Kunststoffen, Kohlenstoffverbindungen, Metallen, -Metalloxiden oder Silikaten oder Silizium oder Germanium oder ZnSe oder einer Mischung dieser Materialien gebildet wird.

42. Bioanalytisches Nachweisverfahren nach einem der Ansprüche 14-41, **dadurch gekennzeichnet, dass** die Oberfläche besagten festen Trägers im Wesentlichen planar ist.

43. Bioanalytisches Nachweisverfahren nach einem der Ansprüche 14-42, **dadurch gekennzeichnet, dass** es sich bei besagtem festen Träger um eine optische oder elektronische Sensorplattform handelt.

44. Bioanalytisches Nachweisverfahren nach einem der Ansprüche 14-42, **dadurch gekennzeichnet, dass** besagter fester Träger mindestens in einem Wellenlängenbereich im ultravioletten bis infraroten Spektrum transparent ist und er vorzugsweise ein Material aus der Gruppe umfasst, die von form-, spritz- oder fräsbaren Kunststoffen, Kohlenstoffverbindungen, Metallen, Metalloxiden oder Silikaten, oder einer Mischung dieser Materialien gebildet wird.

45. Bioanalytisches Nachweisverfahren nach Anspruch 43, **dadurch gekennzeichnet, dass** es sich bei besagtem festen Träger um einen als Sensorplattform dienenden optischen Wellenleiter handelt.

46. Bioanalytisches Nachweisverfahren nach Anspruch 43, **dadurch gekennzeichnet, dass** es sich bei besagtem festen Träger um einen als Sensorplattform dienenden optischen Dünnschichtwellenleiter handelt, mit einer ersten optisch transparenten Schicht (a) mit Brechungsindex n₁ auf einer zweiten optisch transparenten Schicht (b) mit Brechungsindex n₂, wobei n₁ > n₂ ist.

47. Bioanalytisches Nachweisverfahren nach einem der Ansprüche 45-46, **dadurch gekennzeichnet, dass** die Sensorplattform als fester Träger aufgeteilt ist in zwei oder mehr diskrete wellenleitende Bereiche.

48. Bioanalytisches Nachweisverfahren nach einem der Ansprüche 46-47, **dadurch gekennzeichnet, dass** das Material der zweiten optisch transparenten Schicht (b) der Sensorplattform als festen Träger ein Material aus der Gruppe umfasst, welche von Silikaten oder transparenten form-, spritz- oder fräsbaren, vorzugsweise thermoplastischen Kunststoffen gebildet wird.

49. Bioanalytisches Nachweisverfahren nach einem der Ansprüche 46-48, **dadurch gekennzeichnet, dass** der Brechungsindex der ersten optisch transparenten Schicht (a) der Sensorplattform als festem Träger größer als 1.8 ist.

50. Bioanalytisches Nachweisverfahren nach einem der Ansprüche 46-49, **dadurch gekennzeichnet, dass** die erste optisch transparente Schicht (a) der Sensorplattform als festem Träger TiO₂, ZnO, Nb₂O₅, Ta₂O₅, HfO₂, oder ZrO₂, bevorzugt TiO₂, Ta₂O₅ oder Nb₂O₅ umfasst.

51. Bioanalytisches Nachweisverfahren nach einem der Ansprüche 46-50, **dadurch gekennzeichnet, dass** sich zwischen den optisch transparenten Schichten (a) und (b) und in Kontakt mit Schicht (a) eine weitere optisch transparente Schicht (b') mit niedrigerem Brechungsindex als dem der Schicht (a) und einer Stärke von 5 nm - 10000 nm, vorzugsweise von 10 nm - 1000 nm, befindet.

52. Bioanalytisches Nachweisverfahren nach einem der Ansprüche 46-51, **dadurch gekennzeichnet, dass** die Einkopplung von Anregungslicht in die optisch transparente Schicht (a) zu den Messbereichen (d) auf der Sensorplattform als Träger über ein oder mehrere optische Einkoppelelemente aus der Gruppe erfolgt, die von Prismenkopplern, evaneszenten Kopplern mit zusammengebrachten optischen Wellenleitern mit überlappenden evaneszenten Feldern, Stirnflächenkopplern mit vor einer Stirnseite der wellenleitenden Schicht angeordneten fokussierenden Linsen, vorzugsweise Zylinderlinsen, und Gitterkopplern gebildet wird.

53. Bioanalytisches Nachweisverfahren nach einem der Ansprüche 46-52, **dadurch gekennzeichnet, dass** die Einkopplung des Anregungslichts zu den Messbereichen (d) mit Hilfe von einer oder mehreren Gitterstrukturen (c) erfolgt, die in der optisch transparenten Schicht (a) ausgeprägt sind.

54. Bioanalytisches Nachweisverfahren nach einem der Ansprüche 14-53, **dadurch gekennzeichnet, dass** eine oder mehrere flüssige Proben, umfassend das Plasmamembran-Vesikel mit den damit assoziierten Rezeptoren, mit den in einem oder mehreren Messbereichen immobilisierten Liganden für diese Rezeptoren in Kontakt gebracht werden und eine Signaländerung als Folge einer Bindung der mit besagten Plasmamembran-Vesikeln assoziierten Rezeptoren an ihre immobilisierten Liganden gemessen wird.

55. Bioanalytisches Nachweisverfahren nach Anspruch 54, **dadurch gekennzeichnet, dass** die Signaltransduktion von Rezeptoren, welche mit den Plasmamembran-Vesikeln assoziiert sind, nach Bindung dieser Rezeptoren an ihre immobilisierten Liganden gemessen wird.

56. Bioanalytisches Nachweisverfahren nach Anspruch 54, **dadurch gekennzeichnet, dass** die Bindung von Rezeptoren, welche mit den Plasmamembran-Vesikeln assoziiert sind, an besagte immobilisierte Liganden in Kompetition zur Bindung dieser mit besagten Plasmamembran-Vesikeln assoziierten Rezeptoren an in Lösung befindliche Liganden stattfindet.

57. Bioanalytisches Nachweisverfahren nach einem der Ansprüche 14-53, **dadurch gekennzeichnet, dass** eine oder mehrere flüssige Proben mit den in einem oder mehreren Messbereichen immobilisierten Plasmamembran-Vesikeln mit den damit assoziierten Rezeptoren, in Kontakt gebracht werden und eine Signaländerung als Folge der Bindung von Liganden oder einer anderen induzierenden Einwirkung auf besagte Rezeptoren gemessen wird.

58. Bioanalytisches Nachweisverfahren nach einem der Ansprüche 14-53, **dadurch gekennzeichnet, dass** eine oder mehrere flüssige Proben mit den in einem oder mehreren Messbereichen immobilisierten Plasmamembran-Vesikeln mit den damit assoziierten Rezeptoren, in Kontakt gebracht werden und die Signaltransduktion besagter Rezeptoren als Folge der Bindung von Liganden oder einer anderen induzierenden Einwirkung auf besagte Rezeptoren gemessen wird.

59. Bioanalytisches Nachweisverfahren nach einem der Ansprüche 57-58, **dadurch gekennzeichnet, dass** die Bindung von Liganden aus einer zugegebenen flüssigen Probe an Rezeptoren, welche mit den immobilisierten Plasmamembran-Vesikeln assoziiert sind, in Kompetition zur Bindung dieser Liganden an in Lösung befindliche, gegebenenfalls mit Vesikeln assoziierte Rezeptoren stattfindet.

60. Bioanalytisches Nachweisverfahren nach einem der Ansprüche 54-56, **dadurch gekennzeichnet, dass** eine oder mehrere flüssige Proben, umfassend die Plasmamembran-Vesikel mit den damit assoziierten Rezeptoren, mit den in einem oder mehreren Messbereichen immobilisierten Liganden für diese Rezeptoren in Kontakt gebracht werden, Anregungslicht von einer oder mehreren Lichtquellen gleicher oder unterschiedlicher Wellenlänge über eine oder mehrere Gitterstrukturen (c) zu den Messbereichen (d) eingekoppelt wird und die Änderung von ein oder mehr von den Messbereichen (d) ausgehenden optischen Signalen, als Folge einer Bindung der mit besagten Plasmamembran-Vesikeln assoziierten Rezeptoren an ihre immobilisierten Liganden, gemessen wird.

61. Bioanalytisches Nachweisverfahren nach einem der Ansprüche 57-59, **dadurch gekennzeichnet, dass** eine oder mehrere flüssige Proben mit den in einem oder mehreren Messbereichen (d) immobilisierten Plasmamembran-Vesikeln mit den damit assoziierten Rezeptoren in Kontakt gebracht werden, Anregungslicht von einer oder mehreren Lichtquellen gleicher oder unterschiedlicher Wellenlänge über eine oder mehrere Gitterstrukturen (c) zu den Messbereichen (d) eingekoppelt wird und die Änderung von ein oder mehr von den Messbereichen (d) ausgehenden optischen Signalen, als Folge der Bindung von Liganden an besagte Rezeptoren oder von anderen induzierenden Einwirkungen auf besagte Rezeptoren, gemessen wird.

62. Bioanalytisches Nachweisverfahren nach einem der Ansprüche 60-61, **dadurch gekennzeichnet, dass** besagte Änderungen von optischen Signalen aus den Messbereichen (d) hervorgerufen sind durch Änderungen des effektiven Brechungsindex im Nahfeld der optisch transparenten Schicht (a) in diesen Messbereichen und bei der jeweiligen Anregungswellenlänge gemessen werden.

63. Bioanalytisches Nachweisverfahren nach einem der Ansprüche 60-61, **dadurch gekennzeichnet, dass** es sich bei besagten Änderungen von optischen Signalen aus den Messbereichen (d) um Änderungen von ein oder mehreren Lumineszenzen gleicher oder unterschiedlicher Wellenlänge handelt, welche in besagten Messbereichen im Nahfeld der optisch transparenten Schicht (a) angeregt wurden und bei jeweils von der zugehörigen Anregungswellenlänge unterschiedlichen Wellenlängen gemessen werden.

64. Bioanalytisches Nachweisverfahren nach einem der Ansprüche 60-63, **dadurch gekennzeichnet, dass** die einen oder mehreren Lumineszenzen und / oder Bestimmungen von Lichtsignalen bei der Anregungswellenlänge polarisationsselektiv vorgenommen werden, wobei vorzugsweise die einen oder mehreren Lumineszenzen bei einer anderen Polarisation als der des Anregungslichts gemessen werden.

65. Bioanalytisches Nachweisverfahren nach einem der Ansprüche 12-64, **dadurch gekennzeichnet, dass** die zu untersuchenden Proben wässrige Lösungen oder Oberflächenwasser oder Boden- oder Pflanzenextrakte oder Bio- oder Syntheseprozessbrühen sind oder aus biologischen Gewebeteilen entnommen sind oder aus Lebensmitteln, Geruchs- oder Aromastoffen oder aus Kosmetikstoffen stammen.

66. In-vitro-Verwendung eines bioanalytischen Nachweisverfahrens nach einem der Ansprüche 12-65 zum Nachweis sekundärer Botenstoffe nach Ligandenbindung an einen Rezeptor und einer **dadurch** ausgelösten Signaltransduktion.

## Claims

1. Method for the production of a bioanalytical reagent with a plasma membrane vesicle generated from a living cell, which vesicle comprises at least one receptor and, in addition to said at least one receptor, further cellular products respectively cellular proteins which are involved in a signal transduction mechanism initiated by said receptor in the living cell used, whereby there is no mixture of the cell and vesicle contents with the surrounding external cell medium during the vesicle formation process and a signal transduction mechanism initiated by said receptor in said living cell is preserved in said plasma membrane vesicle as a component of the bioanalytical reagent, **characterized in that** said plasma membrane vesicle pinch-off from said living cell occurs after addition of cytochalasin B and/or cytochalasin D to pre-heated DMEM medium, and the method furthermore comprising the application of shear forces, preferably by means of vortexing.

2. Method according to Claim 1, **characterized in that** said method comprises the use of centrifugation steps and/or chromatography steps and/or filtration steps and/or electrophoretic methods.

3. Method according to either of Claims 1 and 2, **characterized in that** the inside of a plasma membrane vesicle prepared according to said method is free of nuclear material, and no replicative processes occur as a result.

4. Method according to any of Claims 1-3, **characterized in that** an ability, in the plasma membrane vesicle-generating living cell, of said at least one receptor which is associated with the plasma membrane vesicle as part of the bioanalytical reagent to bind to a specific ligand is preserved.

5. Method according to any of Claims 1-4, **characterized in that** the at least one receptor is selected from the group of signal-transmitting receptors, which group is composed of plasma membrane receptors and intercellular hormone receptors.

6. Method according to any of Claims 1-5, **characterized in that** said at least one plasma membrane vesicle produced by said method comprises, in addition to said at least one receptor, further biological components from the group composed of G proteins and G protein regulators, enzymes forming intracellular secondary messengers, and enzymes activating or inhibiting proteins by phosphorylation or dephosphorylation.

7. Method according to any of Claims 1-6, **characterized in that** biological, biochemical or synthetic components are associated with the outer membrane of the at least one plasma membrane vesicle produced by said method, which components serve to transport said vesicle to cells and/or organs and/or predetermined tissue in a living organism as predetermined target sites, and/or to bind to a biological or biochemical or synthetic recognition element which specifically recognizes and binds said biological or biochemical or synthetic components.

8. Method according to any of Claims 1-7, **characterized in that** said at least one plasma membrane vesicle produced by said method additionally comprises components for generating an experimentally detectable signal.

9. Method for the production of a bioanalytical reagent with a plasma membrane vesicle, generated from a living cell, according to any of Claims 1-8, **characterized in that** said plasma membrane vesicle is fused to an artificial lipid vesicle to give a mixed vesicle.

10. Method according to Claim 9, **characterized in that** said mixed vesicle is substantially enlarged in comparison to the plasma membrane vesicle generated from a living cell.

11. Method according to either of Claims 9-10, **characterized in that** said mixed vesicle includes additional natural and/or synthetic lipids and/or also additional proteins having additional functionalities, in comparison to the plasma membrane vesicle generated from a living cell.

12. Bioanalytical in-vitro detection method using a bioanalytical regent produced according to any of Claims 1 to 11 with at least one plasma membrane vesicle which is generated from a living cell and which comprises at least one receptor, wherein said at least one plasma membrane vesicle comprises, in addition to the at least one receptor, further cellular products respectively cellular proteins which are involved in a signal transduction mechanism initiated by said receptor in the living cell used, wherein said plasma membrane vesicles do not have any admixtures due to mixing with the surrounding external cell medium during the vesicle formation process and a signal transduction mechanism initiated by said receptor is preserved in said plasma membrane vesicle as a component of the bioanalytical reagent, for simultaneous or sequential, quantitative or/and qualitative determination of one or more analytes from the group composed of receptors or ligands, chelators or "histidine-tag components", enzymes, enzyme co-factors or inhibitors, wherein said detection method is selected from the group composed of, for example, optical detection methods, detection of energy transfer or charge transfer, mass spectroscopy, electrical or electrochemical detection methods, electronic resonance measurements, radioactive methods or electrophoretic measurements.

13. Bioanalytical detection method according to Claim 12, **characterized in that** said method is carried out in a homogeneous solution.

14. Bioanalytical detection method according to Claim 12, **characterized in that** said detection method is carried out with the aid of a measurement arrangement with at least 2 electrodes and separated compartments suitable for receiving liquids, wherein a solid support is located between two arbitrarily shaped electrodes which are situated opposite to one another, protruding into at least one compartment or touching such a compartment with at least one aperture and separating in each case at least 2 compartments from one another.

15. Bioanalytical detection method according to Claim 14, **characterized in that** said measurement arrangement has, on one side or on both sides of the support, means for enabling liquid to be added and/or stored and/or exchanged and/or the plasma membrane vesicle to be added between the support and the electrode.

16. Bioanalytical detection method according to either of Claims 14-15, **characterized in that** the at least one aperture of said measurement arrangement has a diameter such that, in the presence of a voltage difference across the measurement arrangement, mediated by the at least two electrodes, an inhomogeneous electric field is built up around the aperture, which increases in strength approaching said aperture and, in the proximity of said aperture, is able to move the plasma membrane vesicle electrophoretically towards said aperture.

17. Bioanalytical detection method according to either of Claims 14-15, **characterized in that** the at least one aperture of said measurement arrangement has a diameter such that the plasma membrane vesicle can be positioned by a hydrodynamic or electrokinetic flow or by other mechanical manipulation above or in the aperture.

18. Bioanalytical detection method according to any of Claims 14-17, **characterized in that** the support of said measurement arrangement has an electrically charged surface which is attractive to the plasma membrane vesicle, or that an adhesion-promoting layer for binding said plasma membrane vesicles has been applied to its surface.

19. Bioanalytical detection method according to any of Claims 14-18, **characterized in that** the plasma membrane vesicles are placed in a compartment between the partition or support and the electrode, which compartment has initially been filled with buffer or is empty, and are moved by a voltage difference applied to the electrodes onto the aperture opening and/or are positioned by a hydrodynamic or electrokinetic flow or/and mechanically onto the aperture opening.

20. Bioanalytical detection method according to any of Claims 14-19, **characterized in that** the plasma membrane vesicles are positioned onto said aperture opening, the vesicle membranes form with the surface of the support an electrically dense connection above the aperture opening and enable membrane resistance to be recorded.

21. Bioanalytical detection method according to any of Claims 14-20, **characterized in that** artificial lipid vesicles with a diameter larger than the diameter of said aperture are added to at least one compartment with the aim of generating therewith on the surface of the support a planar lipid bilayer stretching across the aperture, and **in that** the plasma membrane vesicles are then added to said compartment with the aim of fusing them to the planar lipid membrane generated, thereby making receptors associated with said plasma membrane vesicles generated from living cells accessible to electrical or optical measurements.

22. Bioanalytical detection method according to any of Claims 14-21, **characterized in that** membrane proteins are incorporated into one of the plasma membrane vesicles, after the latter has been positioned above an aperture.

23. Bioanalytical detection method according to any of Claims 14-22, **characterized in that** the plasma membrane vesicle above an aperture, or a planar membrane generated from said plasma membrane vesicle and stretching across the aperture, is accessible to optical measurements, in particular fluorescence measurements, or simultaneous optical and electrical measurements that these are performed thereon.

24. Bioanalytical detection method according to any of Claims 14-23, **characterized in that** a measurement arrangement or a measurement system having a plurality of apertures on a support is used, and **in that** measurements are carried out sequentially and/or in parallel across at least two apertures.

25. Bioanalytical detection method according to any of Claims 14-23, **characterized in that** a multiplicity of the plasma membrane vesicles generated from living cells are arranged in an array on a solid, electrically insulating support, wherein said array of plasma membrane vesicles is contacted in an electrically insulating manner with an array of patch-clamp pipettes geometrically arranged in the same way as the plasma membrane vesicle array, in order to enable electrical measurements independent of one another, or simultaneous electrical and optical measurements, to be carried out at the same time on a multiplicity of individual plasma membrane vesicles.

26. Bioanalytical detection method according to Claim 12, **characterized in that** at least one of the plasma membrane vesicles is immobilized on the surface of a solid support.

27. Bioanalytical detection method according to Claim 26, **characterized in that** a signal transduction mechanism initiated by said receptor in said living cell is preserved in said plasma membrane vesicle after immobilization.

28. Bioanalytical detection method according to Claim 26, **characterized in that** the plasma membrane vesicles are immobilized in discrete measurement regions (d), with each one or more vesicles, on the surface of said solid support.

29. Bioanalytical detection method according to Claim 28, **characterized in that** in a multiplicity of measurement regions (d) plasma membrane vesicles containing at least two different types of receptors are immobilized, whereby preferably within an individual measurement region vesicles are immobilized with an uniform type of receptors.

30. Bioanalytical detection method according to any of Claims 26-29, **characterized in that** the immobilization of the plasma membrane vesicle on the surface of said solid support is performed by means of covalent bonding or by means of physical adsorption.

31. Bioanalytical detection method according to any of Claims 26-30, **characterized in that** between the surface of said solid support and the at least one plasma membrane vesicle immobilized an adhesion-promoting layer has been applied thereon.

32. Bioanalytical detection method according to Claim 31, **characterized in that** the adhesion-promoting layer comprises a chemical compound from the group of silanes, epoxides, functionalized, charged or polar polymers and "self-assembled functionalized mono- or multilayers".

33. Bioanalytical detection method according to Claim 31, **characterized in that** the adhesion-promoting layer comprises a monomolecular layer of mainly one type of proteins or modified proteins.

34. Bioanalytical detection method according to Claim 33, **characterized in that** the adhesion-promoting layer comprises self-assembled, alkane-terminal monolayers of mainly one type of chemical or biochemical molecule.

35. Bioanalytical detection method according to any of Claims 31-34, **characterized in that** biological, biochemical or synthetic components are associated with the outer membrane of the at least one plasma membrane vesicle, which components serve to transport said plasma membrane vesicle to cells and/or organs and/or predetermined tissue in a living organism as predetermined target sites, and/or to bind to a biological or biochemical or synthetic recognition element which specifically recognizes and binds said biological or biochemical or synthetic components, and said biological or biochemical or synthetic components for specific recognition and binding with the adhesion-promoting layer are associated biological or biochemical or synthetic recognition elements which recognize and bind the plasma membrane vesicle.

36. Bioanalytical detection method according to Claim 12, **characterized in that** at least one ligand of a receptor bound to a plasma membrane vesicle is immobilized on the surface of a solid support, where appropriate via a spacer molecule.

37. Bioanalytical detection method according to Claim 36, **characterized in that** in a multiplicity of measurement regions (d) at least two different ligands of receptors bound on a plasma membrane vesicle , whereby preferably a uniform type of ligands is immobilized within an individual measurement region.

38. Bioanalytical detection method according to either of Claims 36-37, **characterized in that** said ligands are immobilized on the surface of the solid support by means of covalent binding or by means of physical adsorption.

39. Bioanalytical detection method according to either of Claims 36-37, **characterized in that** an adhesion-promoting layer has been applied between the surface of the solid support and said ligands immobilized thereon.

40. Bioanalytical detection method according to any of Claims 28-39, **characterized in that** regions between the spatially separated measurement regions, with the plasma membrane vesicles immobilized therein or with ligands of receptors bound to the plasma membrane vesicle being immobilized therein, or/and that regions within said measurement regions, between the mentioned compounds immobilized therein, are "passivated" to minimize unspecific binding of analytes or their detection substances, i.e. such that compounds which are "chemically neutral" with respect to the analytes, preferably consisting of the groups composed of albumins, casein, detergents, detergent-lipid mixtures, synthetic and natural lipids, or else hydrophilic polymers, are applied between the spatially separated measurement regions (d) or/and, within said measurement regions (d), between the mentioned compounds immobilized therein.

41. Bioanalytical detection method according to any of Claims 14-40, **characterized in that** the material of the surface of said solid support with the immobilized plasma membrane vesicles or with the immobilized ligands thereon of receptors which are bound to the plasma membrane vesicle comprises a material from the group composed of mouldable, injectable or millable plastics, carbon compounds, metals, metal oxides or silicates or silicium or germanium or ZnSe or a mixture of these materials.

42. Bioanalytical detection method according to any of Claims 14-41, **characterized in that** the surface of said solid support is essentially planar.

43. Bioanalytical detection method according to any of Claims 14-42, **characterized in that** said solid support is an optical or electronic sensor platform.

44. Bioanalytical detection method according to any of Claims 14-42, **characterized in that** said solid support is transparent over at least one wavelength range in the ultraviolet to infrared spectrum and preferably comprises a material from the group composed of mouldable, injectable or millable plastics, carbon compounds, metals, metal oxides or silicates, or a mixture of these materials.

45. Bioanalytical detection method according to Claim 43, **characterized in that** said solid support is an optical waveguide serving as sensor platform.

46. Bioanalytical detection method according to Claim 43, **characterized in that** said solid support is an optical thin-film waveguide serving as sensor platform, with a first optically transparent layer (a) with a refractive index n₁ on a second optically transparent layer (b) with a refractive index n₂, where n₁ > n₂.

47. Bioanalytical detection method according to either of Claims 45-46, **characterized in that** the sensor platform as solid support is divided into two or more discrete wave-guiding areas.

48. Bioanalytical detection method according to either of Claims 46-47, **characterized in that** the material of the second optically transparent layer (b) of the sensor platform as solid support comprises a material from the group composed of silicates or transparent mouldabie, injectable or millable plastics, preferably thermoplastics.

49. Bioanalytical detection method according to any of Claims 46-48, **characterized in that** the refractive index of the first optically transparent layer (a) of the sensor platform as solid support is greater than 1.8.

50. Bioanalytical detection method according to any of Claims 46-49, **characterized in that** the first optically transparent layer (a) of the sensor platform as solid support comprises TiO₂, ZnO, Nb₂O₅, Ta₂O₅, HfO₂, or ZrO₂, preferably TiO₂, Ta₂O₅ or Nb₂O₅.

51. Bioanalytical detection method according to any of Claims 46-50, **characterized in that** a further optically transparent layer (b') with a lower refractive index than that of layer (a) and a thickness of 5 nm-10 000 nm, preferably of 10 nm-1000 nm, is located between the optically transparent layers (a) and (b) and in contact with layer (a).

52. Bioanalytical detection method according to any of Claims 46-51, **characterized in that** in coupling of excitation light into the optically transparent layer (a) to the measurement regions (d) on the sensor platform as support is via one or more optical coupling elements from the group composed of prism couplers, evanescent couplers with optical waveguides brought into contact with each other and having overlapping evanescent fields, and end face couplers with focusing lenses, preferably cylindrical lenses, arranged in front of an end side of the wave-guiding layer, and grating couplers.

53. Bioanalytical detection method according to any of Claims 46-52, **characterized in that** the coupling of the excitation light into the measurement regions (d) is with the aid of one or more grating structures (c) formed in the optically transparent layer (a).

54. Bioanalytical detection method according to any of Claims 14-53, **characterized in that** one or more liquid samples, comprising the plasma membrane vesicle with the receptors associated therewith, are brought into contact with the ligands of said receptors, which are immobilized in one or more measurement regions, and a signal change as a result of the receptors associated with said plasma membrane vesicles binding to their immobilized ligands is measured.

55. Bioanalytical detection method according to Claim 54, **characterized in that** the signal transduction of receptors associated with the plasma membrane vesicles is measured after binding of said receptors to their immobilized ligands.

56. Bioanalytical detection method according to Claim 54, **characterized in that** the binding of receptors associated with the plasma membrane vesicles to said immobilized ligands happens in competition with the binding of these receptors associated with said plasma membrane vesicles to ligands in solution.

57. Bioanalytical detection method according to any of Claims 14-53, **characterized in that** one or more liquid samples are brought into contact with the plasma membrane vesicles with the receptors associated therewith and immobilized in one or more measurement regions, and a signal change as a result of the binding of ligands or another inducing effect on said receptors is measured.

58. Bioanalytical detection method according to any of Claims 14-53, **characterized in that** one or more liquid samples are brought into contact with the plasma membrane vesicles with the receptors associated therewith and immobilized in one or more measurement regions, and the signal transduction of said receptors as a result of the binding of ligands or another inducing effect on said receptors is measured.

59. Bioanalytical detection method according to either of Claims 57-58, **characterized in that** the binding of ligands from an added liquid sample to receptors associated with the immobilized plasma membrane vesicles is in competition to the binding of these ligands to receptors in solution which may or may not be associated with vesicles.

60. Bioanalytical detection method according to any of Claims 54-56, **characterized in that** one or more liquid samples, comprising the plasma membrane vesicles with the receptors associated therewith, are brought into contact with the ligands of these receptors, which are immobilized in one or more measurement regions, excitation light from one or more light sources of the same or different wavelength is coupled via one or more grating structures (c) to the measurement regions (d), and the change in one or more optical signals emitted from the measurement regions (d) as a result of the receptors associated with said plasma membrane vesicles binding to their immobilized ligands is measured.

61. Bioanalytical detection method according to any of Claims 57-59, **characterized in that** one or more liquid samples are brought into contact with the plasma membrane vesicles with the receptors associated therewith and immobilized in one or more measurement regions, excitation light from one or more light sources of the same or different wavelength is coupled via one or more grating structures (c) to the measurement regions (d), and the change in one or more optical signals emitted from the measurement regions (d) as a result of the binding of ligands to said receptors or of other inducing effects on said receptors is measured.

62. Bioanalytical detection method according to either of Claims 60-61, **characterized in that** said changes in optical signals from the measurement regions (d) are caused by changes in the effective refractive index in the near field of the optically transparent layer (a) in said measurement regions and are measured at the particular excitation wavelength.

63. Bioanalytical detection method according to either of Claims 60-61, **characterized in that** said changes in optical signals from the measurement regions (d) are changes in one or more luminescent events of the same or different wavelength, which have been excited in said measurement regions in the near field of the optically transparent layer (a) and are measured at wavelengths which are in each case different from the corresponding excitation wavelength.

64. Bioanalytical detection method according to any of Claims 60-63, **characterized in that** the one or more luminescent events and/or determinations of light signals are carried out at the excitation wavelength in a polarization-selective manner, preferably the one or more luminescent events being measured at a different polarization from that of the excitation light.

65. Bioanalytical detection method according to any of Claims 12-64, **characterized in that** the samples to be tested are aqueous solutions or surface water or soil or plant extracts or bioprocess or synthesis process broths, or are taken from biological tissue parts or are from food products, fragrances or flavourings or from cosmetics.

66. In-vitro use of a bioanalytical detection method according to any of Claims 12-65 for detecting secondary messengers after ligand binding to a receptor and a signal transduction caused thereby.

## Revendications

1. Procédé de préparation d'un réactif bioanalytique avec une vésicule à membrane plasmatique produite à partir d'une cellule vivante, comprenant au moins un récepteur et, outre l'au moins un récepteur, d'autres produits cellulaires ou protéines cellulaires qui participent à un mécanisme, déclenché par ce récepteur dans la cellule vivante utilisée, de transduction de signal, dans lequel, lors du processus de vésicularisation, il ne se produit pas de mélange du contenu de la cellule et de la vésicule avec le fluide cellulaire extérieur environnant et un mécanisme, déclenché par ledit récepteur dans ladite cellule vivante, de transduction de signal persiste dans ladite vésicule à membrane plasmatique en tant que composant du réactif bioanalytique, **caractérisé en ce que** la constriction de ladite vésicule à membrane plasmatique par ladite cellule vivante a lieu après ajout de cytochalasine B et/ou de cytochalasine D dans du fluide DMEM préchauffé, et le procédé incluant en outre l'utilisation de forces de cisaillement, de préférence en vortexant.

2. Procédé selon la revendication 1, **caractérisé en ce que** celui-ci comprend le recours à des étapes de centrifugation et/ou des étapes de chromatographie et/ou des étapes de filtration et/ou des méthodes électrophorétiques.

3. Procédé selon l'une quelconque des revendications 1 ou 2, **caractérisé en ce que** l'intérieur d'une vésicule à membrane plasmatique produite selon ce procédé est exempt de matériau de noyau cellulaire, de sorte qu'il ne se produit pas de processus réplicatifs.

4. Procédé selon l'une quelconque des revendications 1 à 3, **caractérisé en ce qu'**une aptitude à la liaison existant dans la cellule vivante produisant la vésicule à membrane plasmatique dudit au moins un récepteur qui est associé à la vésicule à membrane plasmatique en tant qu'élément du réactif bioanalytique persiste pour un ligand spécifique.

5. Procédé selon l'une quelconque des revendications 1 à 4, **caractérisé en ce que** l'au moins un récepteur est sélectionné dans le groupe des récepteurs transmetteurs de signaux qui est constitué des récepteurs de membrane plasmatique et des récepteurs hormonaux intracellulaires.

6. Procédé selon l'une quelconque des revendications 1 à 5, **caractérisé en ce que** ladite au moins une vésicule à membrane plasmatique produite selon ce procédé, outre ledit au moins un récepteur, comprend d'autres composants biologiques du groupe qui est constitué des protéines G et des régulateurs de protéines G, des enzymes qui forment des transmetteurs intracellulaires secondaires et des enzymes qui activent ou inhibent les protéines par phosphorylation ou déphosphorylation.

7. Procédé selon l'une quelconque des revendications 1 à 6, **caractérisé en ce que** sont associés à la membrane extérieure de l'au moins une vésicule à membrane plasmatique produite selon ce procédé des composants biologiques, biochimiques ou synthétiques qui servent au transport de ladite vésicule vers des cellules et/ou des organes et/ou des tissus prédéfinis dans un organisme vivant faisant office de lieux de destination prédéfinis et/ou à la liaison à un élément de reconnaissance biologique ou biochimique ou synthétique qui reconnaît et lie spécifiquement lesdits composants biologiques ou biochimiques ou synthétiques.

8. Procédé selon l'une quelconque des revendications 1 à 7, **caractérisé en ce que** ladite au moins une vésicule à membrane plasmatique produite selon ce procédé comprend en outre des composants pour la génération d'un signal détectable expérimentalement.

9. Procédé de préparation d'un réactif bioanalytique avec une vésicule à membrane plasmatique produite à partir d'une cellule vivante selon l'une quelconque des revendications 1 à 8, **caractérisé en ce que** ladite vésicule à membrane plasmatique est fusionnée avec une vésicule lipidique artificielle pour former une vésicule mixte.

10. Procédé selon la revendication 9, **caractérisé en ce que** ladite vésicule mixte est sensiblement agrandie par comparaison avec la vésicule à membrane plasmatique produite à partir d'une cellule vivante.

11. Procédé selon l'une quelconque des revendications 9 et 10, **caractérisé en ce que** ladite vésicule mixte, comparativement à la vésicule à membrane plasmatique produite à partir d'une cellule vivante, contient des lipides supplémentaires naturels et/ou synthétiques et/ou également des protéines supplémentaires à fonctionnalités supplémentaires.

12. Procédé de mise en évidence bioanalytique in vitro avec un réactif bioanalytique préparé selon l'une quelconque des revendications 1 à 11, comprenant au moins une vésicule à membrane plasmatique produite à partir d'une cellule vivante comportant au moins un récepteur, ladite au moins une vésicule à membrane plasmatique comportant, outre l'au moins un récepteur, d'autres produits cellulaires ou protéines cellulaires qui participent à un mécanisme, déclenché par ce récepteur dans la cellule vivante utilisée, de transduction de signal, lesdites vésicules à membrane plasmatique ne présentant pas de matières ajoutées suite à des mélanges avec le fluide cellulaire extérieur environnant pendant le processus de vésicularisation et un mécanisme, déclenché par ledit récepteur, de transduction de signal persistant dans ladite vésicule à membrane plasmatique en tant que composant du réactif bioanalytique, pour la définition simultanée ou séquentielle, quantitative et/ou qualitative d'un ou plusieurs analytes du groupe des récepteurs ou ligands, chélatants ou « composants marqueurs d'histidine », enzymes, cofacteurs enzymatiques ou inhibiteurs, ledit procédé de mise en évidence étant sélectionné dans le groupe qui est composé par exemple de procédés de mise en évidence optiques, détection de transfert d'énergie ou de charge, spectroscopie de masse, procédés de mise en évidence électriques ou électrochimiques, mesures électroniques de résonance, procédés radioactifs ou mesures électrophorétiques.

13. Procédé de mise en évidence bioanalytique selon la revendication 12, **caractérisé en ce que** celui-ci est exécuté dans de la solution homogène.

14. Procédé de mise en évidence bioanalytique selon la revendication 12, **caractérisé en ce que** ce procédé de mise en évidence est réalisé à l'aide d'un dispositif de mesure comportant au moins 2 électrodes et des compartiments séparés adaptés à la réception de liquides, dans lequel se trouve, entre deux électrodes se faisant face et rentrant chacune dans au moins un compartiment ou en touchant un et de forme quelconque, un support fixe qui contient au moins une ouverture et sépare respectivement au moins 2 compartiments l'un de l'autre.

15. Procédé de mise en évidence bioanalytique selon la revendication 14, **caractérisé en ce que** ledit dispositif de mesure présente sur une face ou les deux faces du support des moyens qui permettent un ajout de liquide et/ou une accumulation de liquide et/ou un échange de liquide et/ou l'ajout de la vésicule à membrane plasmatique entre le support et l'électrode.

16. Procédé de mise en évidence bioanalytique selon l'une quelconque des revendications 14 et 15, **caractérisé en ce que** l'au moins une ouverture dudit dispositif de mesure présente un diamètre tel que, en cas d'existence d'une différence de tension au dessus du dispositif de mesure sous l'effet des au moins deux électrodes, il s'établit autour de l'ouverture un champ électrique inhomogène qui prend plus d'importance quand on s'approche de l'ouverture et, près de l'ouverture, peut déplacer vers celle-ci la vésicule à membrane plasmatique par voie électrophorétique.

17. Procédé de mise en évidence bioanalytique selon l'une quelconque des revendications 14 et 15, **caractérisé en ce que** l'au moins une ouverture dudit dispositif de mesure présente un diamètre tel que la vésicule à membrane plasmatique peut être positionnée par un flux hydrodynamique ou électrocinétique ou par une autre manipulation mécanique sur ou dans l'ouverture.

18. Procédé de mise en évidence bioanalytique selon l'une quelconque des revendications 14 à 17, **caractérisé en ce que** le support dudit dispositif de mesure présente une surface chargée électriquement qui est attirante à la vésicule à membrane plasmatique ou qu'est appliquée sur sa surface une couche favorisant l'adhérence pour la liaison de ladite vésicule à membrane plasmatique.

19. Procédé de mise en évidence bioanalytique selon l'une quelconque des revendications 14 à 18, **caractérisé en ce que** les vésicules à membrane plasmatique sont mises dans un compartiment rempli ou non rempli préalablement de tampon entre la paroi séparatrice ou le support et l'électrode et sont déplacées par une différence de tension électrique appliquée aux électrodes sur l'orifice d'ouverture et/ou positionnées par flux hydrodynamique ou électrocinétique et/ou mécaniquement sur l'orifice d'ouverture.

20. Procédé de mise en évidence bioanalytique selon l'une quelconque des revendications 14 à 19, **caractérisé en ce que** les vésicules à membrane plasmatique sont positionnées sur ledit orifice d'ouverture, les membranes des vésicules forment une liaison électriquement dense avec la surface du support au dessus de l'orifice d'ouverture et permettent un enregistrement de la résistance de la membrane.

21. Procédé de mise en évidence bioanalytique selon l'une quelconque des revendications 14 à 20, **caractérisé en ce qu'**on met dans au moins un compartiment des vésicules lipidiques artificielles ayant un diamètre supérieur au diamètre de ladite ouverture avec pour but de créer sur la surface du support une double couche lipidique planaire tendue au dessus de l'ouverture et que la vésicule à membrane plasmatique est ensuite ajoutée dans ledit compartiment avec pour but de fusionner celle-ci avec la membrane lipidique planaire créée et de rendre ainsi les récepteurs qui sont associés à ces vésicules à membrane plasmatique produites à partir de cellules vivantes accessibles pour des mesures électriques ou optiques.

22. Procédé de mise en évidence bioanalytique selon l'une quelconque des revendications 14 à 21, **caractérisé en ce que** des protéines de membrane, après le positionnement d'une des vésicules à membrane plasmatique, sont incorporées par une ouverture dans celle-ci.

23. Procédé de mise en évidence bioanalytique selon l'une quelconque des revendications 14 à 22, **caractérisé en ce que** la vésicule à membrane plasmatique se trouvant au dessus d'une ouverture ou une membrane planaire tendue au dessus de l'ouverture et créée à partir de cette vésicule à membrane plasmatique est accessible pour des mesures optiques, en particulier des mesures de fluorescence ou des mesures optiques et électriques simultanées et ces dernières sont réalisées dessus.

24. Procédé de mise en évidence bioanalytique selon l'une quelconque des revendications 14 à 23, **caractérisé en ce qu'**on utilise un dispositif de mesure ou un système de mesure à plusieurs ouvertures sur un support et que les mesures se font séquentiellement et/ou parallèlement par au moins deux ouvertures.

25. Procédé de mise en évidence bioanalytique selon l'une quelconque des revendications 14 à 23, **caractérisé en ce qu'**une multitude des vésicules à membrane plasmatique produites à partir de cellules vivantes sont disposées en réseau sur un support fixe électriquement isblant', ce réseau étant amené en contact électriquement isolant avec un réseau de pipettes de patch-clamp de même disposition géométrique que le réseau de vésicules à membrane plasmatique afin de permettre une réalisation simultanée de mesures électriques indépendantes les unes des autres ou de mesures électriques et optiques simultanées sur une multitude de vésicules à membrane plasmatique distinctes.

26. Procédé de mise en évidence bioanalytique selon la revendication 12, **caractérisé en ce qu'**au moins une des vésicules à membrane plasmatique est immobilisée sur la surface d'un support fixe.

27. Procédé de mise en évidence bioanalytique selon la revendication 26, **caractérisé en ce qu'**un mécanisme, déclenché par ledit récepteur dans ladite cellule vivante, de transduction de signal persiste dans ladite vésicule à membrane plasmatique après l'immobilisation.

28. Procédé de mise en évidence bioanalytique selon la revendication 26, **caractérisé en ce que** les vésicules à membrane plasmatique sont immobilisées sur la surface dudit support fixe dans des plages de mesure discrètes (d) comprenant respectivement une ou plusieurs vésicules.

29. Procédé de mise en évidence bioanalytique selon la revendication 28, **caractérisé en ce que**, dans une multitude de plages de mesure (d), sont immobilisées des vésicules à membrane plasmatique comportant au moins deux types différents de récepteurs, des vésicules comprenant un type uniforme de récepteurs étant respectivement de préférence immobilisées dans une plage de mesure distincte.

30. Procédé de mise en évidence bioanalytique selon l'une quelconque des revendications 26 à 29, **caractérisé en ce que** l'immobilisation de la vésicule à membrane plasmatique sur la surface dudit support fixe est assurée au moyen d'une liaison covalente ou au moyen d'une adsorption physique.

31. Procédé de mise en évidence bioanalytique selon l'une quelconque des revendications 26 à 30, **caractérisé en ce que**, entre la surface dudit support fixe et l'au moins une vésicule immobilisée dessus, une couche favorisant l'adhérence est appliquée.

32. Procédé de mise en évidence bioanalytique selon la revendication 31, **caractérisé en ce que** la couche favorisant l'adhérence contient un composé chimique du groupe des silanes, époxydes, polymères fonctionnalisés, chargés ou polaires et des « couches uniques ou multiples fonctionnalisées auto-organisées ».

33. Procédé de mise en évidence bioanalytique selon la revendication 31, **caractérisé en ce que** la couche favorisant l'adhérence contient une couche monomoléculaire faite majoritairement d'une variété de protéines ou de protéines modifiées.

34. Procédé de mise en évidence bioanalytique selon la revendication 33, **caractérisé en ce que** la couche favorisant l'adhérence contient des monocouches auto-organisées d'extrémité alcane faites majoritairement d'une variété de molécules chimiques ou biochimiques.

35. Procédé de mise en évidence bioanalytique selon l'une quelconque des revendications 31 à 34, **caractérisé en ce que** sont associées à la membrane extérieure de l'au moins une vésicule à membrane plasmatique des composants biologiques, biochimiques ou synthétiques qui servent au transport de ladite vésicule à membrane plasmatique vers des cellules et/ou des organes et/ou des tissus prédéfinis dans un organisme vivant faisant office de lieux de destination prédéfinis et/ou à la liaison à un élément de reconnaissance biologique ou biochimique ou synthétique qui reconnaît et lie spécifiquement lesdits composants biologiques ou biochimiques ou synthétiques et sont associés à ces composants biologiques ou biochimiques ou synthétiques, pour la reconnaissance spécifique et la liaison spécifique à la couche favorisant l'adhérence, des éléments de reconnaissance biologiques ou biochimiques ou synthétiques qui reconnaissent et lient la vésicule à membrane plasmatique.

36. Procédé de mise en évidence bioanalytique selon la revendication 12, **caractérisé en ce qu'**au moins un ligand pour un récepteur qui est lié à une vésicule à membrane plasmatique est immobilisé sur la surface d'un support fixe, le cas échéant par une molécule d'espacement.

37. Procédé de mise en évidence bioanalytique selon la revendication 36, **caractérisé en ce que**, dans une multitude de plages de mesure (d), se trouvent au moins deux ligands différents pour des récepteurs qui sont liés à une vésicule à membrane plasmatique, un type uniforme de ligand étant de préférence respectivement immobilisé dans une plage de mesure distincte.

38. Procédé de mise en évidence bioanalytique selon l'une quelconque des revendications 36 et 37, **caractérisé en ce que** lesdits ligands sont immobilisés sur la surface du support fixe au moyen d'une liaison covalente ou au moyen d'une adsorption physique.

39. Procédé de mise en évidence bioanalytique selon l'une quelconque des revendications 36 et 37, **caractérisé en ce que**, entre la surface du support fixe et lesdits ligands immobilisés dessus, une couche favorisant l'adhérence est appliquée.

40. Procédé de mise en évidence bioanalytique selon l'une quelconque des revendications 28 à 39, **caractérisé en ce que** les zones situées entre les plages de mesure séparées dans l'espace sont « passivées » avec les vésicules à membrane plasmatique immobilisées dedans ou avec les ligands immobilisés dedans pour les récepteurs qui sont liés à la vésicule à membrane plasmatique ou/et que des zones situées dans ces plages de mesure, entre lesdits composés immobilisés dedans, sont « passivées » pour minimiser la liaison non spécifique d'analytes ou leurs substances de mise en évidence, c'est-à-dire que, entre les plages de mesure séparées dans l'espace (d) et/ou dans ces plages de mesure (d) entre lesdits composés immobilisés dedans, sont appliqués des composés « chimiquement neutres » par rapport aux analytes et consistant de préférence en groupes constitués des albumines, caséines, détergents, mélanges de lipides et détergents, lipides synthétiques et naturels ou polymères hydrophiles.

41. Procédé de mise en évidence bioanalytique selon l'une quelconque des revendications 14 à 40, **caractérisé en ce que** le matériau de la surface dudit support fixe comportant les vésicules à membrane plasmatique immobilisées ou les ligands immobilisés dessus pour les récepteurs qui sont liés à la vésicule à membrane plasmatique contient un matériau du groupe qui est constitué des plastiques, composés d'hydrocarbures, métaux, oxydes métalliques ou silicates ou silicium ou germanium ou ZnSe ou d'un mélange de ces matériaux pouvant être moulés, injectés ou fraisés.

42. Procédé de mise en évidence bioanalytique selon l'une quelconque des revendications 14 à 41, **caractérisé en ce que** la surface dudit support fixe est sensiblement planaire.

43. Procédé de mise en évidence bioanalytique selon l'une quelconque des revendications 14 à 42, **caractérisé en ce que** ledit support fixe est une plateforme de capteur optique ou électronique.

44. Procédé de mise en évidence bioanalytique selon l'une quelconque des revendications 14 à 42, **caractérisé en ce que** ledit support fixe est transparent dans au moins une plage de longueur d'onde dans le spectre ultraviolet à infrarouge et il contient de préférence un matériau du groupe qui est constitué des plastiques, composés d'hydrocarbures, métaux, oxydes métalliques ou silicates ou d'un mélange de ces matériaux pouvant être moulés, injectés ou fraisés.

45. Procédé de mise en évidence bioanalytique selon la revendication 43, **caractérisé en ce que** ledit support fixe est un guide d'ondes optique servant de plateforme de capteur.

46. Procédé de mise en évidence bioanalytique selon la revendication 43, **caractérisé en ce que** ledit support fixe est un guide d'ondes optique à couches minces servant de plateforme de capteur et comportant une première couche optiquement transparente (a) ayant un indice de réfraction n₁ sur une seconde couche optiquement transparente (b) ayant un indice de réfraction n₂, sachant que n₁ > n₂.

47. Procédé de mise en évidence bioanalytique selon l'une quelconque des revendications 45 et 46, **caractérisé en ce que** la plateforme de capteur faisant office de support fixe est divisée en deux ou plus zones conductrices d'ondes discrètes.

48. Procédé de mise en évidence bioanalytique selon l'une quelconque des revendications 46 et 47, **caractérisé en ce que** le matériau de la seconde couche optiquement transparente (b) de la plateforme de capteur faisant office de support fixe contient un matériau du groupe qui est constitué des silicates ou des plastiques, de préférence thermoplastiques, transparents pouvant être moulés, injectés ou fraisés.

49. Procédé de mise en évidence bioanalytique selon l'une quelconque des revendications 46 à 48, **caractérisé en ce que** l'indice de réfraction de la première couche optiquement transparente (a) de la plateforme de capteur faisant office de support fixe est supérieur à 1,8.

50. Procédé de mise en évidence bioanalytique selon l'une quelconque des revendications 46 à 49, **caractérisé en ce que** la première couche optiquement transparente (a) de la plateforme de capteur faisant office de support fixe contient du TiO₂, ZnO, Nb₂O₅, Ta₂O₅, HfO₂ ou ZrO₂, de préférence du TiO₂, Ta₂O₅ ou Nb₂O₅.

51. Procédé de mise en évidence bioanalytique selon l'une quelconque des revendications 46 à 50, **caractérisé en ce que**, entre les couches optiquement transparentes (a) et (b) et en contact avec la couche (a), se trouve une autre couche optiquement transparente (b') ayant un plus faible indice de réfraction que celui de la couche (a) et une épaisseur de 5 nm à 10 000 nm, de préférence de 10 nm à 1000 nm.

52. Procédé de mise en évidence bioanalytique selon l'une quelconque des revendications 46 à 51, **caractérisé en ce que** l'intégration de lumière d'excitation dans la couche optiquement transparente (a) pour les plages de mesure (d) sur la plateforme de capteur faisant office de support se fait via un ou plusieurs éléments d'intégration optique du groupe qui est constitué des coupleurs prismatiques, des coupleurs évanescents dotés de conducteurs d'ondes optiques rassemblés avec des champs évanescents chevauchants, des coupleurs de surface frontale dotés de lentilles de focalisation disposées devant une face frontale de la couche conductrice d'ondes, de préférence des lentilles cylindriques et des coupleurs à grille.

53. Procédé de mise en évidence bioanalytique selon l'une quelconque des revendications 46 à 52, **caractérisé en ce que** l'intégration de la lumière d'excitation dans les plages de mesure (d) a lieu à l'aide d'une ou plusieurs structures à grilles (c) qui sont gaufrées dans la couche optiquement transparente (a).

54. Procédé de mise en évidence bioanalytique selon l'une quelconque des revendications 14 à 53, **caractérisé en ce qu'**un ou plusieurs échantillons liquides, comprenant la vésicule à membrane plasmatique avec les récepteurs qui lui sont associés, sont mis en contact avec les ligands immobilisés dans une ou plusieurs plages de mesure pour ces récepteurs et qu'un changement de signal en conséquence d'une liaison desdits récepteurs associés aux vésicules à membrane plasmatique avec leurs ligands immobilisés est mesuré.

55. Procédé de mise en évidence bioanalytique selon la revendication 54, **caractérisé en ce que** la transduction de signal des récepteurs qui sont associés aux vésicules à membrane plasmatique est mesurée après liaison de ces récepteurs avec leurs ligands immobilisés.

56. Procédé de mise en évidence bioanalytique selon la revendication 54, **caractérisé en ce que** la liaison des récepteurs qui sont associés aux vésicules à membrane plasmatique avec lesdits ligands immobilisés a lieu en compétition avec la liaison des récepteurs associés auxdites vésicules à membrane plasmatique avec des ligands se trouvant dans la solution.

57. Procédé de mise en évidence bioanalytique selon l'une quelconque des revendications 14 à 53, **caractérisé en ce qu'**un ou plusieurs échantillons liquides sont mis en contact avec les vésicules à membrane plasmatique immobilisées avec les récepteurs qui leur sont associés dans une ou plusieurs plages de mesure et un changement de signal en conséquence de la liaison des ligands ou d'un autre effet inducteur sur lesdits récepteurs est mesuré.

58. Procédé de mise en évidence bioanalytique selon l'une quelconque des revendications 14 à 53, **caractérisé en ce qu'**un ou plusieurs échantillons liquides sont mis en contact avec les vésicules à membrane plasmatique immobilisées avec les récepteurs qui leur sont associés dans une ou plusieurs plages de mesure et la transduction de signal desdits récepteurs en conséquence de la liaison des ligands ou d'un autre effet inducteur sur lesdits récepteurs est mesurée.

59. Procédé de mise en évidence bioanalytique selon l'une quelconque des revendications 57 et 58, **caractérisé en ce que** la liaison des ligands d'un échantillon liquide ajouté avec des récepteurs qui sont associés aux vésicules à membrane plasmatique immobilisées a lieu en compétition avec la liaison de ces ligands avec des récepteurs se trouvant dans la solution et éventuellement associés à des vésicules.

60. Procédé de mise en évidence bioanalytique selon l'une quelconque des revendications 54 à 56, **caractérisé en ce qu'**un ou plusieurs échantillons liquides, comprenant la vésicule à membrane plasmatique avec les récepteurs qui lui sont associés, sont mis en contact avec les ligands immobilisés dans une ou plusieurs plages de mesure pour ces récepteurs, de la lumière d'excitation d'une ou plusieurs sources lumineuses de longueur d'onde identique ou différente est intégrée par une ou plusieurs structures à grilles (c) dans les plages de mesure (d), et le changement d'un ou plusieurs signaux optiques sortant des plages de mesure (d) en conséquence d'une liaison desdits récepteurs associés auxdites vésicules à membrane plasmatique avec leurs ligands immobilisés est mesuré.

61. Procédé de mise en évidence bioanalytique selon l'une quelconque des revendications 57 à 59, **caractérisé en ce qu'**un ou plusieurs échantillons liquides comprenant les vésicules à membrane plasmatique immobilisées dans une ou plusieurs plages de mesure (d) sont mis en contact avec les récepteurs qui leur sont associés, de la lumière d'excitation d'une ou plusieurs sources lumineuses de longueur d'onde identique ou différente est intégrée par une ou plusieurs structures à grilles (c) dans les plages de mesure (d) et le changement d'un ou plusieurs signaux optiques sortant des plages de mesure (d) en conséquence de la liaison des ligands auxdits récepteurs ou d'autres effets inducteurs sur lesdits récepteurs est mesuré.

62. Procédé de mise en évidence bioanalytique selon l'une quelconque des revendications 60 et 61, **caractérisé en ce que** lesdits changements de signaux optiques émanant des plages de mesure (d) sont provoqués par des changements de l'indice de réfraction effectif dans le champ proche de la couche optiquement transparente (a) dans ces plages de mesure et sont mesurés à la longueur d'onde d'excitation respective.

63. Procédé de mise en évidence bioanalytique selon l'une quelconque des revendications 60 et 61, **caractérisé en ce que** lesdits changements de signaux optiques émanant des plages de mesure (d) sont des changements d'une ou plusieurs luminescences de longueur d'onde identique ou différente qui ont été suscités dans lesdites plages de mesure dans le champ proche de la couche optiquement transparente (a) et sont mesurés à des longueurs d'ondes respectivement différentes de la longueur d'onde d'excitation concernée.

64. Procédé de mise en évidence bioanalytique selon l'une quelconque des revendications 60 à 63, **caractérisé en ce que** l'une ou plusieurs luminescences et/ou définitions de signaux lumineux sont établies avec une polarisation sélective à la longueur d'onde d'excitation, l'une ou plusieurs luminescences étant de préférence mesurées à une autre polarisation que celle de la lumière d'excitation.

65. Procédé de mise en évidence bioanalytique selon l'une quelconque des revendications 12 à 64, **caractérisé en ce que** les échantillons à analyser sont des solutions aqueuses ou de l'eau de surface ou des extraits de sol ou de plantes ou des bouillies bio ou traitées par synthèse ou sont prélevés sur des parties de tissus biologiques ou proviennent d'aliments, de substances odoriférantes ou aromatiques ou de substances cosmétiques.

66. Utilisation in vitro d'un procédé de mise en évidence bioanalytique selon l'une quelconque des revendications 12 à 65 pour la mise en évidence de transmetteurs secondaires après liaison des ligands à un récepteur et transduction de signal déclenchée de ce fait.
